# EUROPEAN PATENT APPLICATION

(11) **EP 3 981 772 A1**
(43) Date of publication of application: **13.04.2022**
(21) Application number: 20818866.4
(22) Date of filing: 05.06.2020
(51) Int. Cl.: C07D 495/04, C07K 7/06, C12Q 1/02, G01N 33/53, G01N 33/566

(54) **TETRA-FUNCTIONAL CHEMICAL PROBE AND METHOD FOR IDENTIFYING TARGET MEMBRANE PROTEIN FROM LIVING CELL OR LIVING TISSUE BY USING SAID PROBE**

(30) Priority: 07.06.2019 JP 2019107511
(71) Applicant: Otsuka Pharmaceutical Co., Ltd., Tokyo 101-8535 (JP)
(72) Inventor: MIYAJIMA, Rin, Osaka-shi, Osaka 540-0021 (JP); HAYASHI, Takashi, Osaka-shi, Osaka 540-0021 (JP); SAKAI, Koji, Osaka-shi, Osaka 540-0021 (JP); OTANI, Yuki, Osaka-shi, Osaka 540-0021 (JP); HAYASHI, Mikayo, Osaka-shi, Osaka 540-0021 (JP); WADATSU, Takashi, Osaka-shi, Osaka 540-0021 (JP); NAGABUKURO, Akira, Osaka-shi, Osaka 540-0021 (JP); TANAKA, Masaki, Osaka-shi, Osaka 540-0021 (JP); NISHIKAWA, Yuki, Osaka-shi, Osaka 540-0021 (JP)
(74) Representative: dompatent von Kreisler Selting Werner - Partnerschaft von Patent- und Rechtsanwälten mbB
(86) International application number: PCT/JP2020/022401
(87) International publication number: WO 2020/246602

(57) **Abstract**

A tetra-functional compound contains a ligand-binding moiety (A) or ligand, a reactive moiety (D), a cleavable moiety (E), and a biotin tag (B), which are linked optionally via a spacer, wherein the ligand-binding moiety (A) is an activated functional group such as an amine-reactive group, or a reactive functional group such as -COOH; the reactive moiety (D) is a group having the structure of a compound such as 2-aryl-5-carboxytetrazole; the cleavable moiety (E) is a group having the structure of a compound such as 1-(4,4-dimethyl-2,6-dioxocyclohex-1-ylidene)ethyl; and the spacer is a group such as a linear or branched alkylene group having one or more carbon atoms, substituted with a crosslinking group.

SEQUENCE LISTING

<110> OTSUKA PHARMACEUTICAL CO., LTD.

<120> Tetrafunctional chemical probe and method for identifying target
membrane protein from living cells or tissues using the probe

<130> P20-079WO

<150>JP 2019-107511
<151>2019-06-07

<160> 2

<210> 1
<211> 7
<212> PRT
<213> Artificial Sequence

<220>
<230> Designed peptide for cleavable moiety of tetrafunctional compound or
tetrafunctional chemical probe

<400>\201@1
Glu Asn Leu Tyr Phe Gln Gly
                5

<210> 2
<211> 7
<212> PRT
<213> Artificial Sequence

<220>
<230> Designed peptide for cleavable moiety of tetrafunctional compound or
tetrafunctional chemical probe

<400>\201@2
Glu Asn Leu Tyr Phe Gln Ser
                5

## Description

### Technical Field

The present invention relates to a probe capable of unbiasedly and comprehensively identifying a target protein of a specific ligand directly from living cells or tissues reflecting a phenotype by using chemoproteomics, and also relates to an identification method using the probe. In particular, the present invention relates to a probe capable of detecting various target membrane proteins containing ion channels, GPCRs, or receptors that form multiple transmembranes or multimers from living cells or brain slices, which are living tissues, and also relates to a method for identifying a target membrane protein by using the probe.

### Background Art

In the research field of chemical biology, organic chemical methods as well as molecular biological methods have been used in studies on the detection and quantification of small amounts of biomolecules, such as proteins or physiologically active substances, and understanding of the functionality or reactions of these biomolecules. As part of these efforts, compounds that act specifically on biomolecules (i.e., chemical probes) are being developed with the aim of revealing the functionality and reactions of biomolecules.

In this research field, chemoproteomics is one of the methods for elucidating the structure and functionality of proteins by using an organic chemical method.

Chemoproteomics is an approach that allows for unbiased and comprehensive identification of a target protein directly from living cells or tissues that reflect a phenotype. It is thought to greatly contribute to the development of pharmaceuticals, such as in the identification of unknown target proteins of drug candidates found in phenotypic screening, or the identification of antigens of antibodies whose antigens are unknown.

There are known conventional techniques for detecting a known membrane protein: a method for labeling living cells by using a reactive group with photoaffinity (NPL 1), a method for identifying a membrane protein by proteomics from cell membrane fractions of living cells (NPL 2), a method for detecting a target membrane protein from genetically engineered cells by using a chemical probe (NPL 3 and 4), a method for detecting a target membrane protein from cultured cells or primary cultured cells by using a chemical probe (NPL 5), a method for detecting a target protein in cells or on membranes from cultured cells by using a chemical probe (NPL 6), a fluorescence imaging method for a target membrane protein on brain slices by using a chemical probe (NPL 7), and a method for identifying a target membrane protein by using proteins such as growth factors (e.g., EGF and FGFR antibodies) (NPL 8).

Such identification of a target protein uses a probe having a part(ligand moiety) that specifically binds to the target protein. For example, a method for identifying a target protein by using a compound having biotin bound to one end and a ligand with an affinity for the target protein bound to the other end is known (e.g., NPL 1 and 9). A target protein is identified with this probe by binding the target protein to the probe via a ligand, then forming a complex of biotin and avidin via biotin, and isolating and purifying the target protein using this complex as an index. However, it is not easy to identify membrane proteins with low expression levels.

Specifically, NPL 1 discloses a method for detecting a target protein having an affinity for a ligand by using a probe containing tetrazole positioned near the ligand. This method performs irradiation with UV light after the target protein is coordinated to the ligand to form a covalent bond between tetrazole and the protein; thus, the method has the feature that the protein coordinated to the ligand is not detached, for example, in a purification step.

NPL 9 discloses a probe having a cleavable moiety incorporated at an appropriate position between the ligand and biotin in order to remove non-specific proteins, including avidin, under mild conditions.

PTL 1 discloses as a probe for identifying a target glycoprotein present in a living cell or biological fluid a trifunctional crosslinking reagent carrying (i) a ligand-reactive group for conjugation to a ligand of interest having at least one binding site on a target glycoprotein receptor, (ii) a hydrazone group for the capturing of oxidized receptor-glycoproteins, and (iii) an affinity group selected from azides and alkynes for the detection, isolation and purification of captured glycoproteins.

However, none of the above methods is considered to be a technique for identifying a target membrane protein with high reproducibility and practicality from living cells or tissues that accurately reflect its phenotype. This is because many of the proteins on cell membranes are expressed at low levels, and the reactive groups widely used in known chemoproteomics techniques are not efficient in capturing target membrane proteins; these make it difficult to specifically detect the true target membrane protein with high sensitivity. Additionally, non-specific proteins resulting from heat treatment of avidin beads or protein digestion on the beads are mixed in purified samples. For these reasons, there have been no reports on the case of identification of a target membrane protein from living tissues by using chemoproteomics.

### Citation List

### Patent Literature

PTL 1: WO2017/081069A

### Non-patent Literature

NPL 1: J. Am. Chem. Soc., 138, 14609-14615 (2016)
NPL 2: Cell Chemical Biology 2017, 24, 3-8
NPL 3: ChemBioChem 2017, 18, 1639-1649
NPL 4: Mol. Pharmacol. 2019, 95, 196-209
NPL 5: J. Am. Chem. Soc. 2018, 140, 6067-6075
NPL 6: J. Am. Chem. Soc. 2018, 140, 4259-4268
NPL 7: Nature Communications, 8, 14850 (2017)
NPL 8: Nature Communications, 9, 1519 (2018)
NPL 9: Chem Commun, 49, 5366 (2013)

### Summary of Invention

### Technical Problem

An object of the present invention is to provide a novel compound usable as a probe for identifying a protein expressed in living cells or living tissues. More preferably, an object of the present invention is to provide a novel compound usable as a probe capable of identifying a membrane protein of even low expression levels. Another object of the present invention is to provide a method for identifying a target protein, preferably a target membrane protein, present in living cells or living tissues, by using the compound as a probe.

### Solution to Problem

The present invention designed a tetra-functional compound having four functions as a means for achieving the objects: specifically, 1) a ligand-binding moiety for conjugating a ligand having an affinity (binding properties) for a target protein, 2) a reactive moiety for capturing the target protein, 3) a cleavable moiety, which is a cleaving site for selectively eluting the target protein captured by avidin beads, and 4) a biotin tag using for enriching or purifying the captured target protein. The inventors found that a target membrane protein can be detected in living cells or brain slices that are living tissue, by binding a small molecule compound or antibody that has an affinity (binding properties) for a target protein as a ligand to the tetra-functional compound, and using it as a tetra-functional chemical probe.

The present invention was completed by conducting further research on the basis of these findings, and includes the following subject matter.
Item 1.
   A tetra-functional compound comprising
   a ligand-binding moiety (A),
   a reactive moiety (D),
   a cleavable moiety (E), and
   a biotin tag (B), the moieties (A), (D), (E), and (B) being linked optionally via a spacer, wherein
   the ligand-binding moiety (A) is at least one activated functional group selected from the group consisting of an amine-reactive group, a hydroxyl-reactive group, a thiol-reactive group, an aldehyde-reactive or ketone-reactive group, an alkyl-halide-reactive or aryl-halide-reactive group, an alkyl-sulfonate-reactive or aryl-sulfonate-reactive group, an amide-reactive group, a sulfonamide-reactive group, an aryl-reactive group, a diol-reactive group, and a carboxyl-reactive group, or at least one reactive functional group selected from the group consisting of -COOH, -NH₂, -OH, -SH, -CH=CH-, -(C=O)-CH=CH-, alkyl halide, vinyl halide, aryl halide, alkyl sulfonate, vinyl sulfonate, aryl sulfonate, alkynyl, azido, epoxy, and a click tag,
   the reactive moiety (D) is a group having a structure of at least one compound selected from the group consisting of 2-aryl-5-carbonyltetrazole, phenyl azido, diazirine, α-ketoamide, 4-hydroxybenzene, phthalhydrazide, and benzophenone,
   the cleavable moiety (E) is a group having a structure of at least one compound selected from the group consisting of 1-(4,4-dimethyl-2,6-dioxocyclohex-1-ylidene)ethyl, levulinoyl ester, vicinal diol, diazobenzene, bisaryl hydrazone, dialkoxydiphenylsilane, disulfide, a peptide containing the sequence of ENLYFQG (SEQ ID NO: 1), and a peptide containing the sequence of ENLYFQS (SEQ ID NO: 2),
   the biotin tag (B) is a group represented by the following formula (1) 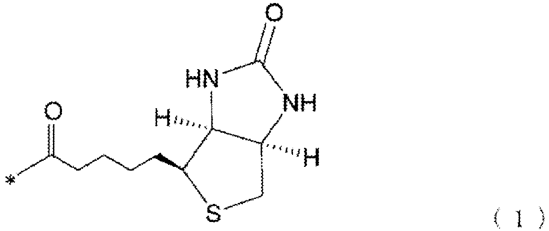 wherein symbol * represents a bond with an adjacent group;
   the spacer is a linear or branched alkylene group having one or more carbon atoms, or a linear or branched alkylene group having three or more carbon atoms wherein non-adjacent -CH₂- groups are independently replaced with at least one crosslinking group selected from the group consisting of -O-, -OCH₂-, -CH₂O-, -CO-, -NRₐ- wherein Rₐ represents a hydrogen atom or a C₁₋₆ alkyl group, the same applies below, -CO-NRₐ-, -NRₐ-CO-, a group represented by the following formula (1-1), and a group represented by the following formula (1-2) 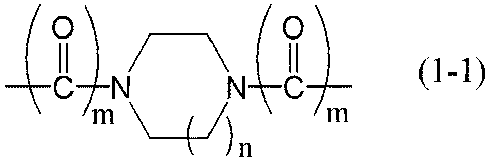 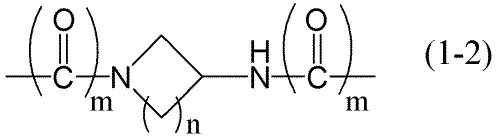 wherein m represents 0 or 1, and n represents 1 or 2.
Item 2.
   The tetra-functional compound according to Item 1, wherein the reactive moiety (D) is a group represented by the following formula (2) or (2'), 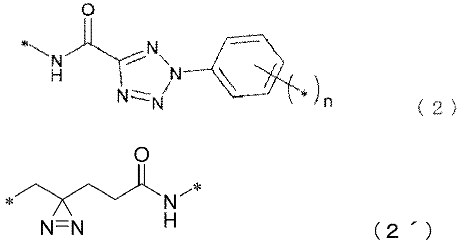
   wherein symbol * represents a bond with an adjacent group, and n represents 0 or 1,
   and/or,
   the cleavable moiety (E) is a group represented by the following formula (3) 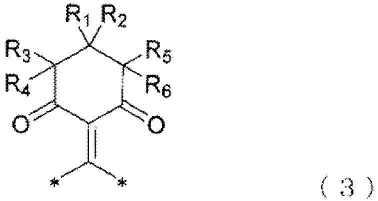
   wherein R₁, R₂, R₃, R₄, R₅, and R₆ each independently represent a hydrogen atom or a C₁₋₆ alkyl group, and symbol * represents a bond with an adjacent group.
Item 3.
   The tetra-functional compound according to Item 1 or 2, which is represented by the following formula (I) or formula (II): 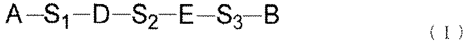 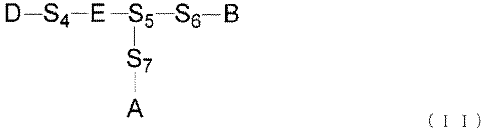 wherein A, B, C, D, and E represent the respective corresponding moieties of Item 1, and S₁, S₂, S₃, S₄, S₅, S₆, and S₇ each independently represent a spacer.
Item 4.
   The tetra-functional compound according to any one of Items 1 to 3, wherein the ligand-binding moiety (A) is an N-hydroxysuccinimide ester group.
Item 5.
   The tetra-functional compound according to Item 4, which is represented by the following formula (i), (ii), or (iii): 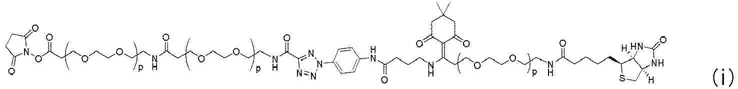 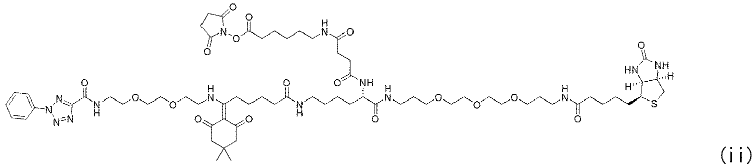 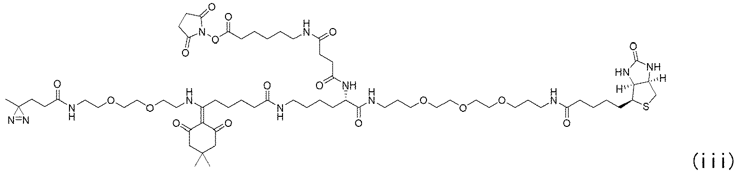 wherein p represents 2.
Item 6.
   A tetra-functional chemical probe comprising
   the tetra-functional compound of any one of Items 1 to 5, and
   a ligand binding to the ligand-binding moiety (A) of the tetra-functional compound.
Item 7.
   The tetra-functional chemical probe according to Item 6, wherein the ligand is one member selected from the group consisting of a protein, a peptide, a lipid, a carbohydrate, and a small molecule compound that all have an affinity for a membrane protein derived from a living cell or living tissue.
Item 8.
   The tetra-functional chemical probe according to Item 6 or 7, which is represented by formula (iv): 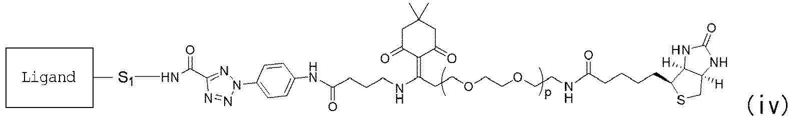 wherein S₁ represents a spacer, and p represents 2.
Item 9.
   A method for detecting a target protein in a cell or tissue by using the tetra-functional chemical probe of any one of Items 6 to 8, the method comprising
   (1) reacting a cell or tissue with the tetra-functional chemical probe to bind the ligand in the tetra-functional chemical probe with a target protein,
   (2) forming a covalent bond between the reactive moiety (D) in the tetra-functional chemical probe and the target protein,
   (3) purifying a fraction containing the target protein bound to the tetra-functional chemical probe,
   (4) binding the biotin tag in the tetra-functional chemical probe to avidin to form a conjugate of the tetra-functional chemical probe and the avidin,
   (5) cleaving the formed conjugate of the tetra-functional chemical probe and the avidin at the cleavable moiety in the tetra-functional chemical probe, and
   (6) detecting the fraction containing the target protein.

### Advantageous Effects of Invention

The tetra-functional compound according to the present invention includes a ligand-binding moiety (A), a reactive moiety (D), a cleavable moiety (E), and a biotin tag (B), which are linked optionally via a spacer, and the tetra-functional compound can be used as a tetra-functional chemical probe by binding a ligand having an affinity (binding properties) for a target protein to be detected or identified to the ligand-binding moiety.

The tetra-functional chemical probe according to the present invention has a reactive moiety that shows high reactivity and high selectivity, and a cleavable moiety that cleaves under mild conditions, which are both derived from the structure of the tetra-functional compound. Due to this structure, the tetra-functional chemical probe according to the present invention shows, in particular, an excellent collection amount of membrane proteins, detection sensitivity, and reproducibility. Thus, the use of the tetra-functional chemical probe according to the present invention enables the identification of a target membrane protein in living cells with higher sensitivity than in prior art. Additionally, the tetra-functional chemical probe according to the present invention is also useful in identifying a target membrane protein in living tissues, which are a more sophisticated and complex physiological environment.

### Brief Description of Drawings

Fig. 1 shows the results of Test Example 1 in which a target molecule was isolated from living cells using compound (CPP-127) .
Fig. 2 shows the results of Test Example 2 in which a target molecule was isolated from living tissues using compound (CPF-224) .
Fig. 3 shows the results of Test Example 2 in which a target molecule was isolated from living tissues using compound (CPF-202) .
Fig. 4 shows the results of Test Example 2 in which a target molecule was isolated from living tissues using compound (CPF-242) .
Fig. 5 shows the results of Test Example 3 in which a cell surface antigen was captured using a labeled antibody (CPA-321-labeled anti-CD71 antibody).
Fig. 6 is a graph showing the results of the amount of CD71, which is an antigen, detected from THP-1 cells using anti-CD71 antibodies labeled with compounds having different linker lengths.
Fig. 7 is a graph showing the results of the amount of CD71, which is an antigen, detected from A431 cells using anti-CD71 antibodies labeled with probes having different crosslinking groups.
Fig. 8 is a graph showing the results of the amount of EGF receptor, which is an antigen, detected from MDA-MB-231 cells using His-Tag EGF labeled with different probes.
Fig. 9 is a graph showing the results of the amount of CD71, which is an antigen, detected from A431 cells using anti-CD71 antibodies labeled by different methods.
Fig. 10 is a graph showing the results of the amount of CD71, which is an antigen, detected from THP-1 cells using anti-CD71 antibody DF1513 labeled by a secondary antibody method.

### Description of Embodiments

### (I) Explanation of Terms Used in the Specification

Unless indicated otherwise, the terms and phrases used in this specification are used in the sense described below.

The term "tetra-functional" means that four functional groups are present. As stated above, of the four functional groups, one is a functional group that constitutes a ligand-binding moiety or a ligand moiety formed at the ligand-binding moiety, one is a functional group that constitutes a reactive moiety, one is a functional group that constitutes a cleavable moiety, and the last one is a biotin residue that can bind to avidin. Biotin refers to (5-[(3aS,4S,6aR)-2-oxahexahydro-1H-thieno[3,4-d]imidazol-4-yl]pentanoic acid), which is one type of water-soluble vitamins classified into B vitamins.

In the present specification, the term "alkylene" refers to a divalent radical represented by formula -(CHR)n- wherein R represents a hydrogen atom or any substituent, derived from a hydrocarbon. An alkylene group has preferably about 1 to 30 (n=1 to 30) carbon atoms, or preferably about 1 to 10 carbon atoms.

In the present specification, the term "alkyl" refers to linear or branched lower alkyl having 1 to 6 carbon atoms (C₁₋₆). Specific examples of such alkyl include methyl, ethyl, n-propyl, isopropyl, n-butyl, isobutyl, sec-butyl, tert-butyl, n-pentyl, isopentyl, neopentyl, n-hexyl, isohexyl, and 3-methylpentyl. Alkyl is preferably linear lower alkyl having 1 to 3 carbon atoms. "C₁₋₆ alkyl" also includes lower alkyl having 1 to 3 hydrogen atoms replaced with a deuterium atom.

In the present specification, the terms "protein" and "peptide" have the same meaning, and refer to an amino acid polymer with any length (typically, a peptide is called "a fragment of a protein"). This polymer may be a linear, branched, or cyclic chain. The amino acid may be a natural or unnatural amino acid, or a mutant amino acid.

In the present invention, the protein may be a naturally occurring protein or a synthesized protein. The protein may also have a sequence prepared by synthetically engineering a naturally occurring protein. The protein may be an intracellular protein, a cell surface protein (i.e., a protein bound to the surface of a cell), or a protein in a solution (i.e., a protein secreted in a medium). The protein may also be a glycoprotein or a membrane protein. The proteins of interest in the present invention can be any pharmaceutically or commercially relevant proteins with useful biological or chemical activity, such as receptors, antibodies, enzymes, hormones, regulators, antigens, and binders. The proteins for use in the method according to the present invention listed below are simply examples, and the proteins of interest in the present invention are not limited to these proteins. Those skilled in the art will understand that any protein can be the target protein for the probe and method of the present invention.

In the present specification, the phrase "target protein" refers to a protein to which one or multiple specific types of ligands can bind. These target proteins are preferably, although not limited to, those present in biological fluids, on cells, or in tissues derived from mammals including humans and non-human animals. These target proteins are more preferably those expressed and present in living cells or in living tissues. Thus, if the target protein is, for example, a membrane protein expressed on the surface of a cell, the protein associates with the cell membrane of the living cell, and may have at least one amino acid exposed to the extracellular space, enabling one or multiple ligands to bind to the protein.

The target proteins in the present invention are, although not particularly limited to, preferably membrane proteins. A membrane protein refers to a protein that can directly or indirectly interact with a lipid membrane, in particular a lipid bilayer membrane, with at least one of the amino acid molecules that constitute the membrane protein being present in the extracellular environment. Examples include G-protein-coupled receptors, seven-transmembrane receptors, receptor tyrosine kinases, immunoglobulin superfamily and related proteins, scavenger receptors, other like receptors, transporters, ion channels, solution transporters, active transporters, co-transporter proteins, enzymes, and other like proteins.

More specifically, examples of G-protein-coupled receptors and seven-transmembrane receptors include Frizzled receptors, nucleic acid receptors, adenosine receptors, adrenergic receptors, angiotensin receptors, apelin receptors, vasopressin receptors, bradykinin receptors, bombesin receptors, chemokine receptors, cholecystokinin receptors, muscarinic acetylcholine receptors, cannabinoid receptors, cysteinyl leukotriene receptors, dopamine receptors, sphingolipid receptors, lysophosphatidic receptors, sphingosine monophosphate receptors, endothelin receptors, protease-activated receptors, free lipid receptors, galanin receptors, growth hormone secretagogue receptors, gonadotropin receptors, bile acid receptors, nicotinic acid receptors, lysophosphatidic acid receptors, anaphylatoxin chemotactic receptors, gastrin-releasing peptide receptors, orexin receptors, histamine receptors, serotonin receptors, interleukin receptors, leucine-rich repeat-containing G-protein coupled receptors, leukotriene receptors, adrenocorticotropin receptors, melanocortin receptors, melanin-concentrating hormone receptors, melatonin receptors, neuromedin receptors, neuropeptide receptors, neurotensin receptors, opioid receptors, nociceptin receptors, oxoglutaric acid receptors, oxytocin receptors, P2Y purine receptors, prostaglandin receptors, rhodopsin, relaxin receptors, somatostatin receptors, succinic acid receptors, substance P receptors, substance K receptors, thromboxane receptors, urotensin receptors, calcitonin receptors, taste receptors, metabotropic glutamate receptors, and olfactory receptors.

Examples of receptor tyrosine kinases include activin receptors, bone morphogenetic protein receptors, TNF-β receptors, AXL receptors, epidermal growth factors, ephrin receptors, insulin receptors, nerve growth factor receptors, discoidin domain receptors, vascular endothelial growth factor receptors, leukocyte receptors, hepatocyte growth factor receptors, macrophage-stimulating protein receptors, platelet-derived growth factor receptors, enterotoxin receptors, and precursors of these.

Examples of immunoglobulin superfamily and related proteins include immunoglobulin receptors, killer-cell immunoglobulin-like receptors, leukocyte immunoglobulin-like receptors, netrin receptors, T-cell receptors, various cytokine receptors, various fragments of T-cell surface glycoprotein CD, and superfamily and its precursors. Examples of other receptors include adiponectin receptors, progestin receptors, contactin-related proteins, delrin, integrin and precursors thereof, neurexin, neuropilin, Notch receptors, plexin and precursors thereof, receptor tyrosine phosphatases, selectin and precursors thereof, syndecan receptors, tumor necrosis factor (TNF) receptors, Toll-like receptors, transferrin receptors, and sortilin and precursors thereof.

Examples of transporters include aquaporin, chlorine ion channels, bestrophin, ryanodine receptors, voltage-gated potassium channels, cyclic nucleotide-gated channels, calcium-activated potassium channels, transient receptor potential channels (TRP channel), voltage-gated sodium channels, potential voltage-gated calcium channels, other voltage-gated channels, serotonin receptors, acetylcholine receptors, γ-amino butyric acid receptors, glycine receptors, ionotropic glutamate receptors, and P2X purinoreceptors. Examples of solution transporters include various SLC family proteins. Examples of active transporters include ion-transporting ATP-degrading enzymes, and ABC transporters. Additionally, examples of enzymes include NADH-ubiquinone oxidoreductase, cytochrome c, flavin-containing monooxygenase, cytochrome P450, other oxidoreductase; acyltransferase, glucose transferase, sulfotransferase, other transferases; ligase, tyrosine phosphatase, phosphodiesterase, glycosylase, serine-peptide-internal hydrolase, metalloendopeptidases, nucleoside diphosphate phosphatase, and other hydrolases.

In the present specification, the phrase "sample" or "biological sample" refers to any living cell or a solid or fluid sample obtained from an organism (biological sample). These include, for example, tissue cultures, bioreactors, human or non-human animal tissues, plants (including fruits and vegetables), unicellular microorganisms (e.g., bacteria and yeast), and multicellular organisms. The biological sample may also be a biological fluid obtained from, for example, blood, plasma, serum, urine, bile, semen, cerebrospinal fluid, aqueous humor or vitreous humor, any body's secretions, leak, or exudate (e.g., abscess, or fluid from any other site of infection or inflammation), or fluid from a joint (e.g., a normal joint or joint affected by diseases such as rheumatoid arthritis, osteoarthritis, gout, or septic arthritis). The biological sample may also be, for example, a sample obtained from any organ or tissue, including a biopsy or autopsy specimen. The cells also include primary cells and cultured cells.

The biological sample is preferably a living cell or living tissue derived from a human or non-human animal. The living cell usable in the present invention is, although not limited to, preferably a cell in which a protein, preferably a membrane protein, detectable by using a ligand, is expressed. Examples include, although not limited to, CHO cells, MDCK cells, 3T3-L1, 293 cells, MCF7 cells, A431 cells, 3T3 cells, CV-I cells, HeLa cells, L cells, BHK21 cells, HL-60 cells, U937 cells, HaK cells, Jurkat cells, THP-1 cells, other cell lines obtained by transformation, blood cells isolated from living organisms, and cell lines derived from an *in vitro* culture of a primary tissue or primary graft. Examples also include, in addition to those cultured in a two-dimensional culture, those cultured in a three-dimensional culture system, such as organoid cultures. The biological sample may also be cells into which a gene encoding a specific protein is introduced with these as a host organism by using a vector. Without any limitation, for example, CHO-K1 cells, in which dopamine D2 receptors are stably expressed, are usable.

Tissues usable in the present invention can be any tissue in which a protein, preferably a membrane protein, detectable by using a ligand, is expressed, and is not particularly limited. Examples include, but are not limited to, tissue slices and organ slice cultures of a brain, liver, kidney, etc.

In the present specification, the phrase "living cell" refers to a cell originated from a mammalian (a human or non-human animal) established as a cell line, or a cell originated from a non-mammalian source established as a cell line that has been cultured and not denatured. The living cell also includes cells isolated from any organ or tissue under non-denaturing conditions (e.g., blood cells, cells constituting an organ, and primary culture cells). Examples also include, in addition to those cultured in a two-dimensional culture, those cultured in a three-dimensional culture system, such as organoid cultures. In the present specification, the phrase "living tissue" refers to a tissue section or tissue culture sample isolated from any organ or tissue of a mammal (a human or non-human animal) under non-denaturing conditions (e.g., tissue slices and organ slice cultures of a brain, liver, kidney etc.).

In the present invention, the term "ligand" refers to a compound having an affinity for and thus bindable to a target protein. The ligand is, although not limited to, preferably any compound that binds to a cell membrane and that can interact or bind to a target membrane protein expressed in and present on the cell surface. Examples of ligands include, although not limited to, peptides, polypeptides, proteins containing glycoproteins or phosphorus proteins, carbohydrates, glycolipids, phospholipids, oligonucleotides, polynucleotides, aptamers, vitamins, antigens and their fragments, hapten, receptor agonists, partial agonists, mixed agonists, antagonists, drugs, chemokine, hormones (e.g., LH, FSH, TRH, TSH, ACTH, CRH, PRH, MRH, MSH, glucagon, and prolactin; transferrins; lactoferrin; angiotensin; histamine; insulin; and lectins), transmitters, autacoids, growth factors (e.g., PDGF, VEGF, EGF, TGFa, TBFβ, GM-CSF, G-CSF, M-CSF, FGF, IGF, bombesin, thrombopoietin, erythropoietin, oncostatin, and endothelin 1), interleukins (e.g., interleukin 1 to 15), lymphokines, cell signaling molecules, cytokines (e.g., including tumor necrosis factors (tumor necrosis factor α and β), and interferons (e.g., interferon α, β, and γ)), prosthetic groups, coenzymes, cofactors, regulators, natural or synthetic organic molecules that can specifically bind to a receptor, fragments of these ligands maintaining the same binding properties, analogues, and other derivatives.

The ligand may be an antibody. Antibodies have an ability of binding specifically to a specific antigen. The antibody may be a monoclonal antibody or polyclonal antibody. The antibody is preferably a monoclonal antibody and can be, for example, particularly a therapeutic antibody such as trastuzumab and bevacizumab. The antibody may also be a humanized antibody.

Either a primary antibody method or a secondary antibody method can be used. The primary antibody method uses an antibody having an affinity for a target protein (primary antibody) as a ligand. The secondary antibody method uses a secondary antibody integrated with the ligand-binding moiety of a probe, and the primary antibody (ligand) forms a conjugate with the secondary antibody (ligand-binding moiety). The affinity for a target protein of an antibody that could not be sensitively detected by the primary antibody method may be sensitively detected by the secondary antibody method.

The ligand may also be a manipulated affinity binder, for example, an ankyrin repeat binder, in particular an His tag binder, an affinity binder generated by phage display, an oligonucleic acid, or a peptide aptamer. The ligand may also be a protein such as a protein receptor, or a domain of a cell surface protein such as a cell surface protein receptor. Additionally the ligand may be a microorganism or virus.

In the present invention, the ligand interacts with its target protein via a binding site. The binding site is a specific peptide fragment of the target protein, for example, a specific amino acid sequence or three-dimensional structure of a fragment of the target protein, and is referred to as a "binding site." The term "interaction" in regards to a ligand that binds to a binding cite of its target protein (cell surface or secreted target protein) includes a temporary or permanent, direct or indirect contact between a cell surface or secreted target protein and the ligand, and can be characterized by its binding affinity, i.e., dissociation equilibrium constant Kd. The typical binding affinity of a ligand for its target protein can be at least 10⁻⁵ M, preferably 10⁻⁶ M or more, for example, from about 10⁻⁷ M to about 10⁻¹² M.

As described above, the ligand can be any compound that has an affinity for a target protein; however, the ligand is preferably a compound (pharmaceutical compound) used as an active ingredient in a pharmaceutical product for the treatment of diseases of human or non-human animals. The pharmaceutical compound may be a small morecule compound, a medium-molecular-weight compound, a natural product, or a protein including an antibody. The pharmaceutical compound may be modified so as to bind to the ligand-binding moiety of the tetra-functional compound according to the present invention to the extent that modification does not affect the affinity for a target protein. In the tetra-functional chemical probe according to the present invention, the ligand can bind to the reactive moiety via a spacer.

In the present specification, alkylation, hydrolysis, amination, esterification, amidation, etherification, nucleophilic substitution, addition, oxidation, and reduction refer to methods that are known *per se.* These methods are, for example, those described in Jikken Kagaku Koza (5th edition, The Chemical Society of Japan, Maruzen Publishing), Organic Functional Group Preparations (2nd edition, Academic Press, Inc., issued in 1989), Comprehensive Organic Transformations (VCH Publishers Inc., issued in 1989), and Greene's Protective Groups in Organic Synthesis (authored by P.G.M. Wuts and T.W. Greene, 4th edition, 2006).

In the present specification, "palladium compound" is not particularly limited. Examples include tetravalent palladium catalysts, such as sodium hexachloropalladium(IV) tetrahydrate, and potassium hexachloropalladium(IV); divalent palladium catalysts, such as [1,1'-bis(diphenylphosphino)ferrocene]palladium(II) dichloride dichloromethane adducts (Pd(dppf)Cl₂·CH₂Cl₂), (2-dicyclohexylphosphino-2',4',6'-triisopropyl-1,1'-biphenyl)[2-(2'-amino-1,1'-biphenyl)]palladium(II) methanesulfonate (XPhos Pd G3), palladium(II) chloride, palladium(II) bromide, palladium(II) acetate, palladium(II) acetylacetonate, dichlorobis(benzonitrile)palladium(II), dichlorobis(acetonitrile)palladium(II), dichlorobis(triphenylphosphine)palladium(II), dichlorotetraamminepalladium(II), dichloro(cycloocta-1,5-diene)palladium(II), palladium(II) trifluoroacetate, and 1,1'-bis(diphenylphosphino)ferrocene dichloropalladium(II)-dichloromethane complexes; and zero-valent palladium catalysts, such as tris(dibenzylideneacetone)dipalladium(0) (Pd₂ (dba)₃), tris(dibenzylideneacetone)dipalladium chloroform complexes (0), and tetrakis(triphenylphosphine)palladium(0) (Pd(PPh₃)₄). These palladium compounds can be used singly, or in a combination of two or more.

In the present specification, examples of carbodiimides include dicyclohexylcarbodiimide (DCC), diisopropylcarbodiimide (DIC), 1-ethyl-3-(3-dimethylaminopropyl)carbodiimide hydrochloride (EDAC), 3-ethyl-1-(3-dimethylaminopropyl)carbodiimide (WSC), N-[3-(dimethylamino)propyl]-N'-ethylcarbodiimide, N-[3-(dimethylamino)propyl]-N'-ethylcarbodiimide methiodide, N-tert-butyl-N'-ethylcarbodiimide, N-cyclohexyl-N'-(2-morpholinoethyl)carbodiimide metho-p-toluenesulfonate, N,N'-di-tert-butylcarbodiimide, and N,N'-di-p-tolylcarbodiimide.

In the present specification, examples of protective groups include a t-butoxycarbonyl (BOC) group, a 9-fluorenylmethyloxycarbonyl (Fmoc) group, a 2,2,2-trichloroethoxycarbonyl (Troc) group, a benzyloxycarbonyl (Z) group, an allyloxycarbonyl (Alloc) group, a trifluoroacetyl group, a phthaloyl group, a para-toluenesulfonyl (Ts) group, and a 2-nitrobenzenesulfonyl (Ns) group.

In the present specification, the term "solvent" refers to a solvent inactive to a reaction. Examples include water, ethers (e.g., dioxane, tetrahydrofuran, diethyl ether, 1,2-dimethoxyethane, diethylene glycol dimethyl ether, and ethylene glycol dimethyl ether), halohydrocarbons (e.g., methylene chloride, chloroform, 1,2-dichloroethane, and carbon tetrachloride), aromatic hydrocarbons (e.g., benzene, toluene, and xylene), lower alcohols (e.g., methanol, ethanol, and isopropanol), polar solvents (e.g., N,N-dimethylformamide (DMF), N-methylpyrrolidone (NMP), dimethylsulfoxide (DMSO), hexamethylphosphoric triamide, and acetonitrile). These solvents can be used singly, or in a combination of two or more.

In the present specification, amines include trialkylamine (e.g., trimethylamine, triethylamine, N,N-diisopropylethylamine), and dialkylamine (e.g., diethylamine, and diisopropylamine).

In the present specification, examples of bases include inorganic bases and organic bases. Inorganic bases include alkali metal hydroxides (e.g., lithium hydroxide, sodium hydroxide, and potassium hydroxide), alkali earth metal hydroxides (e.g., magnesium hydroxide, calcium hydroxide, and barium hydroxide), alkali metal carbonates (e.g., sodium carbonate, potassium carbonate, and cesium carbonate), alkali earth metal carbonates (e.g., magnesium carbonate, calcium carbonate, and barium carbonate), alkali metal hydrogen carbonates (e.g., sodium hydrogen carbonate, and potassium hydrogen carbonate), alkali metal phosphates (e.g., sodium phosphate, potassium phosphate, and cesium phosphate), alkali earth metal phosphates (e.g., magnesium phosphate, and calcium phosphate), alkali metal alkoxides (e.g., sodium methoxide, sodium ethoxide, potassium tert-butoxide), and alkali metal hydrides (e.g., sodium hydride, and potassium hydride). Organic bases include trialkylamines (e.g., trimethylamine, triethylamine (TEA), N,N-diisopropylethylamine (DIPEA)), dialkylamines (e.g., diethylamine, and diisopropylamine), 4-dimethylaminopyridine (DMAP), N-methylmorpholine, picoline, 1,5-diazabicyclo[4.3.0]non-5-ene, 1,4-diazabicyclo[2.2.2]octane, and 1,8-diazabicyclo[5.4.0]-7-undecene (DBU). One or more of these can be suitably selected and mixed for use.

In the present specification, examples of condensing agents include 1-hydroxybenzotriazole (HOBt), 3-hydroxy-3,4-dihydro-1,2,3-benzotriazin-4-one (HOOBt), N-hydroxysuccinimide (NHS), dicyclohexylcarbodiimide (DCC), diisopropylcarbodiimide (DIC), 1-ethyl-3-(3-dimethylaminopropyl)-carbodiimide (EDAC), 2-(1H-7-azabenzotriazol-1-yl)-1,1,3,3-tetramethyluronium hexafluorophosphate (HATU), 2-(1H-benzotriazol-1-yl)-1,1,3,3-tetramethyluronium hexafluorophosphate (HBTU), 3,4-dihydro-1,2,3-benzotriazin-4-one-3-oxytetramethyluronium hexafluorophosphate (HDTU), benzotriazol-1-yloxytris(dimethylamino) phosphonium hexafluorophosphate (BOP), benzotriazol-1-yloxytris-(pyrrolidino)-phosphonium hexafluorophosphate (PyBop), (3,4-dihydro-1,2,3-benzotriazin-4-one-3-oxy)diethylphosphate (DEPBt), 3,4-dihydro-1,2,3-benzotriazin-4-one-3-oxytris-(pyrrolidino)-phosphonium hexafluorophosphate (PDOP), 2-(benzotriazol-1-yloxy)-1,3-dimethyl-2-pyrrolidin-1-yl-1,3,2-diazaphosphoridinium hexafluorophosphate (BOMP), 5-(1H-7-azabenzotriazol-1-yloxy)-3,4-dihydro-1-methyl 2H-pyrroliumhexachloroantimonate (AOMP), (1H-7-azabenzotriazol-1-yloxy) tris(dimethylamino) phosphonium hexafluorophosphate (AOP), 5-(1H-benzotriazol-1-yl)-3,4-dihydro-1-methyl 2H-pyrroliumhexachloroantimonate:N-oxide (BDMP), 2-bromo-3-ethyl-4-methylthiazoliumtetrafluoroborate (BEMT), 2-bromo-1-ethylpyridiniumtetrafluoroborate (BEP), 2-bromo-1-ethylpyridiniumhexachloroantimonate (BEPH), benzotriazol-1-yloxy-N,N-dimethylmethaneiminiumhexachloroantimonate (BOMI), N,N'-bis(2-oxo-3-oxazolidinyl)phosphinic chloride (BOP-Cl), 1-(1H-benzotriazol-1-yloxy)phenylmethylene pyrrolidinium hexachloro-antimonate (BPMP), 1,1,3,3-bis(tetramethylene)fluorouronium hexafluorophosphate (BTFFH), 4-(chloro-4-morpholinylmethylene) morpholinium hexafluorophosphate (CMMM), 2-chloro-1,3-dimethyl-1H-benzimidazolium hexafluorophosphate (CMBI), 2-fluoro-1-ethylpyridinium tetrafluoroborate (FEP), 2-fluoro-1-ethylpyridinium hexachloroantimonate (FEPH), 1-(1-pyrrolidinyl-1H-1,2,3-triazolo[4,5-b]pyridin-1-ylmethylene)pyrrolidinium hexafluorophosphate N-oxide (HAPyU), O-(1H-benzotriazol-1-yl)-N,N,N',N'-bis-(pentamethylene)uronium hexafluorophosphate (HBPipU), O-(1H-benzotriazol-1-yl)-N,N,N0,N0-bis(tetramethylene)uronium hexafluorophosphate (HBPyU), (1H-7-azabenzotriazol-1-yloxy) tris(pyrrolidino)phosphonium hexafluorophosphate (PyAOP), bromo-tripyrrolidinophosphonium hexafluorophosphate (PyBrOp), chloro-tripyrrolidinophosphonium hexafluorophosphate (PyClOP), 1,1,3,3-bis(tetramethylene)chlorouronium hexafluorophosphate (PyClU), tetramethylfluoro-mamidinium hexafluorophosphate (TFFH), triphosgene, triazine-based reagents (cyanuric chloride, cyanuric fluoride, 4-(4,6-dimethoxy-1,3,5-triazin-2-yl)-4-methylmorpholinium chloride (DMT-MM), 2-chloro-4,6-dimethoxy-1,3,5-triazine (CDMT)), bis(2-chlorophenyl)phosphorochloridate, diphenylphosphorochloridate, diphenylphosphorylazide (DPPA), N-[1-(cyano-2-ethoxy-2-oxoethylideneaminooxy)dimethylamino(morpholino)]uronium hexafluorophosphate (COMU), and N,N,N',N'-tetramethyl-O-(N-succinimidyl)uronium tetrafluoroborate (TSTU).

In the present specification, the deprotecting agent can be any agent capable of detaching a protective group. Examples include bases such as piperidine and lithium diisopropylamide, acids such as zinc, and TFA, nucleophilic reagents coexisting with a zero-valent palladium catalyst, aqueous basic solutions, methylamine, hydrazine, one-electron reducing agents, and thiols.

### (II) Tetra-functional Compound and Tetra-functional Chemical Probe

The tetra-functional compound according to the present invention is a compound containing a ligand-binding moiety (A), a reactive moiety (D), a cleavable moiety (E), and a biotin tag (B), which are linked optionally via a spacer. A tetra-functional chemical probe useful in detection, identification, or purification of a target protein can be prepared by binding a ligand having an affinity for the target protein (binding properties) to the ligand-binding moiety of this compound. Specifically, the tetra-functional chemical probe is a compound containing a ligand moiety, a reactive moiety (D), a cleavable moiety (E), and a biotin tag (B), which are linked via or not via a spacer.

The following describes the four functional moieties of the tetra-functional compound and tetra-functional chemical probe according to the present invention, and the spacer linking the four moieties.

### Ligand-binding Moiety

The ligand-binding moiety (A) of the tetra-functional compound according to the present invention is a site at which a ligand binds to the tetra-functional compound (coupling site). Thus, the ligand-binding moiety (A) can be a reactive functional group or activated functional group which reacts with a reactive counterpart group present on the ligand (these groups are collectively referred to as "ligand-reactive group").

The activated functional group refers to a reactive functional group activated by a standard chemical technique in order to obtain a corresponding activated functional group. In a specific embodiment, the activated functional group is selected from the group consisting of amine-reactive groups, hydroxyl-reactive groups, thiol-reactive groups, aldehyde-reactive or ketone-reactive groups, alkyl-halide-reactive or aryl-halide-reactive groups, alkyl-sulfonate-reactive or aryl-sulfonate-reactive groups, amide-reactive groups, sulfonamide-reactive groups, aryl-reactive groups, diol-reactive groups, and carboxyl-reactive groups.

The amine-reactive group refers to an activated functional group reactive with a primary or secondary amine. Typical amine-reactive groups include aryl-activated or alkyl-activated carboxylic ester-COOR, such as N-hydroxysuccinimide ester or derivatives thereof (e.g., sulfo-N-hydroxysuccinimide ester), and phenol ester or derivatives thereof (e.g., R represents phenol, p-nitrophenol, or tetrafluorophenol). Other amine-reactive groups include acyl chloride (-COCl), aryl or alkyl imidate-C (NH) OMe), alkyl or aryl isocyanate (-NCO), isothiocyanate (-NCS), aldehyde, in particular 2-pyridinecarboxy aldehyde, carbonyl, epoxy, α,β-unsaturated carbonyl, alkyl or aryl halide, and alkyl or aryl sulfonate.

The hydroxyl-reactive group refers to an activated functional group reactive with hydroxyl. Examples of typical hydroxyl-reactive groups include alkyl or aryl isocyanate-NCO, aryl-activated or alkyl-activated carboxylic ester-COOR, epoxy, α,β-unsaturated carbonyl, alkyl or aryl halide, and alkyl or aryl sulfonate.

The thiol-reactive group refers to an activated functional group reactive with thiol. Examples of typical thiol-reactive groups include maleimide, α-haloamide (-NH-CO-CH₂-Hal), epoxy, α,β-unsaturated carbonyl, alkyl or aryl halide, and alkyl or aryl sulfonate.

The aldehyde-reactive or ketone-reactive group refers to an activated functional group reactive with aldehyde or ketone. Examples of typical aldehyde-reactive or ketone-reactive groups include aryl or alkyl amine, aryl or alkyl hydrazine (-NHNH₂), aryl or alkyl acyl hydrazine (-CO-NHNH₂), alkyl or aryl hydroxylamine (-ONH₂), and alkyl or aryl magnesium halide.

The alkyl-halide-reactive or aryl-halide-reactive group refers to an activated reactive group reactive with alkyl halide or aryl halide. Typical alkyl-halide-activated or aryl-halide-activated reactive groups include amine, alcohol, mercapto, amide, sulfonamide, carboxylic acid, alkene, alkyne, boronic acid, boronic acid ester, and alkyl tin.

The alkyl-sulfonate-reactive or aryl-sulfonate-reactive group refers to an activated reactive group reactive with alkyl sulfonate or aryl sulfonate. Typical alkyl-sulfonate or aryl-sulfonate activated reactive groups include amine, alcohol, mercapto, amide, sulfonamide, carboxylic acid, alkene, alkyne, boronic acid, boronic acid ester, and alkyl tin.

The amide-reactive group refers to an activated reactive group reactive with unsubstituted or substituted amide. Typical amide-reactive groups include epoxy, α,β-unsaturated carbonyl, alkyl or aryl halide, and alkyl or aryl sulfonate.

The sulfonamide-reactive group refers to an activated reactive group reactive with unsubstituted or substituted sulfonamide. Typical sulfonamide-reactive groups include epoxy, α,β-unsaturated carbonyl, alkyl or aryl halide, and alkyl or aryl sulfonate.

The aryl-reactive group refers to an activated functional group reactive with an aryl group. Examples of typical aryl-reactive groups include 4-hydroxybenzene and phthalhydrazide.

The diol-reactive group refers to an activated functional group reactive with a diol group. Examples of typical diol-reactive groups include boronic acid.

The carboxyl-reactive group refers to an activated functional group reactive with a carboxyl group. Examples of typical carboxyl-reactive groups include halogen, alkyl or aryl sulfonate, hydroxyl, epoxy, mercapto, amino, isocyanato, and carbodiimide.

The reactive functional group refers to a free functional group that is not protected, unless indicated otherwise. Specifically, the reactive functional group can be selected from the group consisting of -COOH, -NH₂, -OH, -SH, -CH=CH-, -(C=O)-CH=CH-, alkyl, vinyl or aryl halide, alkyl, vinyl or aryl sulfonate, alkynyl, azido, epoxy, and a click tag.

Examples of activating reagents for use in activating a reactive functional group include, although not limited to, 1-hydroxybenzotriazole (HOBt), 3-hydroxy-3,4-dihydro-1,2,3-benzotriazin-4-one (HOOBt), N-hydroxysuccinimide (NHS), dicyclohexyl carbodiimide (DCC), diisopropylcarbodiimide (DIC), 1-ethyl-3-(3-dimethylaminopropyl)-carbodiimide (EDAC), 2-(1H-7-azabenzotriazol-1-yl)-1,1,3,3-tetramethyluronium hexafluorophosphate (HATU), 2-(1H-benzotriazol-1-yl)-1,1,3,3-tetramethyluronium hexafluorophosphate (HBTU), 3,4-dihydro-1,2,3-benzotriazin-4-one-3-oxytetramethyluronium hexafluorophosphate (HDTU), benzotriazol-1-yloxytris(dimethylamino) phosphonium hexafluorophosphate (BOP), benzotriazol-1-yloxytris-(pyrrolidino)-phosphonium hexafluorophosphate (PyBop), (3,4-dihydro-1,2,3-benzotriazin-4-one-3-oxy) diethylphosphate (DEPBt), 3,4-dihydro-1,2,3-benzotriazin-4-one-3-oxytris-(pyrrolidino)-phosphonium hexafluorophosphate (PDOP), 2-(benzotriazol-1-yloxy)-1,3-dimethyl-2-pyrrolidin-1-yl-1,3,2-diazaphosphoridinium hexafluorophosphate (BOMP), 5-(1H-7-azabenzotriazol-1-yloxy)-3,4-dihydro-1-methyl 2H-pyrroliumhexachloroantimonate (AOMP), (1H-7-azabenzotriazol-1-yloxy) tris(dimethylamino)phosphonium hexafluorophosphate (AOP), 5-(1H-benzotriazol-1-yl)-3,4-dihydro-1-methyl 2H-pyrroliumhexachloroantimonate:N-oxide (BDMP), 2-bromo-3-ethyl-4-methyl thiazoliumtetrafluoroborate (BEMT), 2-bromo-1-ethylpyridinium tetrafluoroborate (BEP), 2-bromo-1-ethylpyridinium hexachloroantimonate (BEPH), benzotriazol-1-yloxy-N,N-dimethyl methaneiminium hexachloroantimonate (BOMI), N,N'-bis(2-oxo-3-oxazolidinyl) phosphinic chloride (BOP-Cl), 1-(1H-benzotriazol-1-yloxy)phenylmethylene pyrrolidinium hexachloro-antimonate (BPMP), 1,1,3,3-bis(tetramethylene) fluorouronium hexafluorophosphate (BTFFH), 4-(chloro-4-morpholinylmethylene) morpholinium hexafluorophosphate (CMMM), 2-chloro-1,3-dimethyl-1H-benzimidazolium hexafluorophosphate (CMBI), 2-fluoro-1-ethylpyridinium tetrafluoroborate (FEP), 2-fluoro-1-ethylpyridinium hexachloroantimonate (FEPH), 1-(1-pyrrolidinyl-1H-1,2,3-triazolo[4,5-b]pyridin-1-ylmethylene)pyrrolidinium hexafluorophosphate N-oxide (HAPyU), O-(1H-benzotriazol-1-yl)-N,N,N',N'-bis-(pentamethylene) uronium hexafluorophosphate (HBPipU), O-(1H-benzotriazol-1-yl) N,N,N0,N0-bis(tetramethylene)uronium hexafluorophosphate (HBPyU), (1H-7-azabenzotriazol-1-yloxy) tris(pyrrolidino)phosphonium hexafluorophosphate (PyAOP), bromo-tripyrrolidinophosphonium hexafluorophosphate (PyBrOp), chloro-tripyrrolidinophosphonium hexafluorophosphate (PyClOP), 1,1,3,3-bis(tetramethylene) chlorouronium hexafluorophosphate (PyClU), tetramethylfluoro-mamidinium hexafluorophosphate (TFFH), triphosgene, triazine-based reagents [cyanuric chloride, cyanuric fluoride, 4-(4,6-dimethoxy-1,3,5-triazin-2-yl)-4-methylmorpholinium chloride (DMT-MM), 2-chloro-4,6-dimethoxy-1,3,5-triazine (CDMT)], bis(2-chlorophenyl)phosphorochloridate, diphenylphosphorochloridate, diphenylphosphorylazide (DPPA), N-[1-(cyano-2-ethoxy-2-oxoethylideneaminooxy)dimethylamino(morpholino)]uronium hexafluorophosphate (COMU), bases, Lewis acids, palladium acetate, 1,1'-bis(diphenylphosphino)ferrocene, cupric sulfate, (tris(2-carboxyethyl)phosphine (TCEP), sodium ascorbate, tris[(1-benzyl-1H-1,2,3-triazol-4-yl)methyl]amine (TBTA), tris(2-benzoimidazolylmethyl)amine, 3,3',3''-(4,4',4''-(nitrilotris(methylene))tris(1H-1,2,3-triazol-4,1-diyl))tris(propan-1-ol), and any combinations of these.

Those skilled in the art will be easily able to understand which ligand-reactive group should be selected for coupling with a selected ligand, and will be able to suitably select a ligand-reactive group from among those listed above for use.

In a preferable embodiment, the ligand-binding moiety (A) is an activated functional group selected from the group consisting of an amine-reactive group, a hydroxyl-reactive group, a thiol-reactive group, an aldehyde-reactive or ketone-reactive group, an alkyl-halide-reactive or aryl-halide-reactive group, an alkyl-sulfonate-reactive or aryl-sulfonate-reactive group, an amide-reactive group, a sulfonamide-reactive group, an aryl-reactive group, a diol-reactive group, and a carboxyl-reactive group. Particularly preferably, the ligand-binding moiety (A) is aryl-activated or alkyl-activated carboxylic ester-COOR, such as N-hydroxysuccinimide ester represented by the following formula. 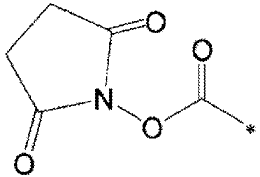 wherein the symbol * represents a binding site with an adjacent group.

In another preferable embodiment, the ligand-binding moiety (A) is a reactive functional group selected from the group consisting of -COOH, -NH₂, -OH, -SH, -CH=CH-, -(C=O)-CH=CH-, alkyl, vinyl or aryl halide, alkyl, vinyl, or aryl sulfonate, alkynyl, azido, epoxy, and a click tag. The click tag is a functional group for use in bioorthogonal reactions. Examples include diphenylphosphinylphenyl, 1,2,4,5-tetragin-3-yl, trans-cyclooctenyl, norbornenyl, cyclopropenyl, boronovinyl, boronoaryl, and halogenated aryl.

### Reactive Moiety: D

The reactive moiety (D), which is another functional moiety of the tetra-functional compound according to the present invention, is a group capable of forming a covalent bond with a target protein. This group can be any group that has this action, and is preferably a group that forms a covalent bond under simple reaction conditions. Examples of such groups include those having the structure (moiety) of at least one compound selected from the group consisting of 2-aryl-5-carbonyltetrazole (ACT), phenyl azido, diazirine, α-ketoamide, 4-hydroxybenzene, phthalhydrazide, and benzophenone. ACT can covalently bind mainly to a carboxyl group of a protein, and phenyl azido can covalently bind mainly to an amino group of a protein. 4-Hydroxybenzene and phthalhydrazide can covalently bind mainly to a 4-hydroxyphenyl group of a protein. Diazirine, α-ketoamide, and benzophenone can form a covalent bond non-specifically with a protein.

A preferable group is the group having the structure of ACT represented by the following formula (2): 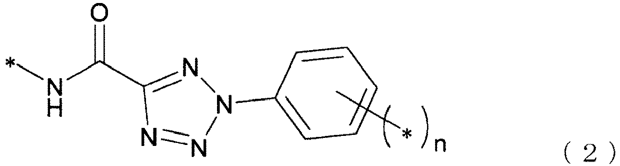 wherein the symbol * represents a binding site with an adjacent group; n represents 0 or 1; n being 0 means that there is no group coordinating to the benzene ring (i.e., ACT is located at the end of the tetra-functional compound according to the present invention). When n is 1, the bond with an adjacent group may be at the ortho-position, meta-position, or para-position with respect to the tetrazole group coordinating to the benzene ring, and is preferably at the para-position.

In the embodiment above, the simple reaction is, for example, irradiation with UV light. The wavelength and irradiation time of UV light to irradiate are not particularly limited as long as the reactive moiety of the tetra-functional compound according to the present invention can covalently bind to a target protein. The wavelength is preferably 254 to 365 nm, and more preferably 302 nm. The irradiation time is preferably 1 second to 10 minutes, more preferably 30 seconds to 5 minutes, and still more preferably 1 minute.

Another preferable group is the group having the structure of diazirine represented by the following formula (2'): 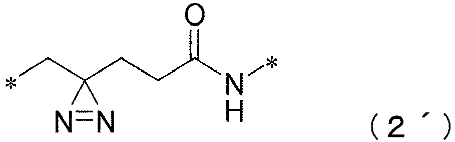 wherein the symbol * represents a binding site with an adjacent group.

In the embodiment above, the simple reaction is, for example, irradiation with UV light. The wavelength and irradiation time of UV light to irradiate are not particularly limited as long as the reactive moiety of the tetra-functional compound according to the present invention can covalently bind to a target protein. The wavelength is preferably 254 to 380 nm, and more preferably 365 nm. The irradiation time is preferably 5 minutes to 30 minutes, more preferably 10 minutes to 20 minutes, and still more preferably 15 minutes.

### Cleavable Moiety: E

The cleavable moiety (E), which is another functional moiety of the tetra-functional compound according to the present invention, is a group capable of cleavage under specific conditions. This group can be any group that has this action, and is preferably a group cleavable under mild conditions. Examples of such groups include a group having the structure (moiety) of at least one compound selected from the group consisting of 1-(4,4-dimethyl-2,6-dioxocyclohex-1-ylidene)ethyl (Dde), levulinoyl ester, vicinal diol, diazobenzene, bisaryl hydrazone, dialkoxydiphenylsilane, disulfide, a peptide containing the sequence of ENLYFQG (SEQ ID NO: 1), and a peptide containing the sequence of ENLYFQS (SEQ ID NO: 2), or a disulfide group. The group preferably has the structure of Dde represented by the following formula (3): 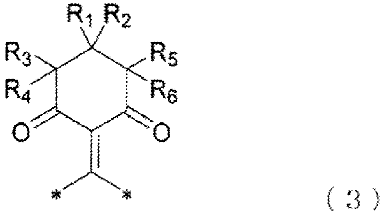 wherein R₁, R₂, R₃, R₄, R₅, and R₆ each independently represent a hydrogen atom or a C₁₋₆ alkyl group; in a preferable embodiment, R₁ and R₂ are an alkyl group, and R₃, R₄, R₅, and R₆ are a hydrogen atom; the alkyl group is preferably a C₁₋₃ alkyl group, and more preferably a methyl group; and the symbol * represents a bond with an adjacent group.

In the embodiment above, the mild reaction conditions refer to the conditions under which proteins captured by a treatment do not degrade, and contaminating proteins non-specifically bound to avidin beads are not eluted. Specifically, the mild reaction conditions mean the conditions under which the cleavable moiety (E) can be chemically or biochemically cleaved; for example, a treatment with a dilute aqueous hydrazine solution, a dilute aqueous sodium periodate solution, a dilute aqueous sodium dithionite solution, a dilute formic acid solution, a tobacco etch virus (TEV) protease solution, a dilute aqueous (tris(2-carboxyethyl)phosphine solution, or a dilute aqueous dithiothreitol solution. More preferably, the mild reaction conditions mean a treatment with a dilute aqueous hydrazine solution. The term "dilute" as used here means, although not limited to, a concentration of 5 mass% or less, and preferably 3 mass% or less.

### Biotin Tag: B

The biotin tag (E), which is another functional moiety of the tetra-functional compound according to the present invention, is a group having the structure of biotin represented by the following formula (1): 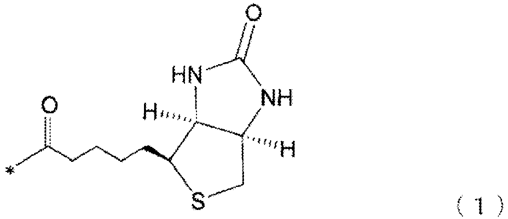 wherein the symbol * represents a bond with an adjacent group.

The biotin tag refers to a tag capable of specifically binding to a solid support to which avidin or streptavidin is bound (e.g., magnetic particles, beads, plates, filters, membranes, and resin for chromatography). The biotin tag effectively functions in detecting, identifying, or purifying a target protein.

### Spacer

The four functional moieties described above link to each other via independent spacers, thereby forming the tetra-functional compound according to the present invention. The spacers that link these four functional moieties may be a linear or branched alkylene group having one or more carbon atoms, or a linear or branched alkylene group having three or more carbon atoms, in the alkylene chain of which, non-adjacent -CH₂- groups are independently replaced with at least one crosslinking group selected from the group consisting of -O-, -OCH₂-, -CH₂O-,-CO-, -NRₐ- (wherein Rₐ represents a hydrogen atom or a C₁₋₆ alkyl group, the same applies below) , -CO-NRₐ-, -NRₐ-CO-, a group represented by the following formula (1-1), and a group represented by the following formula (1-2). The crosslinking group can replace a -CH₂- group within an alkylene chain or a -CH₂- group at the end of an alkylene chain. Rₐ is preferably a hydrogen atom. 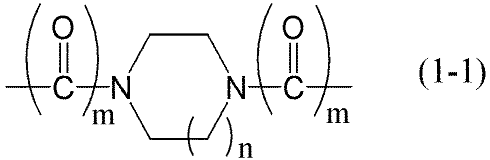 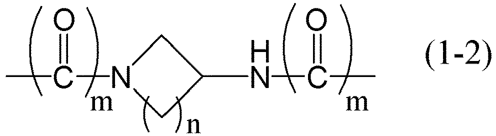 wherein m represents 0 or 1, and n represents 1 or 2.

Specific examples of the group represented by formula (1-1) or the group represented by formula (1-2) include the following formula (1-3) to formula (1-16).

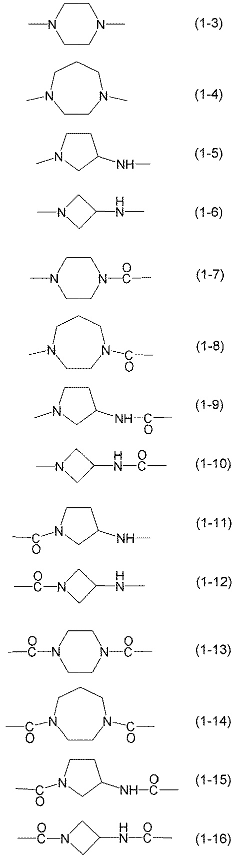

The type and length of the spacer can be selected so as to minimize steric congestion and not to impair the action of the four functional groups described above. As far as this is concerned, the number of carbon atoms of the alkylene group (alkylene chain length) is not limited; for example, the number of carbon atoms of the alkylene group is preferably 1 to 20, and more preferably 2 to 10.

The tetra-functional compound according to the present invention includes the linear compound represented by the following formula (I) and the branched compound represented by the following formula (II).

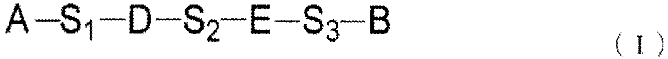

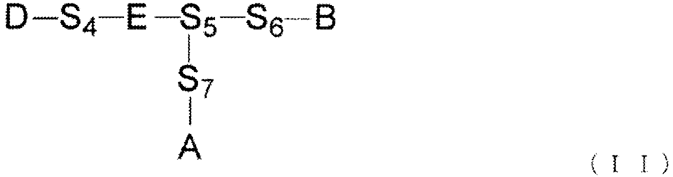

### (II-1) Linear Tetra-functional Compound and Tetra-functional Chemical Probe

In formula (I), the ligand-binding moiety represented by A, the reactive moiety represented by D, the cleavable moiety represented by E, and the biotin tag represented by B are as defined above. In formula (I), the moieties represented by S₁, S₂, and S₃ each independently represent a spacer group.

The spacer group represented by S₁ can be, for example, the group represented by the following formula (5). 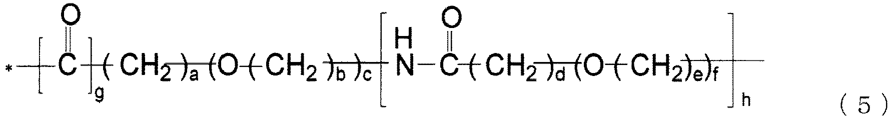 wherein a represents an integer of 0 to 10; and b, c, d, e, f, g, and h each independently represent an integer selected from 0 to 6.

In an embodiment, S₁ is a linear alkylene group having one or more carbon atoms. This corresponds to an embodiment in which g, c, and h are 0, and a is an integer of 1 or more in formula (5). Although a can be an integer of 1 or more, a is preferably an integer of 1 to 30, and more preferably an integer of 1 to 10.

In another embodiment, S₁ is a linear or branched alkylene group having three or more carbon atoms, in the alkylene chain of which, non-adjacent -CH₂- groups are independently replaced with at least one crosslinking group selected from the group consisting of -O-, -OCH₂-, -CH₂O-,-CO-, -NRₐ- (wherein Rₐ represents a hydrogen atom or a C₁₋₆ alkyl group, the same applies below), -CO-NRₐ-, and -NRₐ-CO-, preferably replaced with at least one crosslinking group selected from the group consisting of -O-, -CO-, and -NH-CO-. For example, the number of carbon atoms can be, although not limited to, 3 to 40, and preferably 10 to 35. Specifically, examples include the case in which g is 0, a, b, d, and e are 2, c and f are 4, and h is 1 in formula (5), and the case in which g, c, and f are 0, a is 3, d is 4, and h is 1 in formula (5) .

The spacer groups represented by S₂ and S₃ can be, for example, independently the group represented by the following formula (6). 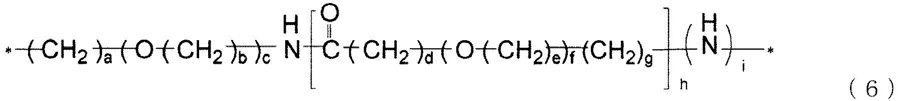 wherein a, b, c, d, e, f, g, and h each independently represent an integer selected from 0 to 6; i represents 0 or 1; however, a, c, h, and i are not 0 at the same time.

In an embodiment, S₂ is a linear alkylene group having one or more carbon atoms, in the alkylene chain of which, non-adjacent -CH₂- groups are independently replaced with at least one crosslinking group selected from the group consisting of -O-, -OCH₂-, -CH₂O-,-CO-, -NRₐ- (wherein Rₐ represents a hydrogen atom or a C₁₋₆ alkyl group, the same applies below) , -CO-NRₐ-, and -NRₐ-CO-, preferably replaced with at least one crosslinking group selected from the group consisting of -O-, -NH-, and -NH-CO-. For example, the number of carbon atoms can be, although not limited to, 1 to 20, and preferably 1 to 5. Specifically, examples include the case in which a and c are 0, d is 3, f and g are 0, and i is 1 in formula (6).

In an embodiment, S₃ is a linear alkylene group having three or more carbon atoms, in the alkylene chain of which, non-adjacent -CH₂- groups are independently replaced with at least one crosslinking group selected from the group consisting of -O-, -OCH₂-, -CH₂O-,-CO-, -NRₐ- (wherein Rₐ represents a hydrogen atom or a C₁₋₆ alkyl group, the same applies below) , -CO-NRₐ-, and -NRₐ-CO-, preferably replaced with at least one crosslinking group selected from the group consisting of -O-, and -NH-. For example, the number of carbon atoms can be, although not limited to, 3 to 20, and preferably 5 to 15. Specifically, examples include the case in which a and b are 2, c is 4, and h and i are 0 in formula (6).

The molecular weight of a linear tetra-functional compound with its ligand-binding moiety having a ligand bound thereto (i.e., tetra-functional chemical probe) is not limited as long as the linear tetra-functional compound is capable of properly coordinating a protein with a high affinity for the ligand; the molecular weight is typically 500 to 3000, preferably 800 to 2000, and more preferably 1000 to 1500. A linear tetra-functional compound having a molecular weight within these ranges can suppress membrane permeability. According to literature discussing the cell membrane permeability of various ligands (Methods Mol Biol. 2015; 1266: 29-53), it is preferred that the number of hydrogen-bond donors be 6 or more, the number of hydrogen-bond acceptors be 15 or more, or the polar surface area be 140 Å2 or more, and more preferably 300 Å2 or more from the standpoint of suppressing membrane permeability. The tetra-functional compound and the tetra-functional chemical probe according to the present invention, described later, have 6 to 9 hydrogen-bond donors, 15 to 28 hydrogen-bond acceptors, and a polar surface area of 300 Å2 or more. In recent years, it has also been believed that membrane permeability depends on molecular size rather than molecular weight. There are examples of cyclic peptides actually measured in the case of medium molecules. It is known that membrane permeability is attenuated at a molecular size of 800 Å or more and extremely attenuated at a molecular size of 1000 Å or less. The tetra-functional compound and the tetra-functional chemical probe according to the present invention, described later, have a molecular size of 900 Å or more. According to the literature mentioned above, membrane permeability is good with the number of rotatable bonds being 10 or less. More specifically, the number of rotatable bonds from the standpoint of suppressing membrane permeability is believed to be preferably 10 or more, and more preferably 30 or more. The tetra-functional compound and the tetra-functional chemical probe according to the present invention have 33 to 60 rotatable bonds. The thus-designed tetra-functional chemical probe according to the present invention exhibits suppressed permeability for cell membrane and does not allow membrane permeation; thus, the tetra-functional chemical probe can efficiently coordinate to a membrane protein.

The following describes a method for producing, among the linear tetra-functional compounds, a compound whose ligand-binding moiety is an N-hydroxysuccinimide ester group (NHS ester group), taking compound (CPA-306) (see Production Example 15) as an example. With the compound whose ligand-binding moiety is an NHS ester group, an antibody probe capable of recognizing and binding to a specific antigen as a target protein can be prepared by binding an antibody to the ligand-binding moiety.

### Method for Producing Linear Tetra-functional Compound Whose Ligand-binding Moiety is NHS Ester Group (Example)

1] 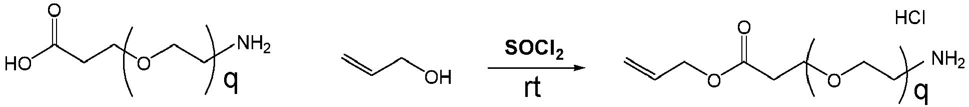
2] 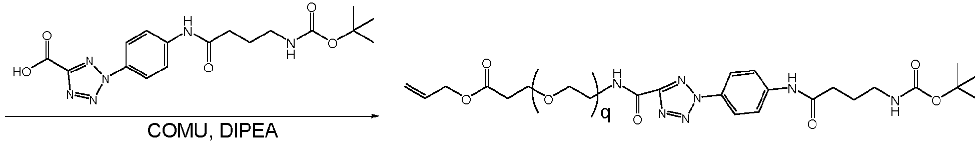
3] 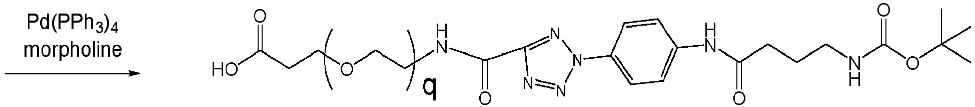
4] 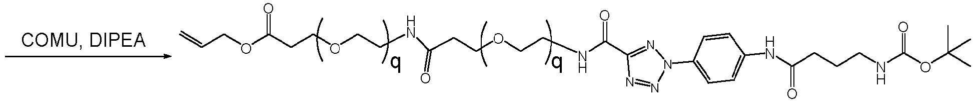
5] 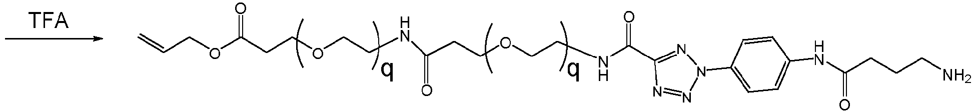
6] 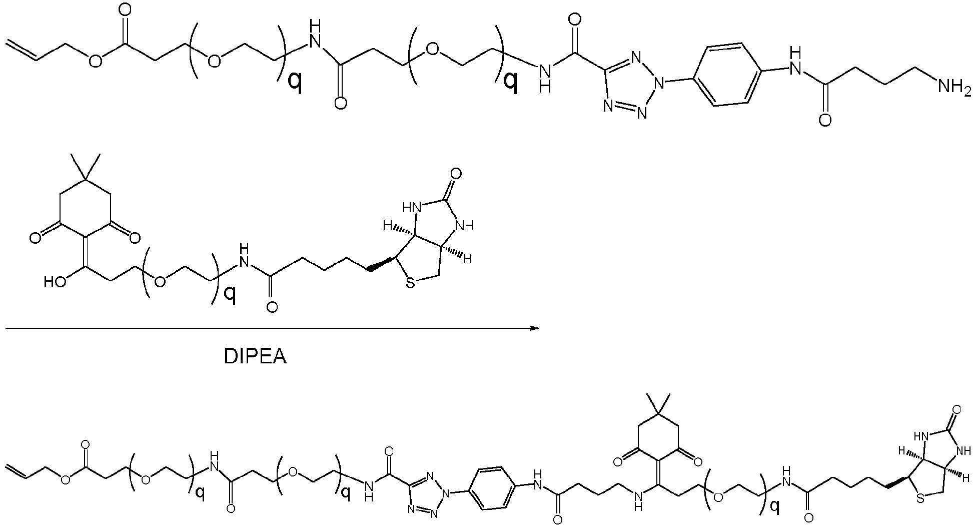
7] 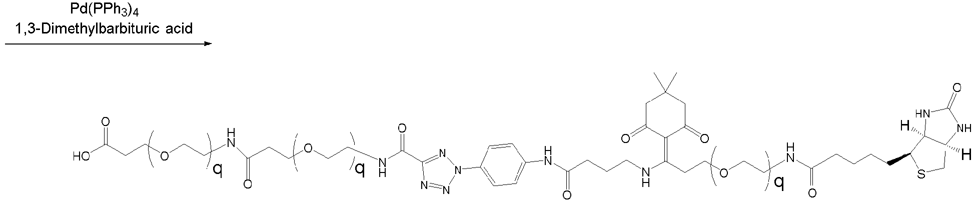
8] 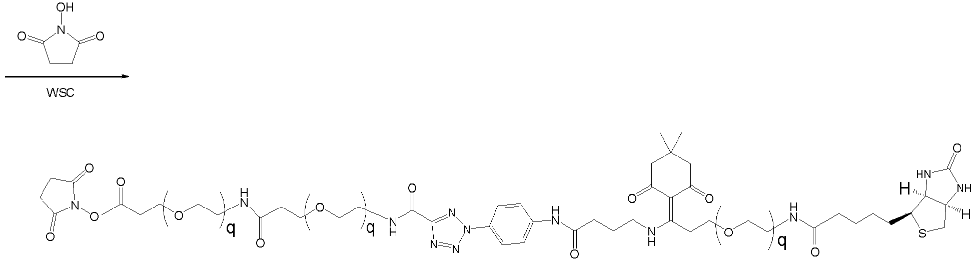 wherein q independently represents an integer of 0 to 6.

### First Step

This step can be performed by stirring PEG-NH₂ and allyl alcohol in the presence of thionyl chloride without a solvent or in a solvent inert to the reaction, typically for 0.1 hours to 5 days at a temperature within the range of 0°C to a temperature reaching heating reflux.

### Second Step

This step can be performed by stirring the compound obtained in the first step and 2-aryl-5-carboxytetrazole (ACT) in the presence of N-[1-(cyano-2-ethoxy-2-oxoethylideneaminooxy)dimethylamino(morpholino)]uronium hexafluorophosphate (COMU) and an amine in a solvent inert to the reaction, typically for 0.1 hours to 5 days at a temperature within the range of 0°C to a temperature reaching heating reflux.

### Third Step: Deprotection

This step can be performed by stirring the compound obtained in the second step in the presence of morpholine and a palladium compound in a solvent inert to the reaction, typically for 0.1 hours to 5 days at a temperature within the range of 0°C to a temperature reaching heating reflux.

### Fourth Step: Side Chain Extension

This step can be performed by stirring the compounds obtained in the first and third steps in the presence of COMU and an amine in a solvent inert to the reaction, typically for 0.1 hours to 5 days at a temperature within the range of 0°C to a temperature reaching heating reflux.

### Fifth Step: Deprotection

This step can be performed by stirring the compound obtained in the fourth step and a deprotecting agent in a solvent inert to the reaction, typically for 0.1 hours to 5 days at a temperature within the range of 0°C to a temperature reaching heating reflux.

### Sixth Step

This step can be performed by stirring the compound obtained in the fifth step and a deprotecting compound in the presence of an amine in a reaction solvent inert to the reaction, typically for 0.1 hours to 5 days at a temperature within the range of 0°C to a temperature reaching heating reflux.

### Seventh Step: Deprotection

This step can be performed by stirring the compound obtained in the sixth step in the presence of 1,3-dimethylbarbituric acid and a palladium compound in a solvent inert to the reaction, typically for 0.1 hours to 5 days at a temperature within the range of 0°C to a temperature reaching heating reflux.

### Eighth Step

This step can be performed by stirring the compound obtained in the seventh step and N-hydroxysuccinimide in the presence of carbodiimide (WSC) in a solvent inert to the reaction, typically for 0.1 hours to 5 days at a temperature within the range of 0°C to a temperature reaching heating reflux.

A desired antibody can be attached to the ligand-binding moiety (NHS ester group) of the thus-prepared linear tetra-functional compound in accordance with a known method, thereby producing a linear tetra-functional chemical probe according to the present invention. An example of the methods for binding a desired antibody to the NHS ester group is described below, although the method is not limited to this example.

### Method for Labelling Antibody with Tetra-functional Compound

The concentration of a desired antibody having an affinity for a target protein is adjusted with a commonly used NHS labeling buffer, and the tetra-functional compound according to the present invention is added thereto, followed by a reaction at a temperature of 0 to 37°C for about 5 to 60 minutes. Subsequently, a lysine solution is added, and the mixture is reacted at 0 to 37°C for about 5 to 60 minutes. Accordingly, a linear tetra-functional chemical probe containing the tetra-functional compound according to the present invention having a desired antibody bound to its ligand-binding moiety (NHS ester group)(labeled antibody) can be obtained.

Of the linear tetra-functional compounds, compounds whose ligand-binding moiety is a ligand-reactive group other than the NHS ester group can be produced with a desired ligand directly bound to the ligand-binding moiety (i.e., tetra-functional chemical probe). The following describes an example of the methods for producing these compounds.

### Method for Producing Linear Tetra-functional Chemical Probe

### (Example)

### First Step

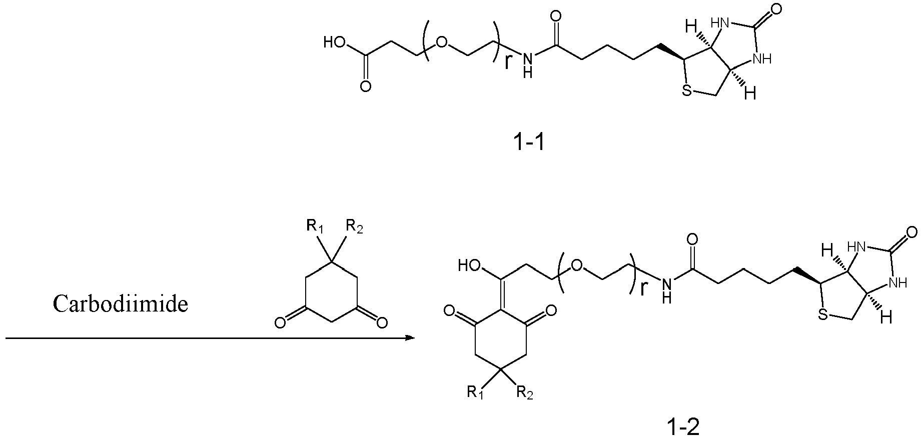

wherein r represents an integer of 1 to 6; and R₁ and R₂ are as defined above.

This step can be performed by stirring compound 1-1, carbodiimide, and cyclohexane-1,3-dione in the presence of N,N-dimethyl-4-aminopyridine (DMAP) in a solvent inert to the reaction, typically for 0.1 hours to 5 days at a temperature within the range of 0°C to a temperature reaching heating reflux.

### Second and Third Steps

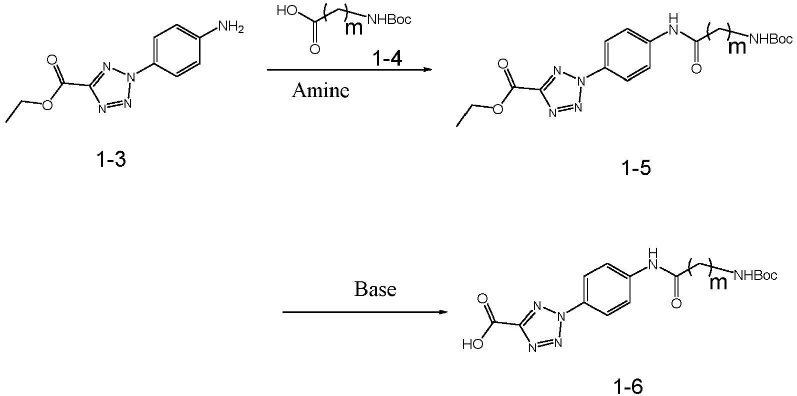

wherein m represents an integer of 1 to 6.

### Second Step

This step can be performed by stirring compound 1-3 and compound 1-4 in the presence of COMU and an amine in a solvent inert to the reaction, typically for 0.1 hours to 5 days at a temperature within the range of 0°C to a temperature reaching heating reflux.

### Third Step

This step can be performed by stirring compound 1-5 in the presence of a base in a solvent inert to the reaction, typically for 0.1 hours to 5 days at a temperature within the range of 0°C to a temperature reaching heating reflux.

### Fourth Step

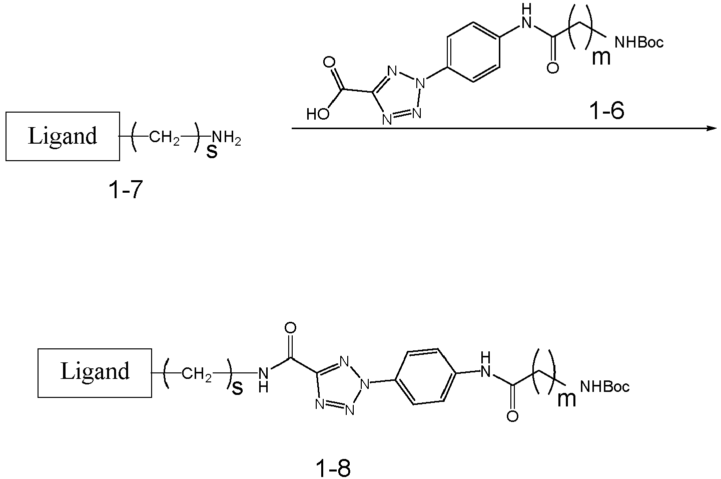

wherein s represents an integer of 1 to 10; and m is as defined above.

This step can be performed by stirring compound 1-7 and compound 1-6, obtained in the third step, in the presence of HATU and an amine in a solvent inert to the reaction, typically for 0.1 hours to 5 days at a temperature within the range of 0°C to a temperature reaching heating reflux.

### Fifth Step

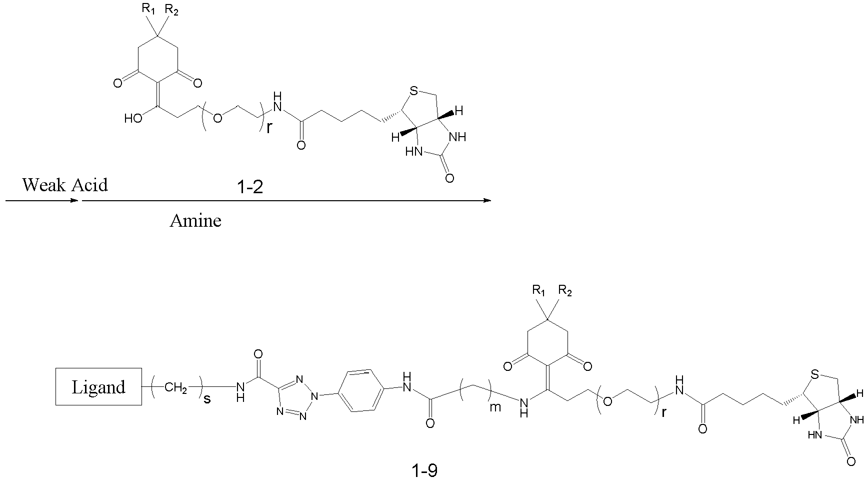

wherein m, r, s, R₁, and R₂ are as defined above.

This step can be performed by stirring compound 1-8, obtained in the fourth step, in the presence of a weak acid in a solvent inert to the reaction, typically for 0.1 hours to 5 days at a temperature within the range of 0°C to a temperature reaching heating reflux to perform a concentration operation, and stirring the concentrated product in the presence of compound 1-2, obtained in the first step, and an amine in a solvent inert to the reaction, typically for 0.1 hours to 5 days at a temperature within the range of 0°C to a temperature reaching heating reflux. The weak acid for use in this reaction includes formic acid, acetic acid, trifluoroacetic acid, citric acid, and oxalic acid. The amine for use in this reaction includes diisopropylethylamine, and triethylamine.

The thus-produced compound 1-9 contains the tetra-functional compound according to the present invention having a desired ligand bound to its ligand-binding moiety, and thus can be used as a tetra-functional chemical probe. Examples of the ligand as used here include, although not limited to, those described above, except for antibodies.

### (II-2) Branched Tetra-functional Compound and Tetra-functional Chemical Probe

In formula (II) above, the ligand-binding moiety represented by A, the reactive moiety represented by D, the cleavable moiety represented by E, and the biotin tag represented by B are as defined above. In formula (II), the sites represented by S₄, S₅, S₆, and S₇ independently represent a spacer group.

The spacer group represented by S₄ is, for example, the group represented by the following formula (7). 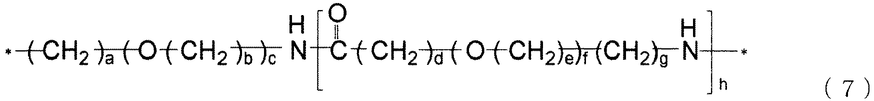 wherein a, b, c, d, e, f, g, h, and i independently represent an integer selected from 0 to 6; however, a, c, and i are not 0 at the same time, and d, f, and g are not 0 at the same time.

In an embodiment, S₄ is a linear alkylene group having three or more carbon atoms, in the alkylene chain of which, non-adjacent -CH₂- groups are independently replaced with at least one crosslinking group selected from the group consisting of -O-, -OCH₂-, -CH₂O-, -CO-, -NRₐ- (wherein Rₐ represents a hydrogen atom or a C₁₋₆ alkyl group, the same applies below) , -CO-NRₐ-, and -NRₐ-CO-, preferably replaced with at least one crosslinking group selected from the group consisting of -O- and -NH-. For example, the number of carbon atoms can be, although not limited to, 1 to 20, preferably 1 to 15, and more preferably 3 to 10. Specifically, examples include the case in which a is 2, b is 2, c is 2, h is 0, and i is 0 in the formula above.

The spacer group represented by S₅ is, for example, the groups represented by the following formulas (8) to (10). 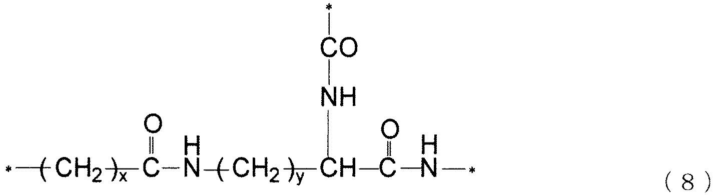 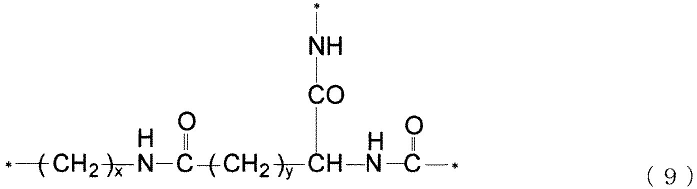 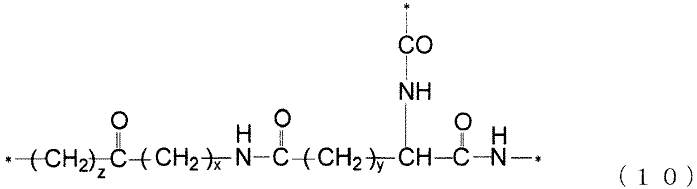 wherein w, x, y, and z each independently represent an integer selected from 0 to 6; however, x and y are not 0 at the same time.

In an embodiment, S₅ is a linear alkylene group having three or more carbon atoms (with substituents), in the alkylene chain of which, non-adjacent -CH₂- groups are independently replaced with at least one crosslinking group selected from the group consisting of -O-, -OCH₂-, -CH₂O-,-CO-, -NRₐ- (wherein Rₐ represents a hydrogen atom or a C₁₋₆ alkyl group, the same applies below), -CO-NRₐ-, and -NRₐ-CO-, preferably replaced with at least one crosslinking group selected from the group consisting of -CO-, -NH-, -CO-NH-, and -NH-CO-. For example, the number of carbon atoms can be, although not limited to, 3 to 20, preferably 3 to 15, and more preferably 5 to 15. Specifically, examples include the case in which x and y are 4 in formula (8).

The spacer group represented by S₆ is, for example, the group represented by the following formula. 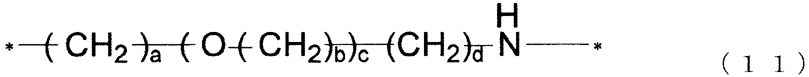 wherein a, b, c, and d each independently represent an integer selected from 0 to 6; however, a and c are not 0 at the same time.

In an embodiment, S₆ is a linear alkylene group having three or more carbon atoms, in the alkylene chain of which, non-adjacent -CH₂- groups are independently replaced with at least one crosslinking group selected from the group consisting of -O-, -OCH₂-, -CH₂O-, -CO-, -NRₐ- (wherein Rₐ represents a hydrogen atom or a C₁₋₆ alkyl group, the same applies below) , -CO-NRₐ-, and -NRₐ-CO-, preferably replaced with at least one crosslinking group selected from the group consisting of -O-, and -NH-. For example, the number of carbon atoms can be, although not limited to, 3 to 20, and preferably 5 to 15. Specifically, examples include the case in which a is 3, b is 2, c is 3, and d is 1 in formula (11).

The spacer group represented by S₇ is, for example, the group represented by the following formula. 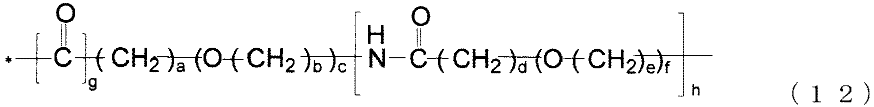 wherein a, b, c, d, e, f, g, and h each independently represent an integer selected from 0 to 6; however, a and c are not 0 at the same time, and a, c, g, and h are not 0 at the same time.

In an embodiment, S₇ is a linear alkylene group having three or more carbon atoms, in the alkylene chain of which, non-adjacent -CH₂- groups are independently replaced with at least one crosslinking group selected from the group consisting of -O-, -OCH₂-, -CH₂O-,-CO-, -NRₐ- (wherein Rₐ represents a hydrogen atom or a C₁₋₆ alkyl group, the same applies below) , -CO-NRₐ-, and -NRₐ-CO-, preferably replaced with at least one crosslinking group selected from the group consisting of -O-, -CO-, and -NH-CO-. For example, the number of carbon atoms can be, although not limited to, 3 to 40, preferably 3 to 30, and more preferably 3 to 20. Specifically, examples include the case in which g is 0, a is 5, c is 0, d is 2, f is 0, and h is 1 in the formula above.

In another embodiment, the spacer represented by S₇ is, for example, the group represented by the following formula. 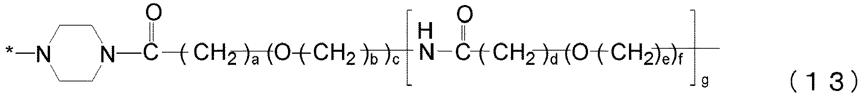 wherein a, b, c, d, e, f, and g each independently represent an integer selected from 0 to 6; however, a and c are not 0 at the same time, and a, c, and h are not 0 at the same time.

In another embodiment, S₇ is a linear alkylene group having three or more carbon atoms, in the alkylene chain of which, non-adjacent -CH₂ -groups are independently replaced with at least one crosslinking group selected from the group consisting of -O-, - OCH₂-, -CH₂O-, -CO-, -NRₐ- (wherein Rₐ represents a hydrogen atom or a C₁₋₆ alkyl group, the same applies below) , -CO-NRₐ-, -NRₐ-CO-, a group represented by the following formula (1-1), and a group represented by the following formula (1-2), preferably replaced with at least one crosslinking group selected from the group consisting of -O-, -CO-, -NH-CO-, a group represented by the following formula (1-3), a group represented by the following formula (1-7), and a group represented by the following formula (1-15). For example, the number of carbon atoms can be, although not limited to, 3 to 40, preferably 3 to 30, and more preferably 3 to 20. Specifically, examples include the case in which g is 0, a is 5, c is 0, d is 2, f is 0, and h is 1 in formula (13). 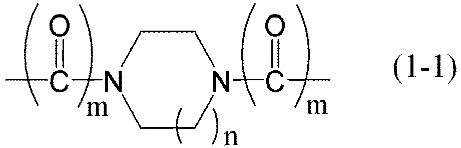 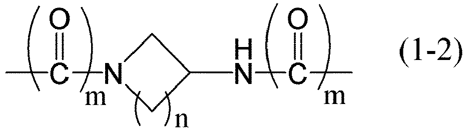 wherein m represents 0 or 1, and n represents 1 or 2.

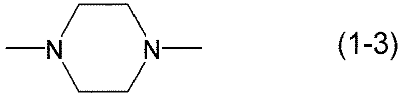

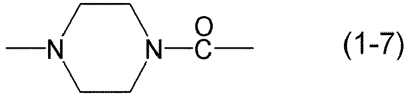

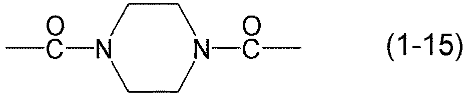

The molecular weight of a branched tetra-functional compound with its ligand-binding moiety having a ligand bound thereto (i.e., tetra-functional chemical probe) is not limited as long as the branched tetra-functional compound is capable of properly coordinating a protein with a high affinity for the ligand; the molecular weight is typically 500 to 3000, preferably 800 to 2000, and more preferably 1000 to 1500. A branched tetra-functional compound having a molecular weight within these ranges can suppress membrane permeability. According to literature discussing the cell membrane permeability of various ligands (Methods Mol Biol. 2015; 1266: 29-53), it is preferred that the number of hydrogen-bond donors be 6 or more, the number of hydrogen-bond acceptors be 15 or more, or the polar surface area be 140 Å2 or more, and more preferably 300 Å2 or more from the standpoint of suppression membrane permeability. The tetra-functional compound and the tetra-functional chemical probe according to the present invention, described later, have 6 to 9 hydrogen-bond donors, 15 to 28 hydrogen-bond acceptors, and a polar surface area of 300 Å or more. In recent years, it has also been believed that membrane permeability depends on molecular size rather than molecular weight. There are examples of cyclic peptides actually measured in the case of medium molecules. It is known that membrane permeability is attenuated at a molecular size of 800 Å or more and extremely attenuated at a molecular size of 1000 Å or less. The tetra-functional compound and the tetra-functional chemical probe according to the present invention, described later, have a molecular size of 900 Å or more. According to the literature mentioned above, membrane permeability is good with the number of rotatable bonds being 10 or less. More specifically, the number of rotatable bonds from the standpoint of suppressing membrane permeability is believed to be preferably 10 or more, and more preferably 30 or more. The tetra-functional compound and the tetra-functional chemical probe according to the present invention have 33 to 60 rotatable bonds. The thus-designed tetra-functional chemical probe according to the present invention exhibits suppressed permeability for cell membrane and does not allow membrane permeation; thus, the tetra-functional chemical probe can efficiently coordinate to a membrane protein.

The following describes a method for producing, among the branched tetra-functional compounds, a compound whose ligand-binding moiety is an N-hydroxysuccinimide ester group (NHS ester group), taking compound (CPA-306) (see Production Example 15) as an example. With the compound whose ligand-binding moiety is an NHS ester group, an antibody probe capable of recognizing and binding to a specific antigen as a target protein can be prepared by binding an antibody to the ligand-binding moiety.

### Method for Producing Branched Tetra-functional Compound Whose Ligand-binding Moiety is NHS Ester Group (Example)

The reaction process is broadly divided into five parts for convenience of explanation below. 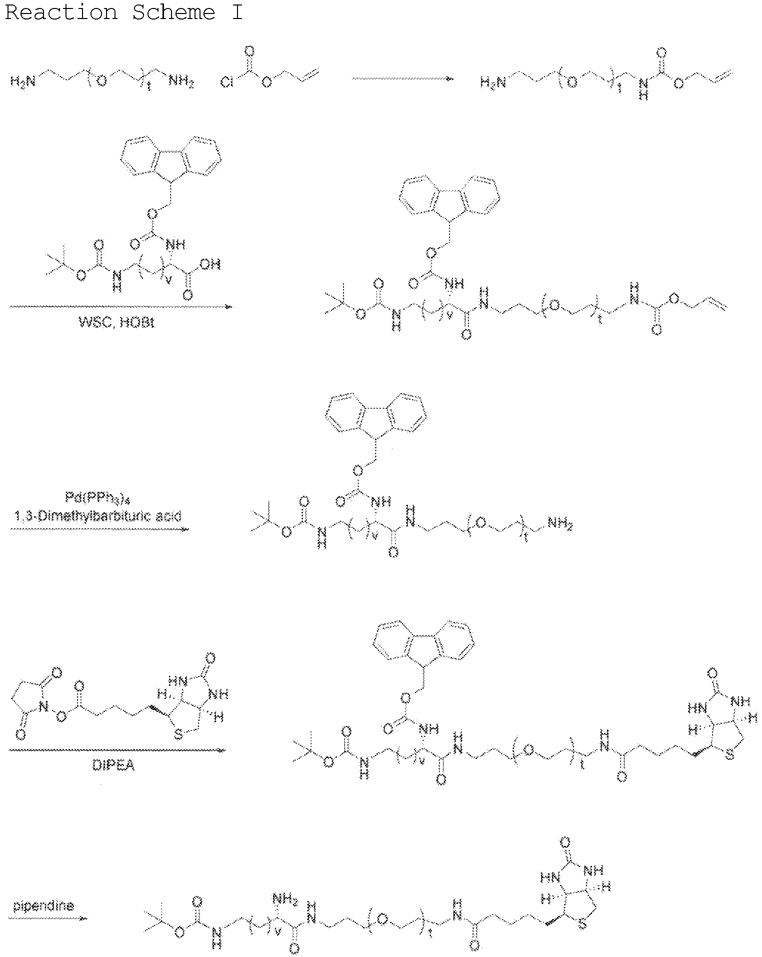 wherein t represents an integer of 0 to 6, and v represents an integer of 1 to 5.

### Frist Step

This step can be performed by stirring a diamine and an acid chloride in a solvent inert to the reaction, typically for 0.1 hours to 5 days at a temperature within the range of 0°C to a temperature reaching heating reflux.

### Second Step

This step can be performed by stirring the diamine to which a protective group is attached in the first step and a linker having a branched amine in the presence of carbodiimide (WSC) and 1-hydroxybenzotriazole (HOBt) in a solvent inert to the reaction, typically for 0.1 hours to 5 days at a temperature within the range of 0°C to a temperature reaching heating reflux.

### Third Step

This step can be performed by stirring the compound obtained in the second step in the presence of 1,3-dimethylbarbituric acid and a palladium compound in a solvent inert to the reaction, typically for 0.1 hours to 5 days at a temperature within the range of 0°C to a temperature reaching heating reflux.

### Fourth Step

This step can be performed by stirring the compound obtained in the third step and a biotin compound in the presence of an amine in a solvent inert to the reaction, typically for 0.1 hours to 5 days at a temperature within the range of 0°C to a temperature reaching heating reflux.

### Fifth Step

This step can be performed by stirring the compound obtained in the third step in the presence of piperidine in a solvent inert to the reaction, typically for 0.1 hours to 5 days at a temperature within the range of 0°C to a temperature reaching heating reflux. 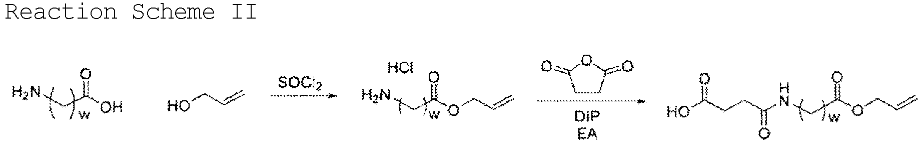 wherein w represents an integer of 0 to 6.

### First Step

This step can be performed by stirring 1-aminoalkyl carboxylic acid and allyl alcohol in the presence of thionyl chloride in a solvent inert to the reaction, typically for 0.1 hours to 5 days at a temperature within the range of 0°C to a temperature reaching heating reflux.

### Second Step

This step can be performed by stirring 1-aminoalkyl carboxylic acid obtained in the first step and alkyldicarboxylic anhydride in the presence of an amine in a solvent inert to the reaction, typically for 0.1 hours to 5 days at a temperature within the range of 0°C to a temperature reaching heating reflux. Examples of alkyldicarboxylic anhydride for use in the reaction include, although not limited to, succinic anhydride, glutaric anhydride, and adipic anhydride. 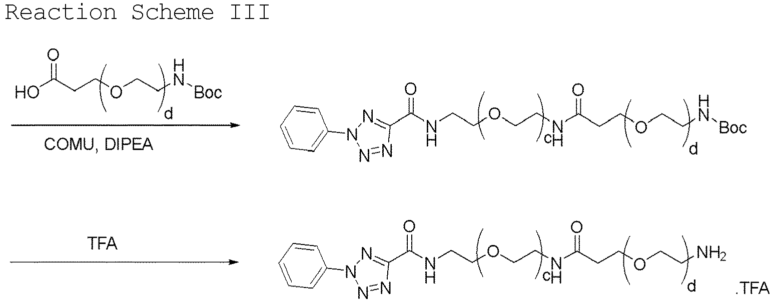 wherein c and d each represent an integer of 0 to 6.

### First Step

This step can be performed by stirring
a diamine protected by BOC and a carboxylic acid containing tetrazole in the presence of an amine and HATU in a reaction solvent inert to the reaction, typically for 0.1 hours to 5 days at a temperature within the range of 0°C to a temperature reaching heating reflux.

### Second Step

This step can be performed by stirring the compound obtained in the first step and a deprotecting agent in a solvent inert to the reaction, typically for 0.1 hours to 5 days at a temperature within the range of 0°C to a temperature reaching heating reflux.

### Third Step

This step and the fourth step are optional steps for further extending the linker length. This step can be performed by stirring the compound obtained in the second step and aminocarboxylic acid whose amino group is protected by BOC in the presence of an amine and COMU in a solvent inert to the reaction, typically for 0.1 hours to 5 days at a temperature within the range of 0°C to a temperature reaching heating reflux.

### Fourth Step

This step can be performed by stirring the compound obtained in the third step and a deprotecting agent in a solvent inert to the reaction, typically for 0.1 hours to 5 days at a temperature within the range of 0°C to a temperature reaching heating reflux. 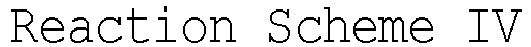 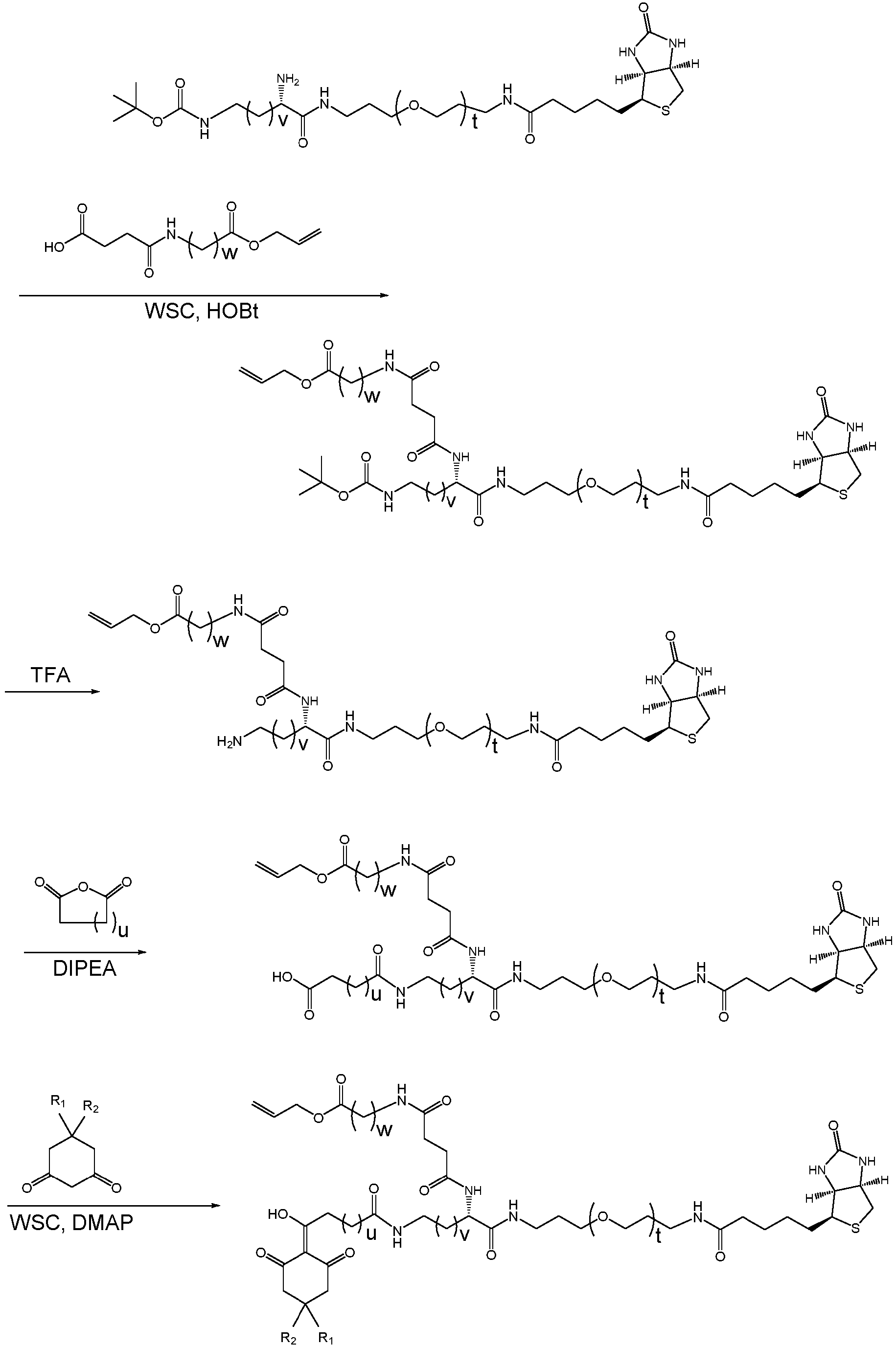 wherein t and u each independently represent an integer of 0 to 6, and v, w, R₁, and R₂ are as defined above.

### First Step

This step can be performed by stirring the compounds obtained in reaction schemes II and III in the presence of a water-soluble carbodiimide (WSC) and 1-hydroxybenzotriazole (HOBt) in a solvent inert to the reaction, typically for 0.1 hours to 5 days at a temperature within the range of 0°C to a temperature reaching heating reflux.

### Second Step

This step can be performed by stirring the compound obtained in the first step and a deprotecting agent in a solvent inert to the reaction, typically for 0.1 hours to 5 days at a temperature within the range of 0°C to a temperature reaching heating reflux.

### Third Step

This step can be performed by stirring the compound obtained in the second step and alkyldicarboxylic anhydride in the presence of an amine in a solvent inert to the reaction, typically for 0.1 hours to 5 days at a temperature within the range of 0°C to a temperature reaching heating reflux.

### Fourth Step

This step can be performed by stirring the compound obtained in the third step and cyclohexane dione in the presence of a water-soluble carbodiimide (WSC) and N,N-dimethyl-4-aminopyridine (DMAP) in a solvent inert to the reaction, typically for 0.1 hours to 5 days at a temperature within the range of 0°C to a temperature reaching heating reflux.

### Reaction Scheme V

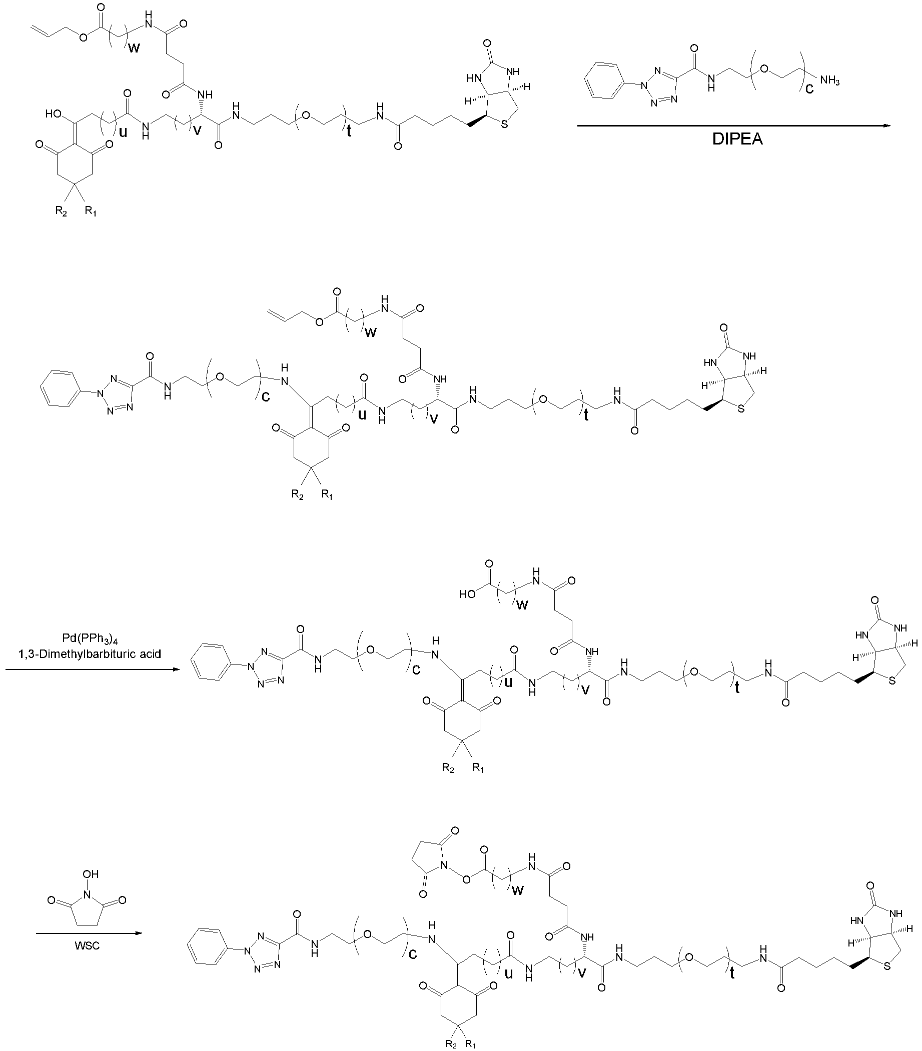

wherein c, u, v, w, t, R₁, and R₂ are as defined above.

### First Step

This step can be performed by stirring the compounds obtained in reaction schemes III and IV in the presence of an amine in a solvent inert to the reaction, typically for 0.1 hours to 5 days at a temperature within the range of 0°C to a temperature reaching heating reflux.

### Second Step

This step can be performed by stirring the compound obtained in the first step in the presence of a palladium compound and 1,3-dimethylbarbituric acid in a solvent inert to the reaction, typically for 0.1 hours to 5 days at a temperature within the range of 0°C to a temperature reaching heating reflux.

### Third Step

This step can be performed by stirring the compound obtained in the second step and N-hydroxysuccinimide in the presence of carbodiimide (WSC) in a solvent inert to the reaction, typically for 0.1 hours to 5 days at a temperature within the range of 0°C to a temperature reaching heating reflux.

### Fourth Step

This step is an optional step for converting the ligand-binding moiety. This step can be performed by stirring the compound obtained in the third step and 6-(piperazin-1-ylmethyl)picolinealdehyde in the presence of an amine (DIPEA) in a solvent inert to the reaction, typically for 0.1 hours to 5 days at a temperature within the range of 0°C to a temperature reaching heating reflux.

A desired antibody can be attached to the ligand-binding moiety (NHS ester group) of the thus-prepared branched tetra-functional compound in accordance with a known method, thereby producing a branched tetra-functional chemical probe according to the present invention. An example of the methods for binding a desired antibody to the NHS ester group is the method for labelling an antibody with a tetra-functional compound described above, although the method is not limited to this example.

In each reaction of the reaction schemes above, a product can be used as a reaction solution as is, or as a crude product, in the subsequent reaction. However, the product can also be isolated from the reaction mixture according to a commonly used method and easily purified by typical separation methods. Examples of typical separation methods include recrystallization, distillation, and chromatography.

The starting material compounds, intermediate compounds, and target compounds (the tetra-functional compound, the tetra-functional chemical probe according to the present invention) in each step include geometric isomers, stereoisomers, optical isomers, and tautomers. These isomers can be separated by a typical optical resolution method, and can also be produced from appropriate optically active starting material compounds.

The tetra-functional compound and the tetra-functional chemical probe according to the present invention are not bound by the synthesis methods shown in the reaction schemes above, and can be produced by methods similar to those methods or other methods. The starting material compounds for use in the production, unless indicated otherwise, may be commercially available compounds, or compounds produced according to a known method *per* se or a method similar to such a known method.

The starting material compounds and the target compound in each step can be used in an appropriate salt form. These salts include, although not limited to, acid addition salts and salts with bases depending on the type of substituent. Examples of acids that form such acid addition salts include inorganic acids (e.g., hydrochloric acid, hydrobromic acid, nitric acid, sulfuric acid, and phosphoric acid); and organic acids (e.g., methanesulfonic acid, p-toluenesulfonic acid, acetic acid, trifluoroacetic acid, citric acid, tartaric acid, maleic acid, fumaric acid, malic acid, and lactic acid). Examples of bases that form salts include inorganic bases (e.g., sodium hydroxide, potassium hydroxide, calcium hydroxide, sodium carbonate, potassium carbonate, sodium hydrogen carbonate, and potassium hydrogen carbonate); and organic bases (e.g., methylamine, diethylamine, trimethylamine triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, and choline); and ammonium salts. Salts may also be formed with, for example, amino acids such as lysine, arginine, aspartic acid, or glutamic.

### (II) Method for Detecting Target Protein

A target protein in a sample can be detected by using the tetra-functional chemical probe according to the present invention described above. A target protein can be detected by using the tetra-functional chemical probe according to the present invention by the method including the following steps, although the method is not limited thereto.
(1) The step of reacting the tetra-functional chemical probe with cells or tissues in a sample to bind the ligand of the tetra-functional chemical probe to a target protein.
(2) The step of forming a covalent bond between the reactive moiety (D) of the tetra-functional chemical probe and the target protein.
(3) The step of purifying a fraction containing the target protein bound to the tetra-functional chemical probe.
(4) The step of binding the biotin tag of the tetra-functional chemical probe to avidin to form a conjugate of the tetra-functional chemical probe and the avidin.
(5) The step of cleaving the formed conjugate of the tetra-functional chemical probe and the avidin at the cleavable moiety of the tetra-functional chemical probe.
(6) The step of detecting or identifying the target protein.

The following describes each step.

### (1) Step of Binding Ligand to Protein

Although there is no limitation, this step can be performed as follows. Cells expressing a target protein are cultured in a 5% CO₂ atmosphere at 37°C. After the cells are confirmed to have become confluent, the tetra-functional chemical probe according to the present invention adjusted to a desired concentration with DMSO is added thereto, and the cells are allowed to stand in a 5% CO₂ atmosphere at 37°C for about 30 to 60 minutes.

### (2) Step of Forming Covalent Bond between Reactive Moiety (D) of Tetra-functional Chemical Probe and Target Protein

Although there is no limitation, this step can be performed by performing irradiation by using an UV crosslinker at a wavelength of 254 to 365 nm, preferably 302 nm, for 1 second to 10 minutes, preferably 30 seconds to 5 minutes, and more preferably 1 minute.

### (3) Step of Purifying Fraction Containing Target Protein Bound to Tetra-functional Chemical Probe

Although there is no limitation, this step can be performed, for example, by the following method. First, a tris homogenate buffer is added to cells, and the mixture is homogenized, followed by centrifugation. The precipitate is separated, and a sodium carbonate buffer is added thereto, followed by homogenization, and then incubation. The precipitate is separated by centrifugation to purify and collect a fraction containing a target protein bound to the tetra-functional chemical probe (e.g., membrane fraction).

### (4) Step of Binding Biotin Tag of Tetra-functional Chemical Probe to Avidin to Form Conjugate of Tetra-functional Chemical Probe and Avidin

Although there is no limitation, this step can be performed, for example, as follows. For example, an 8M urea-containing RIPA buffer is added to the collected fraction, and the fraction is dissolved in the buffer by using an ultrasound disintegrator. The supernatant is separated by centrifugation, and then, for example, streptavidin magnetic particles are added thereto. Accordingly, the biotin tag in the chemical probe binds to avidin, thereby forming a conjugate of the tetra-functional chemical probe and the avidin. Additionally, the conjugate of the tetra-functional chemical probe and the avidin can be purified and collected by performing magnetic separation according to a conventional method and discarding the supernatant.

### (5) Step of Cleaving Formed Conjugate of Tetra-functional Chemical Probe and Avidin at Cleavable Moiety of Tetra-functional Chemical Probe

Although there is no limitation, this step can be performed, for example, by adding SDS (sodium dodecyl sulfate) and an aqueous hydrazine solution to the conjugate of the tetra-functional chemical probe and the avidin (magnetic particle conjugate) collected in the previous step, and allowing the mixture to stand at room temperature (1 to 30°C) for 30 minutes. This reaction allows the cleavable moiety in the tetra-functional chemical probe to be cleaved, thereby separating the conjugate of the tetra-functional chemical probe and the avidin into the binding domain of the target protein and the biotin-avidin binding domain. The reaction solution is magnetically separated to obtain a supernatant; thus, the binding domain fragment of the target protein can be collected.

### (6) Step of Detecting or Identifying Target Protein

In detecting or identifying the target protein, the binding domain fragment of the previously collected target protein may optionally be purified or hydrolyzed. Specifically, although there is not limitation, for example, dithiothreitol is added to the obtained supernatant, and the mixture is stirred for 15 minutes at 70°C. Subsequently, iodoacetamide is added thereto, and the mixture is allowed to stand at room temperature for 30 minutes. SP3 beads are added to the thus-obtained solution, and then MeCN is added, followed by incubation for 18 minutes. After completion of the incubation, magnetic separation is performed to discard the supernatant. For peptide degradation, for example, a trypsin solution is added to the collected residue, and the mixture is incubated at 37°C for 12 to 16 hours. MeCN is added, and the mixture is incubated for 18 minutes, followed by magnetic separation to discard the supernatant. An aqueous TFA solution is added to the residue, and the mixture is incubated for 3 minutes. Magnetic separation is performed to obtain the supernatant.

The thus-prepared peptide is desalted and re-suspended in a suitable buffer solution for analysis using a high-mass-accuracy mass spectrometer. The mass spectrometer is operated in data-dependent acquisition mode in which the device is automatically started to switch from MS to MS/MS mode for ion signals that exceed a predetermined threshold so as to generate a collision-induced dissociation (CID) spectrum or high-energy collision-dissociation (HCD) spectrum of the peptide. All MS/MS spectra are searched across standard protein databases.

The thus-obtained peptide is analyzed. Any method for analyzing such compounds usable in the art can be used, and a preferable method is mass spectrometry. The method for performing mass spectrometry is well known to those skilled in the art (see, for example, Yates, J. Mass Spect. 33: 1-19 (1998); Kinter and Sherman, Protein Sequencing and Identification Using Tandem Mass Spectrometry, John Wiley and Sons, New York (2000); and Aebersold and Goodlett, Chem. Rev. 101: 269-295 (2001)). For high-resolution polypeptide fragment separation, liquid chromatography ESI-MS/MS or automated LC-MS/MS using capillary reverse-phase chromatography can be used as a separation method (Yates et al., Methods Mol. Biol. 112: 553-569 (1999)). Preferably, data-dependent collision-induced dissociation (CID) or high-energy collision dissociation (HCD) involving dynamic exclusion is used as a selected mass spectrometry (Goodlett et al., Anal. Chem. 72: 1112-1118 (2000)). For such analysis, the mass spectrometer is typically operated in data-dependent acquisition mode in which the device is automatically started to switch from MS to MS/MS mode for ion signals that exceed a predetermined threshold so as to generate a collision-induced dissociation (CID) spectrum or high-energy collision-dissociation (HCD) spectrum of the peptide.

In protein identification, standard algorithms (e.g., SEQUEST, Mascot, X!tandem, and MS Aanda) are used to search all MS/MS spectra across standard protein databases and perform typical filtering for decreasing false-positive protein identification to less than 1%.

In an embodiment, the concentration of a membrane protein in a sample can be quantitatively compared with that of a control sample. This allows for the detection of specific enrichment of a target membrane protein receptor. For this unlabeled mass spectrometry, reverse-phase chromatography immediately before mass spectrometry can be displayed as an MS feature map plotting the feature of retention time relative to the mass/electric charge ratio. As detected by mass spectrometer, the peptides in such a map appear in a clear isotope pattern over a predetermined time, and appear at a defined ion current intensity depending on their amount in the sample. Once peptides have been fragmented and identified by MS/MS analysis, this information can be assigned to specific peptide features on the MS map, and can be combined with semiquantitative data analysis using an open source or commercial algorithms such as MaxQuant (Cox et al., Nature Biotechnology (2008) vol. 26, pp. 1367-1372) or Proteome Discoverer (Thermo Fischer Scientific). MS feature maps of different samples (e.g., sample vs. control) can be superposed and compared to determine the ratio of the amount of peptides. The ratio of stochastically labeled peptides originating from a membrane protein should be about 1. The membrane protein peptides that are specifically captured based on the ligand have a ratio higher than that of the control sample.

In another embodiment, alternative quantification methods based on mass spectrometry can be used, for example, single reaction monitoring (SRM), stable isotope labeling by amino acids in cell culture (SILAC; for example, Nilsson et al., Nat Methods (2010) vol. 7(9), pp. 681-5), mass spectrum data-independent acquisition (SWATH MS; see, for example, Gillet et al. (Targeted Data Extraction of the MS/MS Spectra Generated by Data-independent Acquisition: A New Concept for Consistent and Accurate Proteome Analysis)), and tandem mass tag (TMT; see, for example, Dayon et al. (Relative Quantification of Proteins in Human Cerebrospinal Fluids by MS/MS using 6-Plex Isobaric Tags)).

MS analysis can be replaced with other analysis methods. Such other methods are included in the present invention as a part thereof.

The use of the chemical probe according to the present invention allows for the determination of the site at which a target protein is bound to a ligand by identifying the peptide sequence having a portion of the probe structure added by mass spectrometry. This enables the recognition of the binding site of the target protein to the ligand and thus makes the identification of the target protein more accurate. Additionally, this makes it possible to design a probe with enhanced power for detecting a target protein.

The disclosures of all patent and non-patent documents cited in this specification are incorporated herein by reference in their entirety.

### Examples

The present invention is further described below in detail with reference to Test Examples and Production Examples; however, these do not limit the present invention, and modification may be made without departing from the scope of the present invention.

In the present specification, the following abbreviations may be used.

**Table 1**

| Abbreviation | Term |
|---|---|
| AcOEt | Ethyl acetate |
| AcOH | Acetic acid |
| AcONH₄ | Ammonium acetate |
| COMU | N-[1-(Cyano-2-ethoxy-2-oxoethylideneaminooxy)dimethylamino(morpholino)] uronium hexafluorophosphate |
| Boc | tert-Butoxycarbonyl |
| t-BuOH | tert-Butanol |
| DCM | Dichloromethane |
| DIPEA | N,N-Diisopropylethylamine |
| DMAP | 4-(Dimethylamino)pyridine |
| DMBA | 7,12-Dimethylbenz[a]anthracene |
| DMF | N,N-Dimethylformamide |
| DMSO | Dimethyl sulfoxide |
| EtOH | Ethanol |
| HATU | O-(7-Aza-1H-benzotriazol-1-yl)-N,N,N',N'-tetramethyluronium hexafluorophosphate |
| HCl | Hydrochloric acid |
| H₂O₂ | Hydrogen peroxide |
| HOBt | 1-Hydroxybenzotriazole |
| K₂CO₃ | Potassium carbonate |
| LiOH | Lithium hydroxide |
| MeCN | Acetonitrile |
| MeOH | Methanol |
| N₂H₄ | Hydrazine |
| NaH | Sodium hydride |
| NaHCO₃ | Sodium hydrogen carbonate |
| NaI | Sodium iodide |
| NaOH | Sodium hydroxide |
| NHS | N-Hydroxysuccinimide |
| NMP | N-Methyl-2-pyrrolidone |
| Pd(PPh₃)₄ | Tetrakis(triphenylphosphine)palladium(0) |
| TEA | Triethylamine |
| TFA | Trifluoroacetic acid |
| WSC | 3-Ethyl-1-(3-dimethylaminopropyl)carbodiimide |

In the following Examples, "room temperature" generally refers to about 10 to 35°C. A ratio indicated for a mixed solvent refers to a volume ratio, unless otherwise specified. Unless otherwise specified, % refers to mass%.

Proton nuclear magnetic resonance (¹HNMR) spectra were measured by Fourier transform NMR (using any of Bruker AVANCE III 400 (400 MHz) and Bruker AVANCE III HD (500 MHz)). Spectral splitting patterns measured with tetramethylsilane as a standard are designated as singlet (s), doublet (d), triplet (t), quartet (q), multiplet or more overlapping signals (m), and broad signal (br). Solvents are shown in parentheses.

Mass spectra were measured by an electrospray ionization method (ESI) using Waters ACQUITY H-Class in combination with an SQD mass spectrometer. High-resolution mass spectrometry (HRMS) was performed by an electrospray ionization method (ESI) using a Waters Xevo G2-XS QTof spectrometer. [M+H]⁺ means monoisotopic molecular weight.

For silica gel column chromatography in the Examples, a *Hi-Flash column* (Yamazen Corporation) *or* SNAP Ultra HP-Sphere 25 µm (Biotage) was used. For ODS column chromatography, octadecyl C18 (Yamazen Corporation) or SNAP Ultra C18 (Biotage) was used.

In the Test Examples, anti-CD71 antibody OKT9 produced by Thermo Fisher Scientific Inc. (Cat # 16-0719-85), anti-CD71 antibody DF1513 produced by Abcam (ab212863), His-Tag EGF produced by Oriental Yeast Co., Ltd. (47061000), and Goat anti-mouse IgG produced by Southern Biotech (Cat. 1030-01) were used.

### Production Example 1: Production of Compound (CPI-003)

Compound (CPI-003) was produced according the method shown in the following scheme.

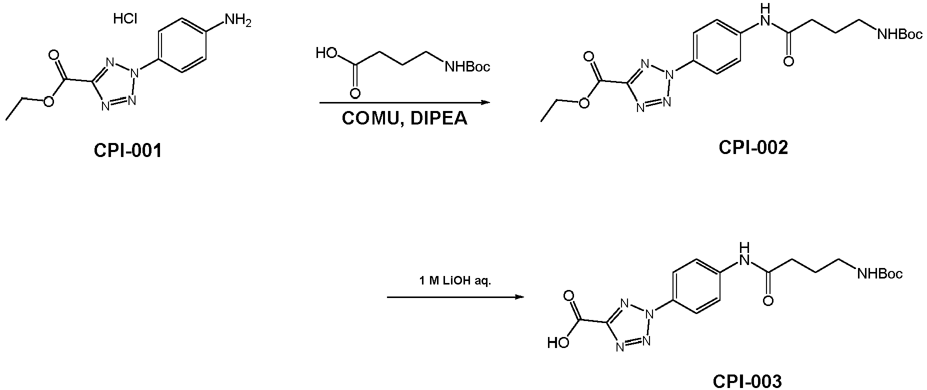

### (1) Production of Compound (CPI-002)

N-(Tert-butoxycarbonyl)-4-aminobutyric acid (0.24 g), compound (CPI-001) shown in the above scheme (0.31 g), COMU (0.55 g), and DIPEA (0.44 ml) were dissolved in DMF (2 mL), followed by stirring at room temperature for 17 hours. The reaction mixture was then concentrated. The residue was purified by silica gel chromatography (ODS, water/MeCN, containing 0.1% AcOH) to give the desired product (CPI-002) (0.45 g).
1H NMR (500 MHz, DMSO-d₆) δ10.31(s, 1H), 8.08 (d, J = 9.0 Hz, 2H), 7.89 (d, J = 9.0 Hz, 2H), 6.86 (t, J = 5.5 Hz, 1H), 4.47 (q, J = 7.0 Hz, 2H), 2.98 (q, J = 6.5 Hz, 2H), 2.36 (t, J = 7.5 Hz, 2H), 1.71 (m, J = 7.2 Hz, 2H), 1.38 (t, J = 7.1 Hz, 12H);
HRMS (ESI) calcd for C₁₉H₂₇N₆O₅ 419.2043 [M+H]⁺, found 419.2054.

### (2) Production of Compound (CPI-003)

A solution of CPI-002 synthesized above (0.45 g) in MeOH (15 ml) and a 0.2 M aqueous LiOH solution (5.9 mL) were added under ice-cooling, followed by stirring for 30 minutes. AcOH (0.30 µL) was added to the reaction mixture, and the solvent was then removed. The residue was purified by silica gel chromatography (ODS, water/MeCN, containing 0.1% AcOH) to give the desired product (0.45 g).
1H NMR (500 MHz, DMSO-d₆) δ 10.30 (s, 1H), 8.06 (d, J = 9.1 Hz, 2H), 7.89 (d, J = 9.1 Hz, 2H), 6.86 (t, J = 5.5 Hz, 1H), 2.98 (q, J = 6.6 Hz, 2H), 2.36 (t, J = 7.4 Hz, 2H), 1.71 (m, J = 7.2 Hz, 2H), 1.38 (s, 9H); HRMS (ESI) calcd for C₁₇H₂₃N₆O₅ 391.1730 [M+H]⁺, found 391.1724.

### Production Example 2: Production of Compound (CPI-005)

Compound (CPI-005) was produced according to the method shown in the following scheme.

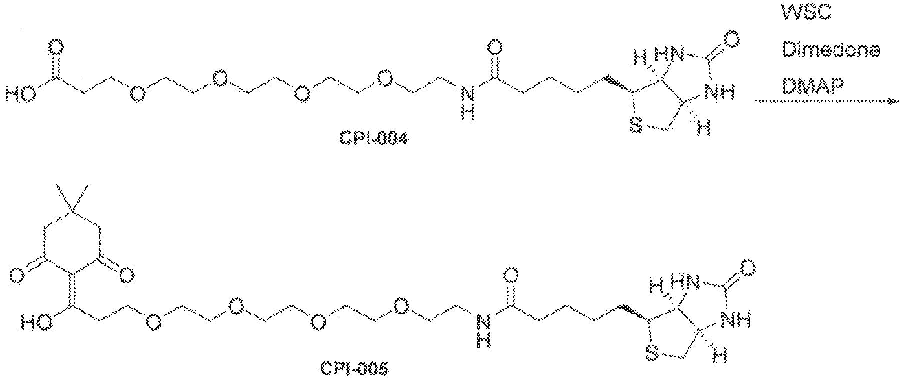

A mixture of dimedone (63 mg), compound (CPI-004) shown above (0.20 g), WSC hydrochloride (82 mg), and DMAP (50 mg) in DMF (3 mL) was stirred at room temperature for 14 hours, and the reaction mixture was then concentrated. The residue was purified by silica gel chromatography (ODS, water/MeCN, containing 0.1% AcOH) to give the desired product (0.14 g).
1H NMR (500 MHz, DMSO-d₆) δ 7.82 (t, J = 5.6 Hz, 1H), 6.41 (br, 1H), 6.35 (br, 1H ), 4.30 (dd, J = 7.9, 5.0 Hz, 1H), 4.14-4.11 (m, 1H), 3.68 (t, J = 6.4 Hz, 2H), 3.52-3.49 (m, 12H), 3.39 (t, J = 5.9 Hz, 2H), 3.23 (t, J = 6.4 Hz, 2H), 3.18 (q, J = 5.7 Hz, 2H), 3.11-3.07 (m, 1H), 2.82 (dd, J= 12.5, 5.1 Hz, 1H), 2.63 (s, 2H), 2.57 (d, J = 12.5 Hz, 1H), 2.33 (s, 2H), 2.06 (t, J = 7.5 Hz, 2H), 1.64-1.57 (m, 1H), 1.54-1.42 (m, 3H), 1.36-1.24 (m, 2H), 1.00 (s, 6H); HRMS (ESI) calcd for C₂₉H₄₈N₃O₉S 614.3111 [M+H]⁺, found 614.3112.

### Production Example 3: Production of Compound (CPI-104)

Compound (CPI-104) was produced according to the method shown in the following scheme.

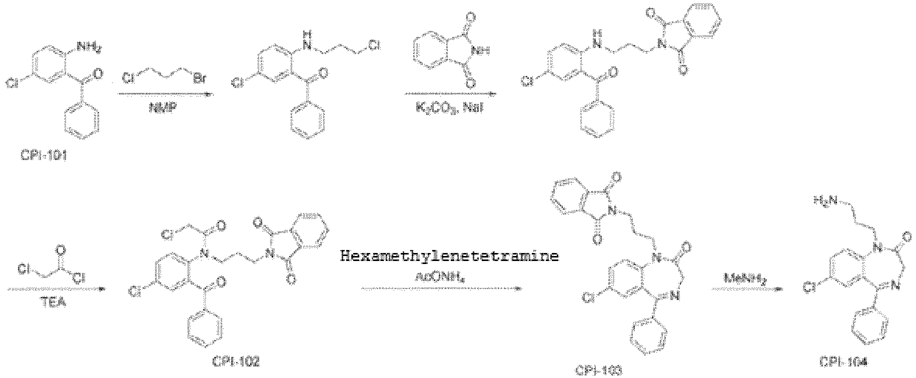

### (1) Production of Compound (CPI-102)

A mixture of compound (CPI-101) shown above (0.21 g) and 1-bromo-3-chloropropane (5.0 mL) in NMP (5 mL) was reacted at 120°C for 6 hours using a microwave reactor. Water was added to the reaction mixture, followed by extraction with AcOEt. The organic layer was washed with saturated saline and then dried over Na₂SO₄, and the solvent was distilled off. The residue was purified by column chromatography (silica gel, hexane/AcOEt) to give an intermediate (0.18 g). A suspension of the intermediate (0.18 g), phthalimide (0.20 g), potassium carbonate (0.19 g), and sodium iodide (14 mg) in DMF (9 mL) was stirred at 70°C for 10 hours. After the solvent was distilled off, water was added thereto, followed by extraction with AcOEt. The organic layer was washed with saturated saline and then dried over Na₂SO₄, and the solvent was distilled off. The residue was purified by column chromatography (silica gel, hexane/AcOEt) to give a compound (0.24 g). The obtained compound (0.24 g) was dissolved in DCM (9 mL), and chloroacetyl chloride (0.15 mL) and triethylamine (0.25 mL) were added thereto, followed by stirring at room temperature for 2 hours. Water was added to the reaction mixture, followed by extraction with DCM. The organic layer was washed with saturated saline and then dried over Na₂SO₄, and the solvent was distilled off. The residue was purified by column chromatography (silica gel, hexane/AcOEt) to give the desired product (0.18 g).
1H NMR (400 MHz, CDCl₃) δ 7.83-7.69 (m, 6H), 7.63-7.59 (m, 2H), 7.55 (d, J= 8.4 Hz, 1H), 7.49-7.45 (m, 3H), 4.15-4.05 (m, 1H), 3.99 (d, J = 13.0 Hz, 1H), 3.87 (d, J = 13.0 Hz, 1H), 3.64 (t, J = 7.5 Hz, 2H), 3.02-2.95 (m, 1H), 1.82-1.75 (m, 2H);
LC-MS: [M+H]⁺= 495.04.

### (2) Production of Compound (CPI-103)

Compound (CPI-102) synthesized above (0.18 g) was dissolved in EtOH (5 mL). Hexamethylenetetramine (0.10 g) and AcONH₄ (56 mg) were added thereto, and the mixture was heated under reflux for 9 hours. Hexamethylenetetramine (51 mg) and AcONH₄ (28 mg) were further added thereto, and the mixture was heated under reflux for 9 hours. Water was added to the reaction mixture, followed by extraction with AcOEt. The organic layer was washed with saturated saline and then dried over Na₂SO₄, and the solvent was distilled off. The residue was purified by column chromatography (silica gel, hexane/AcOEt) to give the desired product (0.14 g).
1H NMR (400 MHz, CDCl₃) δ 7.83-7.79 (m, 2H), 7.73-7.68 (m, 2H), 7.67-7.64 (m, 2H), 7.50-7.40 (m, 4H), 7.31-7.29 (m, 2H), 4.80 (d, J = 10.6 Hz, 1H), 4.38 (dt, J = 14.4, 7.9 Hz, 1H), 3.74 (d, J = 10.6 Hz, 1H), 3.71-3.52 (m, 3H), 1.93 (tt, J = 7.2, 7.2 Hz, 2H); LC-MS: [M+2H] ²⁺ = 458.08.

### (3) Production of Compound (CPI-104)

Methylamine and a 40% MeOH solution (22 µL) were added to a solution (0.5 mL) of compound (CPI-103) synthesized above (10 mg) in MeOH, followed by stirring at room temperature for 2 hours. Methylamine and a 40% MeOH solution (22 µL) were further added thereto, followed by stirring at room temperature for 2 hours. Water was added to the reaction mixture, followed by extraction with AcOEt. The organic layer was washed with saturated saline and then dried over Na₂SO₄, and the solvent was distilled off. The residue was purified by column chromatography (ODS, water/MeCN) to give the desired product (1.6 mg).
1H NMR (400 MHz, CD₃OD) δ 7.66 (d, J = 1.4 Hz, 2H), 7.58-7.45 (m, 6H), 7.24 (t, J = 1.4 Hz, 1H), 4.64 (d, J = 10.6 Hz, 1H), 4.53-4.43 (m, 1H), 3.86 (d, J= 10.7 Hz, 1H), 3.82-3.76 (m, 1H), 2.46-2.43 (m, 2H), 1.73-1.53 (m, 2H); LC-MS: [M+H]⁺= 328.17.

### Production Example 4: Production of Compound (CPP-112)

Compound (CPP-112) was produced according to the method shown in the following scheme.

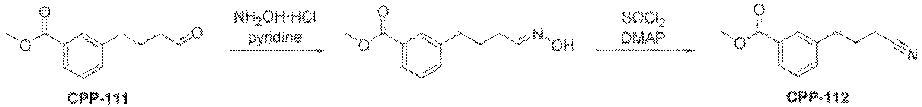

A mixture of compound (CPP-111) shown in the above scheme (0.44 g), hydroxylamine hydrochloride (0.18 g), pyridine (1.0 ml), and EtOH (20 mL) was heated under reflux for 2 hours. The solvent was distilled off, and the residue was acidified with 2M hydrochloric acid, followed by extraction with DCM. The organic layer was washed with saturated saline and then dried over Na₂SO₄. The solvent was distilled off to give a colorless oil crude product (0.51 g). Thionyl chloride (0.31 ml) was added to a solution of DMAP (0.26 g) in DCM (15 mL) under ice-cooling, and the mixture was stirred for 30 minutes. A solution (5 mL) of the crude product obtained in the previous step (0.51 g) in DCM and DMAP (0.39 g) were added thereto under ice-cooling, and the mixture was warmed to room temperature and stirred for 3 days. Water was added to the reaction mixture, followed by extraction with DCM. The organic layer was washed with saturated saline and then dried over Na₂SO₄, and the solvent was distilled off. Purification was performed by column chromatography (silica gel, hexane/AcOEt) to give the desired product (0.30 g).
1H NMR (400 MHz, CDCl₃)δ 7.94-7.89 (m, 1H), 7.87 (s, 1H), 7.42-7.37 (m, 2H), 3.92 (s, 3H), 2.84 (t, J = 7.3 Hz, 2H), 2.34 (t, J = 7.3 Hz, 2H), 2.02 (tt, J = 7.3, 7.3 Hz, 2H);

LC-MS: [M+H]⁺= 204.02.

### Production Example 5: Production of Compound (CPP-117)

Compound (CPP-117) was produced according to the method shown in the following scheme.

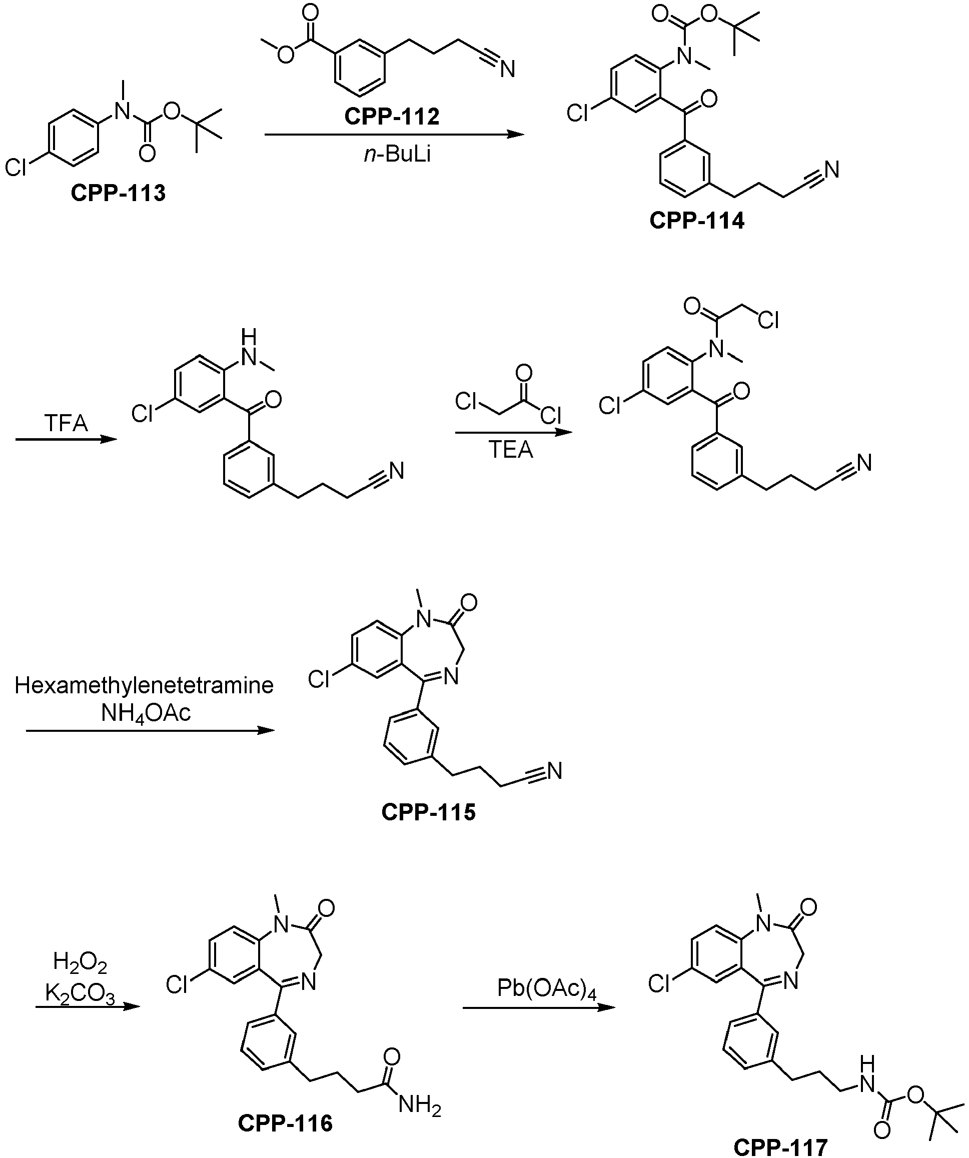

### (1) Production of CPP-114

1.6 M n-butyllithium and an n-hexane solution (0.53 mL) were added to a mixture of compound (CPP-113) shown in the above scheme (100 mg) and THF (3 mL), at -78°C, and the mixture was stirred for 1 hour. A solution (2 mL) of compound (CPP-112) produced in Production Example 4 (84 mg) in THF was added thereto, and the mixture was stirred at -78°C for 5 hours. The mixture was warmed to room temperature over a period of 3 hours and stirred at room temperature for 13 hours. Water was added to the reaction mixture, followed by extraction with AcOEt. The organic layer was washed with saturated saline and then dried over Na₂SO₄, and the solvent was distilled off. The residue was purified by column chromatography (silica gel, hexane/AcOEt) to give the desired product (48 mg).
1H NMR (400 MHz, CDCl₃) δ 7.66-7.36 (m, 6H), 7.31-7.13 (brm, 1H), 3.14-2.92 (brm, 3H), 2.90-2.77 (brs, 2H), 2.34 (t, J = 6.9 Hz, 2H), 2.00 (tt, J = 6.9, 6.9 Hz, 2H), 1.26 (s, 9H);

LC-MS: [M+Na] ⁺= 434.94.

### (2) Production of CPP-115

TFA (0.45 ml) was added to a solution of compound (CPP-114) produced above (48 mg) in DCM (2 mL). The mixture was stirred at room temperature for 2 hours, and the solvent was distilled off. The residue was diluted with DCM (2 mL). TEA (49 µL) and chloroacetyl chloride (28 µL) were added thereto, and the mixture was stirred at room temperature for 3 hours. Water was added to the reaction mixture, followed by extraction with AcOEt. The organic layer was washed with saturated saline and then dried over Na₂SO₄, and the solvent was distilled off. The residue was purified by column chromatography (silica gel, hexane/AcOEt). A mixture of the purified product (38 mg), hexamethylenetetramine (33 mg), AcONH₄ (18 mg), and EtOH (2 mL) was heated under reflux for 6 hours. The solvent was distilled off, and purification was performed by column chromatography (silica gel, hexane/AcOEt) to give the desired product (28 mg).
1H NMR (400 MHz, CDCl₃) δ 7.53 (dd, J = 8.8, 2.5 Hz, 1H), 7.50 (s, 1H), 7.41-7.30 (m, 4H), 7.27 (d, J = 2.5 Hz, 1H), 4.84 (d, J = 10.8 Hz, 1H), 3.77 (d, J = 10.8 Hz, 1H), 3.40 (s, 3H), 2.88-2.77 (m, 2H), 2.36 (t, J = 7.0 Hz, 2H), 2.05-1.97 (m, 2H);

LC-MS: [M+H]⁺= 352.08.

### (3) Production of CPP-116

A mixture of compound (CPP-115) produced above (28 mg), a 30% hydrogen peroxide solution (0.16 mL), K₂CO₃ (11 mg), and DMSO (1 mL) was stirred at room temperature for 2 hours. An aqueous sodium thiosulfate solution was added thereto, and the mixture was stirred, followed by extraction with AcOEt. The organic layer was washed with saturated saline and then dried over Na₂SO₄, and the solvent was distilled off. The residue was purified by column chromatography (silica gel, AcOEt/MeOH) to give the desired product (28 mg) .
1H NMR (400 MHz, CDCl₃) δ 7.52 (dd, J = 8.8, 2.5 Hz, 1H), 7.50-7.46 (brm, 1H), 7.37-7.28 (m, 5H), 5.60-5.18 (brm, 2H), 4.82 (d, J = 10.8 Hz, 1H), 3.77 (d, J = 10.8 Hz, 1H), 3.40 (s, 3H), 2.79-2.65 (m, 2H), 2.23 (t, J = 7.5 Hz, 2H), 2.08-1.91 (m, 2H);
LC-MS: [M+H]⁺= 370.00.

### (4) Production of CPP-117

A mixture of compound (CPP-116) produced above (28 mg), lead tetraacetate (40 mg), and t-BuOH (0.72 mL) was stirred at 80°C for 3 hours. Lead tetraacetate (40 mg) was further added thereto, and the mixture was stirred at 80°C for 3 hours. Water/AcOEt were then added thereto, and the mixture was filtered through Celite. The filtrate was extracted with AcOEt. The organic layer was washed with saturated saline and then dried over Na₂SO₄, and the solvent was distilled off. The residue was purified by column chromatography (silica gel, hexane/AcOEt) to give the desired product (19 mg) .
1H NMR (400 MHz, CDCl₃) δ 7.53-7.49 (m, 2H), 7.33-7.28 (m, 5H), 4.83 (d, J = 10.8 Hz, 1H), 4.62-4.47 (brs, 1H), 3.77 (d, J = 10.8 Hz, 1H), 3.40 (s, 3H), 3.22-3.10 (brm, 2H), 2.68 (t, J = 7.6 Hz, 2H), 1.82 (tt, J = 7.6, 7.6 Hz, 2H), 1.44 (s, 9H);
LC-MS: [M+H]⁺= 442.11.

### Production Example 6: Production of Compound (CPP-124)

Compound (CPP-124) was produced according to the method shown in the following scheme.

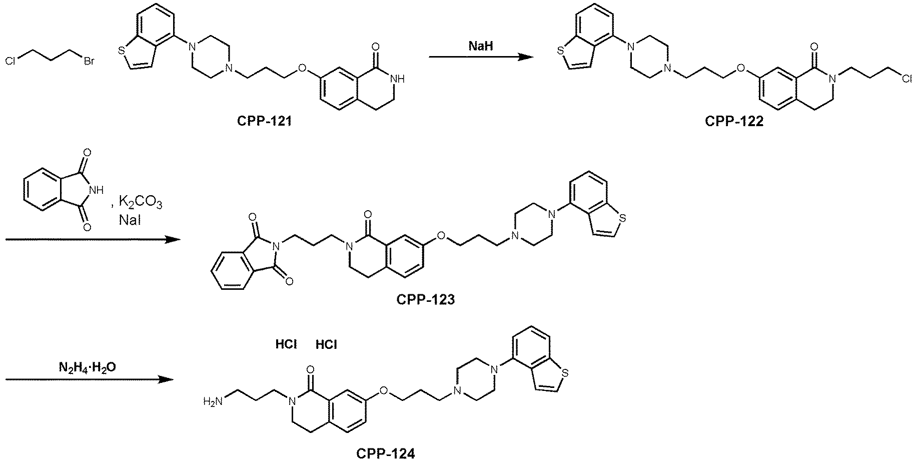

### (1) Production of Compound (CPP-122)

A mixture of compound (CPP-121) shown in the above scheme (0.51 g), 60% NaH (72 mg), and DMF (50 mL) was stirred at room temperature for 30 minutes. 1-Bromo-3-chloropropane (0.18 mL) was added to the reaction mixture, and the mixture was stirred at room temperature for 60 hours. Water was added to the reaction mixture, followed by concentration. AcOEt was added to the residue. The mixture was washed with water and then dried over Na₂SO₄, and the solvent was distilled off. The residue was purified by column chromatography (silica gel, AcOEt/MeOH) to give the desired product (0.45 g).
1H NMR (500 MHz, CDCl₃) δ 7.61 (d, J = 2.7 Hz, 1H), 7.55 (d, J = 8.1 Hz, 1H), 7.42 (d, J = 5.5 Hz, 1H), 7.38 (d, J = 5.5 Hz, 1H), 7.27 (t, J = 7.8 Hz, 1H), 7.09 (d, J = 8.3 Hz, 1H), 6.99 (dd, J = 8.3, 2.7 Hz, 1H), 6.90 (d, J = 7.6 Hz, 1H), 4.11 (t, J = 6.3 Hz, 2H), 3.70 (t, J = 6.8 Hz, 2H), 3.63 (t, J = 6.4 Hz, 2H), 3.59 (t, J = 6.7 Hz, 2H), 3.20 (br, 4H), 2.94 (t, J = 6.6 Hz, 2H), 2.73 (br, 4H), 2.64 (t, J = 7.3 Hz, 2H), 2.15 (m, J = 6.7 Hz, 2H), 2.04 (m, J = 6.5 Hz, 2H) ; HRMS (ESI) calcd for C₂₇H₃₃N₃O₂S 498.1982 [M+H]⁺, found 498.1979.

### (2) Production of Compound (CPP-123)

A suspension of compound (CPP-122) produced above (0.45 g), phthalimide (0.16 mg), K₂CO₃ (0.15 g), and NaI (41 mg) in DMF (10 mL) was stirred at 70°C for 17 hours. The reaction mixture was concentrated, and water was added to the residue, followed by extraction with EtOAc. The organic layer was dried over Na₂SO₄, and the solvent was distilled off. The residue was purified by column chromatography (silica gel, AcOEt/MeOH) to give the desired product (0.43 g).
1H NMR (500 MHz, CDCl₃) δ 7.84 (d, J = 3.0 Hz, 1H) 7.83 (d, J = 3.0 Hz, 1H), 7.60 (d, J = 2.7 Hz, 1H), 7.54 (d, J = 8.1 Hz, 1H), 7.41 (dd, J = 5.5, 0.6 Hz, 1H), 7.38 (d, J = 5.5 Hz, 1H), 7.27 (t, J = 7.8 Hz, 1H), 7.09 (d, J = 8.3 Hz, 1H), 6.98 (dd, J = 8.3, 2.8 Hz, 1H), 6.90 (dd, J = 7.7, 0.6 Hz, 1H), 4.10 (t, J = 6.4 Hz, 2H), 3.77 (t, J = 7.3 Hz, 2H), 3.66 (t, J = 7.1 Hz, 2H), 3.57 (t, J = 6.7 Hz, 2H), 3.20 (br, 4H), 2.96 (t, J = 6.6 Hz, 2H), 2.75 (br, 4H), 2.65 (t, J = 7.4 Hz, 2H), 2.07- 2.01 (m, 4H);
HRMS (ESI) calcd for C₃₅H₃₇N₄O₄S 609.2535 [M+H]⁺, found 609.2538.

### (3) Production of Compound (CPP-124)

A mixture of compound (CPP-123) produced above (0.43 g), hydrazine monohydrate (0.069 mL), and EtOH (12 mL) was heated under reflux for 8 hours. The reaction mixture was concentrated, and a 1M aqueous NaOH solution (50 mL) was added to the residue, followed by extraction with AcOEt. The organic layer was washed with saturated saline and then dried over Na₂SO₄, and the solvent was distilled off. The residue was purified by column chromatography (silica gel, DCM/MeOH). The purified product was dissolved in MeOH (10 mL). A 2M aqueous HCl solution (2.1 mL) was added thereto dropwise, and the mixture was stirred at room temperature for 15 hours. The solvent was then distilled off. AcOEt was added to the residue, followed by stirring. The formed solid was collected by filtration to give the desired product (0.35 g).
1H NMR (500 MHz, CDCl₃) δ 11.4 (br, 1H), 7.98 (br, 3H), 7.78 (d, J = 5.5 Hz, 1H), 7.71 (d, J = 8.1 Hz, 1H), 7.50 (d, J = 5.5 Hz, 1H), 7.43 (d, J = 2.7 Hz, 1H), 7.33 (t, J = 7.8 Hz, 1H), 7.26 (d, J = 8.4 Hz, 1H), 7.10 (dd, J = 8.3 , 2.7 Hz, 1H), 6.98 (d, J = 7.6 Hz, 1H), 4.13 (t, J = 5.9 Hz, 2H), 3.66 (br, 2H), 3.57-3.53 (m, 6H), 3.37 (br, under the water signal), 3.27 (t, J = 11.9 Hz, 2H), 2.92 (t, J = 6.5 Hz, 2H), 2.82-2.76 (m, 2H), 2.30-2.25 (m, 2H), 1.88 (m, J = 7.3 Hz, 2H);
HRMS (ESI) calcd for C₂₇H₃₅N₄O₂S 479.2481 [M+H]⁺, found 479.2482.

### Production Example 7: Production of Compound (CPP-125)

Compound (CPP-125) was produced according to the method shown in the following scheme.

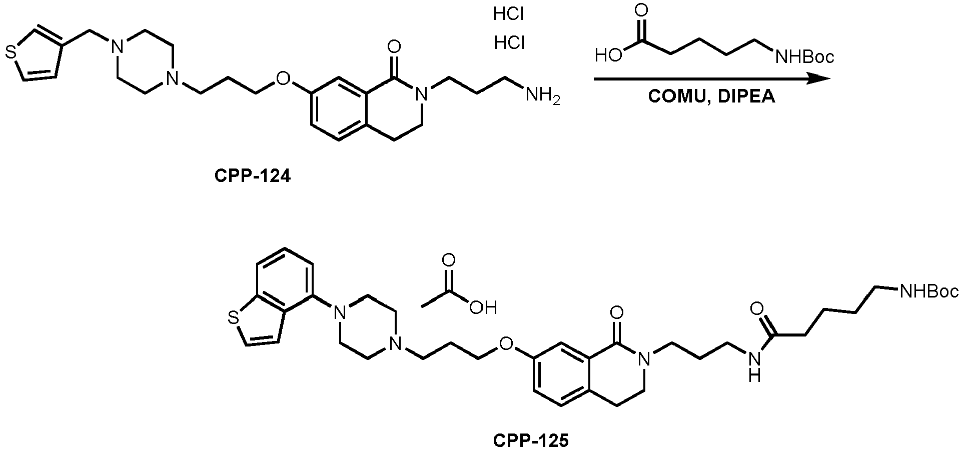

A mixture of compound (CPP-124) produced in Production Example 6 (20 mg), Boc-5-amino-n-valeric acid (7.9 mg), COMU (17 mg), DIPEA (27 µL), and DMF (1 ml) was stirred at room temperature for 5 hours, and the reaction mixture was concentrated. The residue was purified by silica gel chromatography (ODS, water/MeCN, containing 0.1% AcOH) to give the desired product (15 mg).
1H NMR (500 MH, DMSO-d₆) δ 7.78 (t, J = 5.5 Hz, 1H), 7.69 (d, J = 5.6 Hz, 1H), 7.61 (d, J = 7.9 Hz, 1H), 7.40 (d, J = 5.6 Hz, 1H), 7.39 (d, J = 2.7 Hz, 1H), 7.27 (t, J = 7.8 Hz, 1H), 7.20 (d, J = 8.4 Hz, 1H), 7.05 (dd, J = 8.3, 2.6 Hz, 1H), 6.90 (d, J = 7.7 Hz, 1H), 6.77 (t, J = 5.4 Hz, 1H), 4.06 (t, J = 6.3 Hz, 2H), 3.50 (t, J = 7.0 Hz, under the water signal), 3.46 (t, J = 6.6 Hz, under the water signal), 3.08-3.03 (m, 6H), 2.91-2.87 (m, 4H), 2.64 (br, 4H), 2.54 (t, J = 7.1 Hz, 2H), 2.04 (t, J = 7.3 Hz, 2H), 1.93 (m, J = 6.7 Hz, 2H), 1.82 (s, 3H), 1.67 (m, J = 7.0 Hz, 2H), 1.46 (m, J = 7.5 Hz, 2H), 1.36-1.31 (m, 11H) ; HRMS (ESI) calcd for C₃₇H₅₂N₅O₅S 678.3689 [M+H]⁺, found 678.3693.

### Production Example 8: Production of Compound (CPF-202)

Compound (CPF-202) was produced according to the method shown in the following scheme.

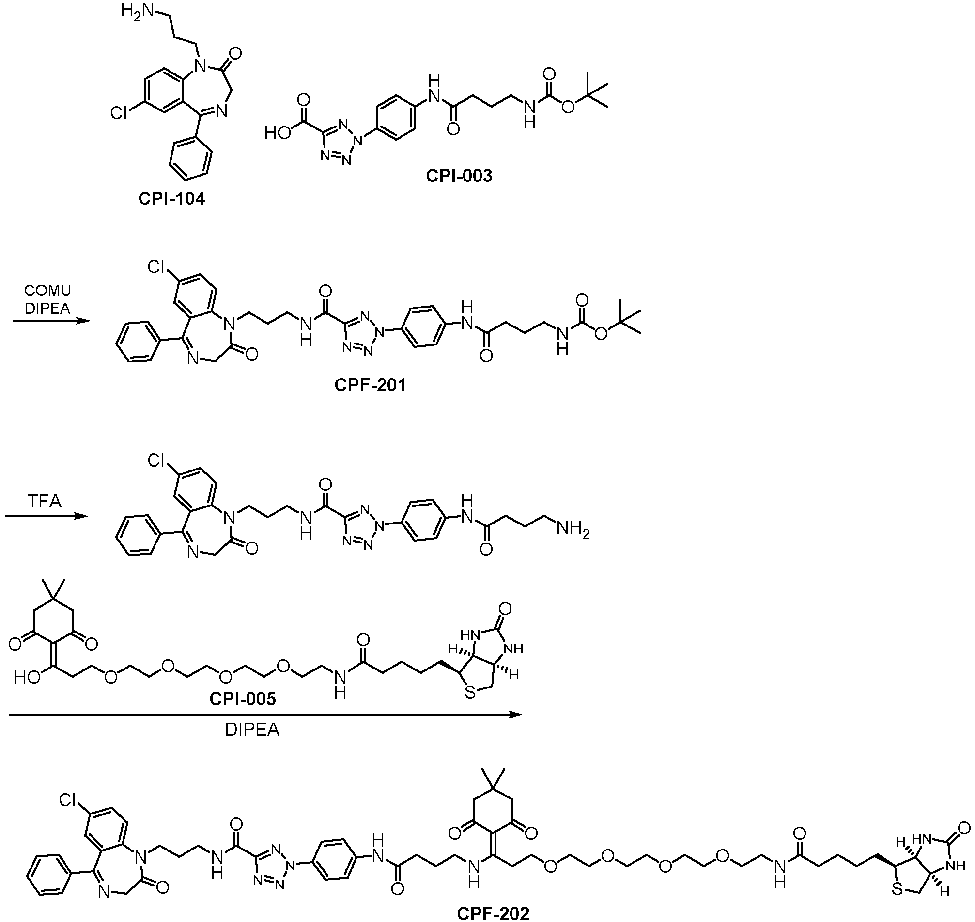

### (1) Production of CPF-201

A mixture of compound (CPI-104) produced in Production Example 3 (9.0 mg), compound (CPI-003) produced in Production Example 1 (11 mg), COMU (13 mg), DIPEA (11 µL), and DMF (1 mL) was stirred at room temperature for 2 hours. The solvent was distilled off, and purification was performed by column chromatography (ODS, water/MeCN, containing 0.1% AcOH) to give the desired product (10 mg) .
1H NMR (400 MHz, CDCl₃) δ 9.57 (s, 1H), 8.13 (dt, J = 9.1, 2.4 Hz, 2H), 7.87 (d, J = 8.9 Hz, 2H), 7.64-7.59 (m, 3H), 7.54-7.47 (m, 2H), 7.45-7.39 (m, 3H), 7.30 (d, J = 2.4 Hz), 4.88-4.83 (m, 2H), 4.54-4.47 (m, 1H), 3.81-3.71 (m, 2H), 3.64-3.56 (m, 1H), 3.29 (dt, J= 6.1, 6.1 Hz, 2H), 3.11-3.03 (m, 1H), 2.44-2.41 (m, 2H), 2.00-1.86 (m, 3H), 1.77-1.65 (m, 1H), 1.50 (s, 9H); LC-MS: [M+H]⁺= 700.29.

### (2) Production of CPF-202

A mixture of compound (CPF-201) produced above (10 mg), TFA (0.11 mL), and DCM (1 mL) was stirred at room temperature for 2 hours, and the solvent was then distilled off. A mixture of the residue, compound (CPI-005) produced in Production Example 2 (10 mg), DIPEA (10 µL), and DMF (1 mL) was stirred at 60°C for 3 hours, and the solvent was then distilled off. The residue was purified by column chromatography (ODS, water/MeCN) to give the desired product (11 mg) .
1H NMR (400 MHz, CDCl₃) δ 13.69-13.66 (m, 1H), 9.41 (s, 1H), 8.09 (dt, J = 9.0, 2.4 Hz, 2H), 7.88-7.86 (m, 3H), 7.61-7.59 (m, 2H), 7.54-7.48 (m, 2H), 7.45-7.39 (m, 3H), 7.30 (d, J= 2.4 Hz, 1H), 6.44-6.39 (m, 1H), 5.61-5.58 (m, 1H), 4.85 (d, J = 10.5 Hz, 1H), 4.68 (d, J= 7.4 Hz, 1H), 4.53-4.46 (m, 2H), 4.35-4.32 (m, 1H), 3.81-3.48 (m, 20H), 3.37-3.29 (m, 4H), 3.17-3.05 (m, 2H), 2.95-2.90 (m, 1H), 2.72 (dd, J = 12.8, 4.0 Hz, 1H), 2.57 (t, J = 7.1 Hz, 2H), 2.34 (s, 4H), 2.14 (dd, J= 7.1, 7.1 Hz, 4H), 1.99-1.91 (m, 1H), 1.77-1.52 (m, 6H), 1.47-1.41 (m, 2H), 1.00 (s, 6H);
LC-MS: [M+H]⁺= 1195.3.

### Production Example 9: Production of Compound (CPF-212)

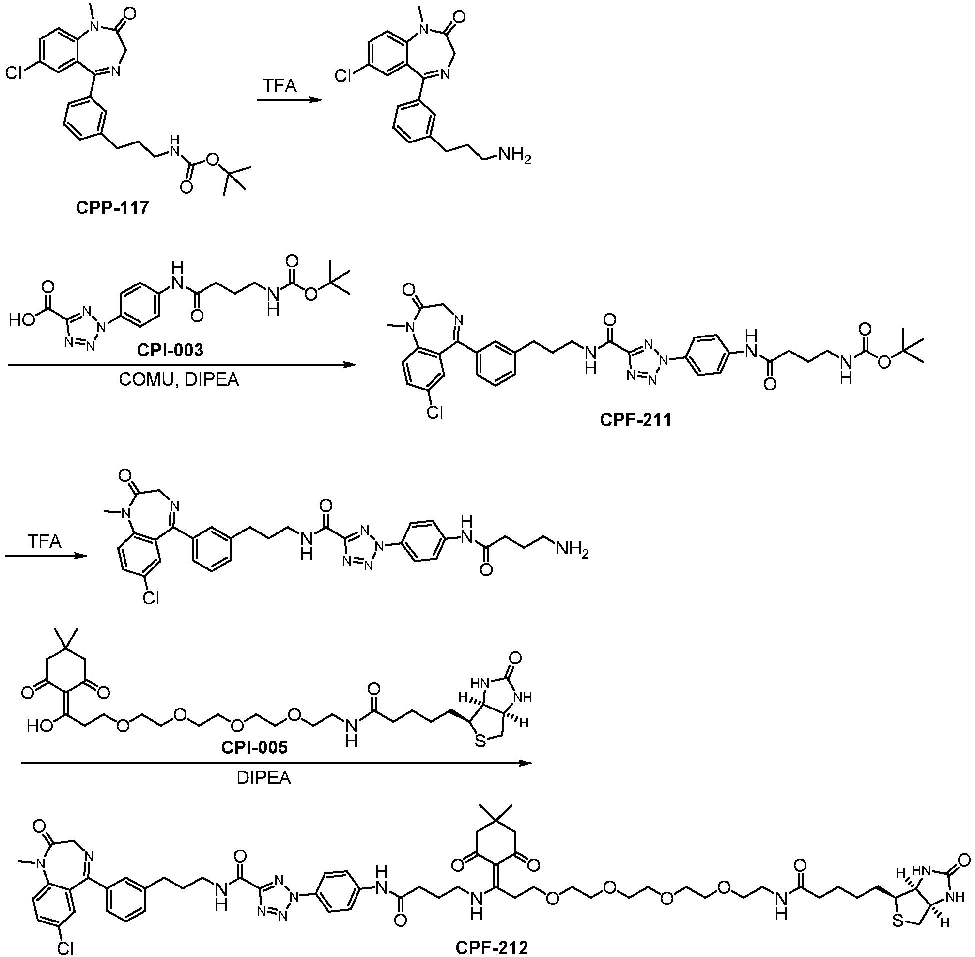

### (1) Production of CPF-211

TFA (0.17 mL) was added dropwise to a solution of compound (CPP-117) produced in Production Example 5 (19 mg) in DCM (1 mL). The mixture was stirred at room temperature for 2 hours, and the solvent was then distilled off. The residue was purified by column chromatography (ODS, water/MeCN) to give an intermediate compound (13 mg). The intermediate compound (13 mg) and compound (CPI-003) produced in Production Example 1 (16.78 mg) were dissolved in DMF (1 mL). COMU (18 mg) and DIPEA (0.23 mL) were added thereto, and the mixture was stirred at room temperature for 3 hours. The solvent was then distilled off. The residue was purified by column chromatography (ODS, water/MeCN) to give the desired product (18 mg).
1H NMR (400 MHz, CDCl₃) δ 9.62 (s, 1H), 8.14 (dt, J = 9.1, 2.4 Hz, 2H), 7.90-7.87 (m, 2H), 7.53-7.50 (m, 2H), 7.38-7.33 (m, 3H), 7.30 (dd, J= 5.6, 3.1 Hz, 2H), 7.26-7.22 (m, 1H), 4.84-4.82 (m, 2H), 3.77 (d, J = 10.8 Hz, 1H), 3.60 (dt, J = 6.8, 6.8 Hz, 2H), 3.40 (s, 3H), 3.29 (dt, J = 6.1, 6.1 Hz, 2H), 2.79 (t, J = 7.7 Hz, 2H), 2.44-2.41 (m, 2H), 2.07-2.00 (m, 2H), 1.92-1.86 (m, 2H), 1.50 (s, 9H) ;

LC-MS: [M+H]⁺= 714.08.

### (2) Production of CPF-212

TFA (97 µL) was added dropwise to a solution of compound (CPF-211) produced above (18 mg) in DCM (1 mL). The mixture was stirred at room temperature for 1.5 hours, and the solvent was then distilled off. DIPEA (18 µL) was added to a solution of the residue and compound (CPI-005) produced in Production Example 2 (22 mg) in DMF (1 mL), and the mixture was stirred at 60°C for 3 hours. DIPEA (18 µL) was further added thereto, and the mixture was stirred at 60°C for another 3 hours. The solvent was then distilled off. The residue was purified by column chromatography (ODS, water/MeCN) to give the desired product (13 mg).
1H NMR (400 MHz, DMSO-d₆) δ 13.53 (t, J = 5.1 Hz, 1H), 10.37 (s, 1H), 9.19 (t, J = 5.7 Hz, 1H), 8.06 (dt, J = 9.1, 2.3 Hz, 2H), 7.89 (dt, J = 9.1, 2.3 Hz, 2H), 7.81 (t, J = 5.6 Hz, 1H), 7.70 (dd, J = 8.9, 2.5 Hz, 1H), 7.60 (d, J = 8.9 Hz, 1H), 7.49 (s, 1H), 7.42-7.36 (m, 2H), 7.30 (dt, J = 7.4, 1.6 Hz, 1H), 7.22 (d, J = 2.5 Hz, 1H), 6.40 (s, 1H), 6.35 (s, 1H), 4.57 (d, J = 10.7 Hz, 1H), 4.31-4.28 (m, 1H), 4.13-4.10 (m, 1H), 3.78 (d, J = 10.7 Hz, 1H), 3.63-3.58 (m, 3H), 3.47-3.46 (m, 9H), 3.38-3.05 (m, under the water signal), 2.80 (dd, J= 12.4, 5.1 Hz, 1H), 2.71-2.66 (m, 3H), 2.58-2.49 (m, overlapped with the DMSO signal), 2.28 (s, 4H), 2.05 (t, J = 7.4 Hz, 2H), 1.97-1.84 (m, 4H), 1.64-1.55 (m, 1H), 1.52-1.40 (m, 3H), 1.32-1.24 (m, 2H), 0.94 (s, 6H); LC-MS: [M+H]⁺= 1209.12.

### Production Example 10: Production of Compound (CPF-224)

Compound (CPF-224) was produced according to the method shown in the following scheme.

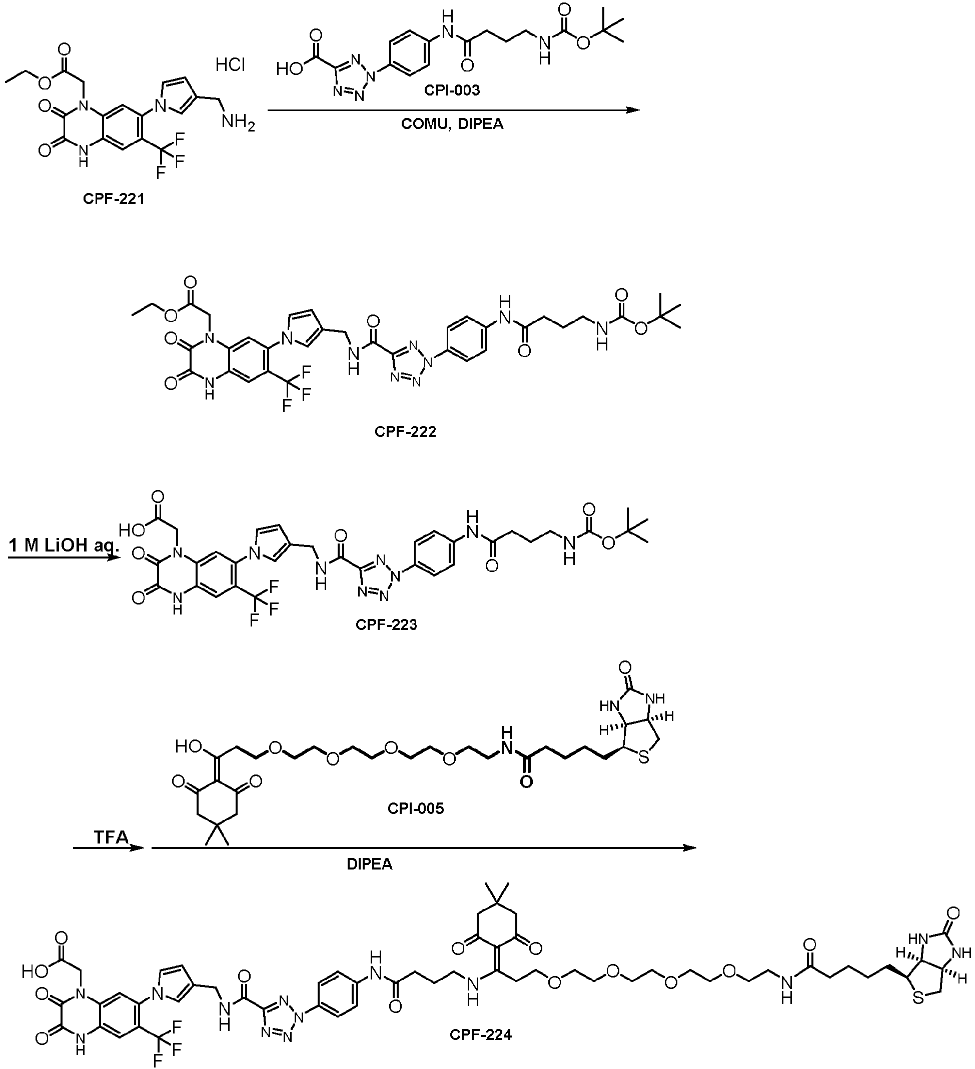

### (1) Production of Compound (CPF-222)

Compound (CPI-003) produced in Production Example 1 (20 mg) and compound (CPF-221) shown in the above scheme (23 mg) were dissolved in DMF (2 mL). COMU (24 mg) and DIPEA (0.020 mL) were added thereto, and the mixture was stirred for 1 hour. The solvent was then distilled off. The residue was purified by silica gel chromatography (ODS, water/MeCN, containing 0.1% AcOH) to give the desired product (33 mg).
1H NMR (400 MHz, DMSO-d₆) δ 12.46 (br, 1H), 10.30 (s, 1H), 9.43 (t, J = 6.1 Hz, 1H), 8.06 (d, J = 9.2 Hz, 2H), 7.89 (d, J = 9.1 Hz, 2H), 7.60 (s, 1H), 7.52 (br, 1H), 6.88-6.83 (m, 3H), 6.28 (dd, J = 2.9, 1.7 Hz, 1H), 5.00 (s, 2H), 4.41 (d, J = 6.0 Hz, 2H), 4.13 (q, J = 7.1 Hz, 2H), 2.98 (q, J = 6.6 Hz, 2H), 2.36 (t, J = 7.3 Hz, 2H), 1.71 (m, J = 7.3 Hz, 2H), 1.38 (s, 9H), 1.17 (t, J = 7.1 Hz, 3H) ;
LC-MS: [M+H]⁺= 783.31.

### (2) Production of Compound (CPF-223)

A 1M aqueous LiOH solution (0.13 mL) and water (0.36 mL) were added to a solution of compound (CPF-222) synthesized above (33 mg) in MeOH (2 ml), and the mixture was stirred for 21 hours. The reaction mixture was neutralized with a 0.1% aqueous AcOH solution, and the resulting solution was concentrated. The residue was purified by silica gel chromatography (ODS, water/MeCN, containing 0.1% AcOH) to give the desired product (30 mg).
1H NMR (400 MHz, DMSO-d₆) δ 12.46 (s, 1H), 10.30 (s, 1H), 9.43 (t, J = 5.9 Hz, 1H), 8.06 (d, J = 9.1 Hz, 2H), 7.88 (d, J = 9.1 Hz, 2H), 7.61 (s, 1H), 7.47 (br, 1H), 6.90-6.83 (m, 3H), 6.28 (dd, J = 2.9, 1.7 Hz, 1H), 4.92 (s, 2H), 4.41 (d, J = 5.9 Hz, 2H), 2.98 (q, J = 6.5 Hz, 2H), 2.36 (t, J = 7.4 Hz, 2H), 1.71 (m, J = 7.2 Hz, 2H), 1.38 (s, 9H);
LC-MS: [M+H]⁺= 754.99.

### (3) Production of Compound (CPF-224)

Compound (CPF-223) synthesized above (29 mg) was dissolved in DCM (3 ml). TFA (0.29 mL) was added thereto dropwise, followed by stirring at room temperature for 2 hours. The resulting solution was then concentrated. DIPEA (27 µL), compound (CBI-455) produced in Production Example 2 (39 mg), and DMF (2 mL) were added to the residue, followed by stirring at 60°C for 5 hours. The reaction mixture was then concentrated. The residue was purified by silica gel chromatography (ODS, water/MeCN, containing 0.1% TFA) to give the desired product (17 mg).
1H NMR (400 MHz, DMSO-d₆) δ 13.53 (t, J = 5.5 Hz, 1H), 12.47 (s, 1H), 10.38 (s, 1H), 9.44 (t, J = 6.2 Hz, 1H), 8.07 (d, J = 9.1 Hz, 2H), 7.89 (d, J = 9.1 Hz, 2H), 7.82 (t, J = 5.7 Hz, 1H), 7.61 (s, 1H), 7.49 (br, 1H), 6.90 (br, 1H), 6.84 (t, J = 2.4 Hz, 1H), 6.41 (br, 1H), 6.35 (br, 1H), 6.28 (dd, J = 2.8, 1.7 Hz, 1H), 4.95 (s, 2H), 4.41 (d, J = 5.9 Hz, 2H), 4.30 (dd, J = 7.8, 4.4 Hz, 1H), 4.12 (dd, J = 7.6, 4.4 Hz, 1H), 3.61 (t, J = 6.3 Hz, 4H), 3.49-3.46 (m, under the water signal), 3.30 -3.25 (m, 4H), 3.17 (q, J = 5.7 Hz, 2H), 3.10-3.06 (m, 1H), 2.81 (dd, J = 12.5, 5.1 Hz, 1H), 2.57 (d, J = 12.5 Hz, 1H), 2.47 (t, overlapped with the DMSO signal), 2.28 (s, 4H), 2.05 (t, J = 7.4 Hz, 2H), 1.93 (m, J = 6.9 Hz, 2H), 1.60-1.55 (m, 1H), 1.53-1.40 (m, 3H), 1.33-1.23 (m, 2H)), 0.94 (s, 6H);
HRMS (ESI) calcd for C₅₇H₇₁N₁₃O₁₄F₃S 1250.4916 [M+H]⁺, found 1250.4919.

### Production Example 11: Production of Compound (CPF-232)

Compound (CPF-232) was produced according to the method shown in the following scheme.

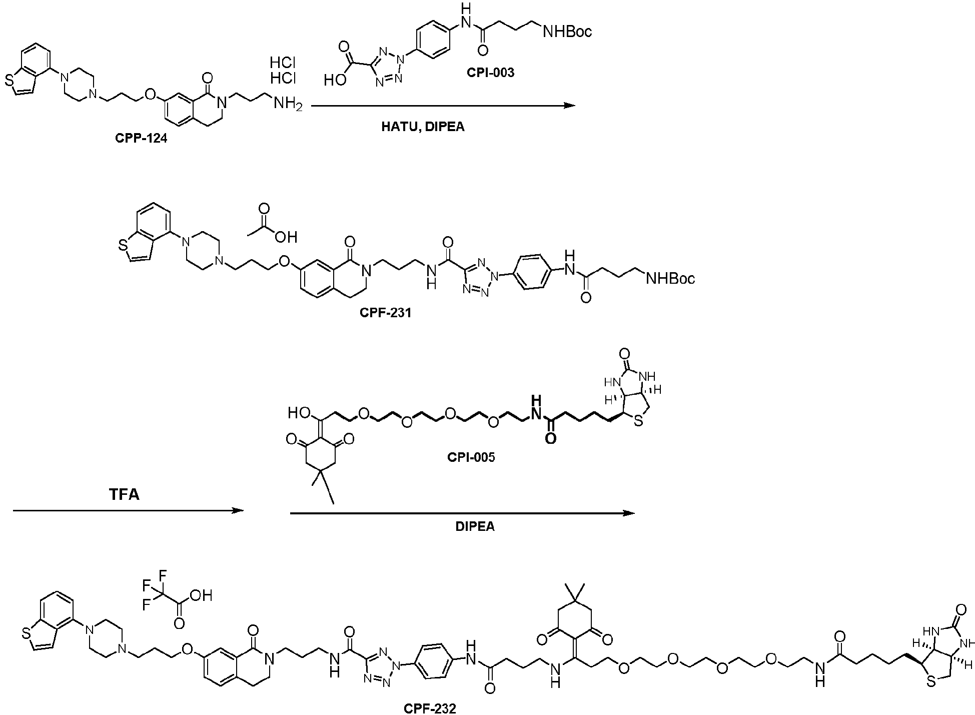

### (1) Production of Compound (CPF-231)

Compound (CPP-124) produced in Production Example 6 (40 mg) and compound (CPI-003) produced in Production Example 1 (26 mg) were diluted with DMF (2 ml). HATU (26 mg) and DIPEA (0.036 ml) were added thereto, followed by stirring for 5 hours. The reaction mixture was then concentrated. The residue was purified by silica gel chromatography (ODS, water/MeCN, containing 0.1% TFA) to give the desired product (50 mg).
1H NMR (500 MHz, DMSO-d₆) δ 11.96 (br, 1H), 10.30 (s, 1H), 9.16 (t, J = 5.8 Hz, 1H), 8.06 (d, J = 9.1 Hz, 2H), 7.89 (d, J = 9.1 Hz, 2H), 7.74 (d, J = 5.1 Hz, 1H), 7.67 (d, J = 7.7 Hz, 1H), 7.46 (d, J = 4.1 Hz, 1H), 7.43 (d, J = 2.3 Hz, 1H), 7.30 (t, J = 7.8 Hz, 1H), 7.23 (d, J = 8.4 Hz, 1H), 7.07 (dd, J = 8.3, 2.7 Hz, 1H), 6.95 (d, J = 7.4 Hz, 1H), 6.86 (t, J = 5.4 Hz, 1H), 4.10 (t, J = 5.4 Hz, 2H), 3.62-3.51 (m, 6H), 3.38-3.35 (m, under the water signal), 3.10 (br, 2H), 2.98 (q, J = 6.5 Hz, 2H), 2.92 (t, J = 6.5 Hz, 2H), 2.36 (t, J = 7.4 Hz, 2H), 2.08 (br, 2H), 1.91 (s, 3H), 1.86 (m, J = 7.0 Hz, 2H), 1.72 (m, J = 7.2 Hz, 2H) ; LC-MS: [M+H]⁺ = 609.52

### (2) Production of Compound (CPF-232)

Compound (CPF-231) produced above (19 mg) was dissolved in DCM (2 mL). TFA (0.16 mL) was added thereto dropwise, followed by stirring at room temperature for 1 hour. The reaction mixture was then concentrated. Compound (CPI-005) produced in Production Example 2 (15 mg), DIPEA (17 µL), and DMF (2 ml) were added to the residue, followed by stirring at 60°C for 2 hours. The reaction mixture was then concentrated. The residue was purified by silica gel chromatography (ODS, water/MeCN, containing 0.1% TFA) to give the desired product (8.7 mg).
1H NMR (500 MHz, DMSO-d₆) δ 13.53 (t, J = 5.3 Hz, 1H), 10.38 (s, 1H), 9.51 (br, 1H ), 9.17 (t, J = 5.9 Hz, 1H), 8.06 (d, J = 9.1 Hz, 2H), 7.89 (d, J = 9.1 Hz, 2H), 7.81 (t, J = 5.5 Hz, 1H), 7.78 (d, J = 5.5 Hz, 1H), 7.72 (d, J = 8.1 Hz, 1H), 7.51 (d, J = 5.6 Hz, 1H), 7.45 (d, J = 2.7 Hz, 1H), 7.33 (t, J = 7.9 Hz, 1H), 7.25 (d, J = 8.5 Hz, 1H), 7.08 (dd, J = 8.3, 2.7 Hz, 1H), 6.99 (d, J = 7.7 Hz, 1H), 6.41 (br, 1H), 6.36 (br, 1H ), 4.29 (dd, J = 7.8, 4.9 Hz, 1H), 4.14-4.10 (m, 3H), 3.70 (br, 2H), 3.62-3.55 (m, under the water signal), 3.41-3.34 (m, under the water signal), 3.27 (t, J = 6.2 Hz, 2H), 3.16 (q, J = 5.8 Hz, 2H), 3.10-3.06 (m, 3H), 2.92 (t, J = 6.6 Hz, 2H), 2.80 (dd, J = 12.3, 5.1 Hz, 1H), 2.57 (d, J = 12.4 Hz, 1H), 2.28 (s, 4H), 2.22-2.17 (m, 2H), 2.05 (t, J = 7.4 Hz, 2H), 1.93 (m, J = 7.1 Hz, 2H), 1.86 (m, J = 6.8 Hz, 2H), 1.63-1.56 (m, 1H), 1.51-1.40 (m, 3H), 1.33-1.22 (m, 2H), 0.94 (s, 6H); HRMS (ESI) calcd for C₆₈H₉₂N₁₃O₁₂S₂ 1346.6429 [M+H]⁺, found 1346.6445.

### Production Example 12: Production of Compound (CPP-127)

Compound (CPP-127) was produced according to the method shown in the following scheme.

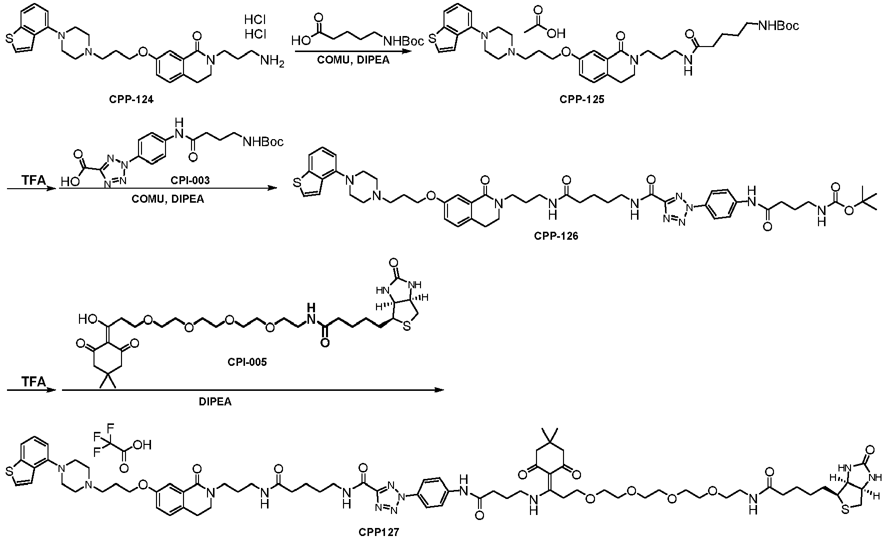

### (1) Production of Compound (CPP-126)

Compound (CPP-125) produced in Production Example 7 (15 mg) was dissolved in DCM (3 mL). TFA (0.15 µL) was added thereto dropwise, followed by stirring at room temperature for 2 hours. The reaction mixture was then concentrated. The residue was diluted with compound (CPI-003) produced in Production Example 1 (9.1 mg) and DMF (2 ml). COMU (9.35 mg) and DIPEA (15 µL) were added thereto, followed by stirring at room temperature for 1 hour. The reaction mixture was then concentrated. The residue was purified by silica gel chromatography (ODS, water/MeCN, containing 0.1% AcOH) to give the desired product (16 mg).
1H NMR (500 MHz, DMSO-d₆) δ 11.94 (br, 1H), 10.29 (s, 1H), 9.14 (t, J = 5.9 Hz, 1H), 8.04 (d, J = 9.1 Hz, 2H), 7.87 (d, J = 9.1 Hz, 2H), 7.81 (t, J = 5.5 Hz, 1H), 7.69 (br, 1H), 7.61 (br, 1H ), 7.41-7.38 (m, 2H), 7.28 (t, J = 7.6 Hz, 1H), 7.19 (d, J = 8.6 Hz, 1H), 7.04 (dd, J = 8.5, 2.7 Hz, 1H), 6.90 (br, 1H), 6.86 (t, J = 5.4 Hz, 1H), 4.05 (br, 2H), 3.50 (t, J = 6.5 Hz, 2H), 3.46 (t, J = 7.1 Hz, 2H), 3.09-3.04 (m, 6H), 2.98 (q, J = 6.5 Hz, 2H), 2.87 (t, J = 6.7 Hz, 2H), 2.64(br,4H),2.36 (t, J = 7.4 Hz, 2H), 2.10 (br, 2H), 1.94 (br, 2H), 1.91 (s, 3H), 1.71 (m, J = 7.2 Hz, 2H), 1.67 (m, J = 7.0 Hz, 2H), 1.55 (br, 4H), 1.37 (s, 9H);
HRMS (ESI) calcd for C₄₉H₆₄N₁₁O₇S 950.4711 [M+H]⁺, found 950.4713.

### (2) Production of Compound (CPP-127)

Compound (CPP-126) produced above (14 mg) was dissolved in DCM (5 ml). TFA (0.23 mL) was added thereto dropwise, followed by stirring at room temperature for 12 hours. The reaction mixture was then concentrated. Compound (CPI-005) produced in Production Example 2 (9.2 mg), DIPEA (12 µL), and DMF (2 mL) were added to the residue, followed by stirring at 60°C for 2 hours. The reaction mixture was then concentrated. The residue was purified by silica gel chromatography (ODS, water/MeCN, containing 0.1% TFA) to give the desired product (13 mg).
1H NMR (500 MHz, DMSO-d₆) δ 13.53 (t, J = 5.4 Hz, 1H), 10.38 (s, 1H), 9.63 (br, 1H), 9.15 (t, J = 5.9 Hz, 1H), 8.05 (d, J = 9.1 Hz, 2H), 7.88 (d, J = 9.1 Hz, 2H), 7.84-7.81 (m, 2H), 7.78 (d, J = 5.6 Hz, 1H), 7.72 (d, J = 8.1 Hz, 1H), 7.51 (t, J = 5.5 Hz, 1H), 7.43 (d, J = 2.8 Hz, 1H), 7.33 (t, J = 7.9 Hz, 1H), 7.22 (d, J = 8.4 Hz, 1H), 7.06 (dd, J = 8.4, 2.8 Hz, 1H), 6.99 (d, J = 7.3 Hz, 1H), 6.41 (br, 1H), 6.36 (br, 1H), 4.29 (dd, J = 7.9, 4.9 Hz, 1H), 4.13-4.10 (m, under the water signal), 3.70 (br, 2H), 3.62-3.58 (m, 6H), 3.52-3.49 (m, 4H), 3.47-3.46 (m, 12H), 3.41 (br, 4H), 3.37 (t, J = 5.9 Hz, 2H), 3.32 (br, 2H), 3.27 (t, J = 6.1 Hz, 2H), 3.17 (q, J = 5.8 Hz, 2H), 3.11-3.04 (m, 5H), 2.88 (t, J= 6.4 Hz, 2H), 2.81 (dd, J = 12.4, 5.1 Hz, 1H), 2.57 (d, J = 12.3 Hz, 1H), 2.48 (d, overlapped with the DMSO signal), 2.28 (s, 4H), 2.22-2.17 (m, 2H), 2.11 (br, 2H), 2.05 (t, J = 7.3 Hz, 2H), 1.93 (m, J = 7.0 Hz, 2H), 1.68 (m, J= 6.9 Hz, 2H), 1.62-1.41 (m, 8H), 1.33-1.23 (m, 2H), 0.94 (s, 6H) ; HRMS (ESI) calcd for C₇₃H₁₀₁N₁₄O₁₃S₂ 1445.7114 [M+H]⁺, found 1445.7126.

### Production Example 13: Production of Compound (CPP-133)

Compound (CPP-133) was produced according to the method shown in the following scheme.

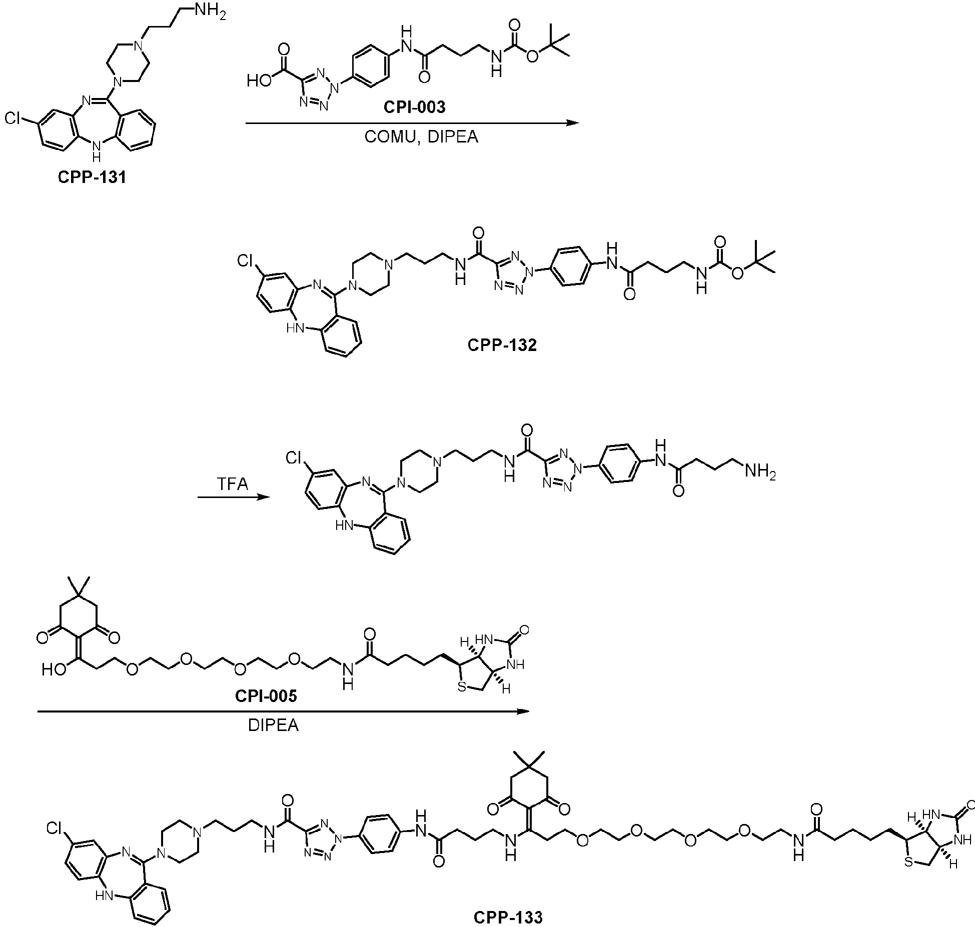

### (1) Production of CPP-132

Compound (CPP-131) (15 mg) and compound (CPI-003) produced in Production Example 1 (16 mg) were dissolved in a DMF solution (1 mL), and COMU (19 mg) and DIPEA (15 µL) were added thereto. The mixture was stirred at room temperature for 20 hours, and the solvent was then distilled off. The residue was purified by column chromatography (ODS, water/MeCN) to give the desired product (22 mg) .
1H NMR (400 MHz, CDCl₃) δ 9.30 (s, 1H), 9.11 (s, 1H), 8.10 (dt, J = 9.1, 2.4 Hz, 2H), 7.85 (d, J = 9.1 Hz, 2H), 7.31-7.25 (m, 2H), 7.05 (d, J = 2.4 Hz, 1H), 7.01 (td, J = 7.5, 0.9 Hz, 1H), 6.86 (dd, J = 7.7, 0.7 Hz, 1H), 6.82 (dd, J = 8.3, 2.4 Hz, 1H), 6.65 (d, J = 8.3 Hz, 1H), 5.21 (s, 1H), 4.87-4.78 (brm, 1H), 3.70 (dt, J = 5.6, 5.6 Hz, 2H), 3.66-3.50 (brs, 4H), 3.28 (dt, J = 6.0, 6.0 Hz, 2H), 2.67-2.64 (m, 6H), 2.43-2.40 (m, 2H), 1.93-1.84 (m, 4H), 1.48 (s, 9H); LC-MS: [M+H]⁺= 742.29.

### (2) Production of CPP-133

Compound (CPP-132) produced above (22 mg) was diluted with DCM (1 mL). TFA (0.11 mL) was added thereto dropwise, and the mixture was stirred at room temperature for 20 hours. The solvent was then distilled off. Compound (CPI-005) produced in Production Example 2 (18 mg), DIPEA (21 µL), and DMF (1.5 mL) were added to the residue, and the mixture was stirred at 60°C for 3 hours. DIPEA (26 µL) was further added thereto, and the mixture was stirred at 60°C for another 1 hour. The solvent was distilled off, and the residue was purified by column chromatography (ODS, water/MeCN, containing 0.1% AcOH) to give the desired product (16 mg).
1H NMR (400 MHz, CDCl₃) δ 13.73-13.66 (brm, 1H), 9.28-9.16 (brm, 2H), 8.06 (dt, J = 9.1, 2.4 Hz, 2H), 7.86 (d, J = 9.1 Hz, 2H), 7.31-7.26 (m, 2H), 7.04 (d, J = 2.4 Hz, 1H), 7.00 (td, J = 7.5, 0.9 Hz, 1H), 6.87 (d, J = 7.9 Hz, 1H), 6.81 (dd, J = 8.3, 2.4 Hz, 1H), 6.68 (d, J = 8.3 Hz, 1H), 6.49-6.39 (brs, 1H), 5.58 (s, 1H), 5.39 (s, 1H), 4.65 (s, 1H), 4.52-4.49 (m, 1H), 4.34-4.31 (m, 1H), 3.77 (t, J= 11.4 Hz, 2H), 3.72-3.48 (m, 23H), 3.38-3.28 (m, 4H), 3.16-3.12 (m, 1H), 2.92 (dd, J= 12.9, 5.0 Hz, 1H), 2.72-2.54 (m, 8H), 2.35 (s, 4H), 2.19-2.09 (m, 4H), 1.90-1.82 (m, 2H), 1.73-1.41 (m, 6H) , 1.01 (s, 6H) ; LC-MS: [M+H]⁺ = 1237.14.

### Production Example 14: Production of Compound (CPA-302)

Compound (CPA-302) was produced according to the method shown in the following scheme.

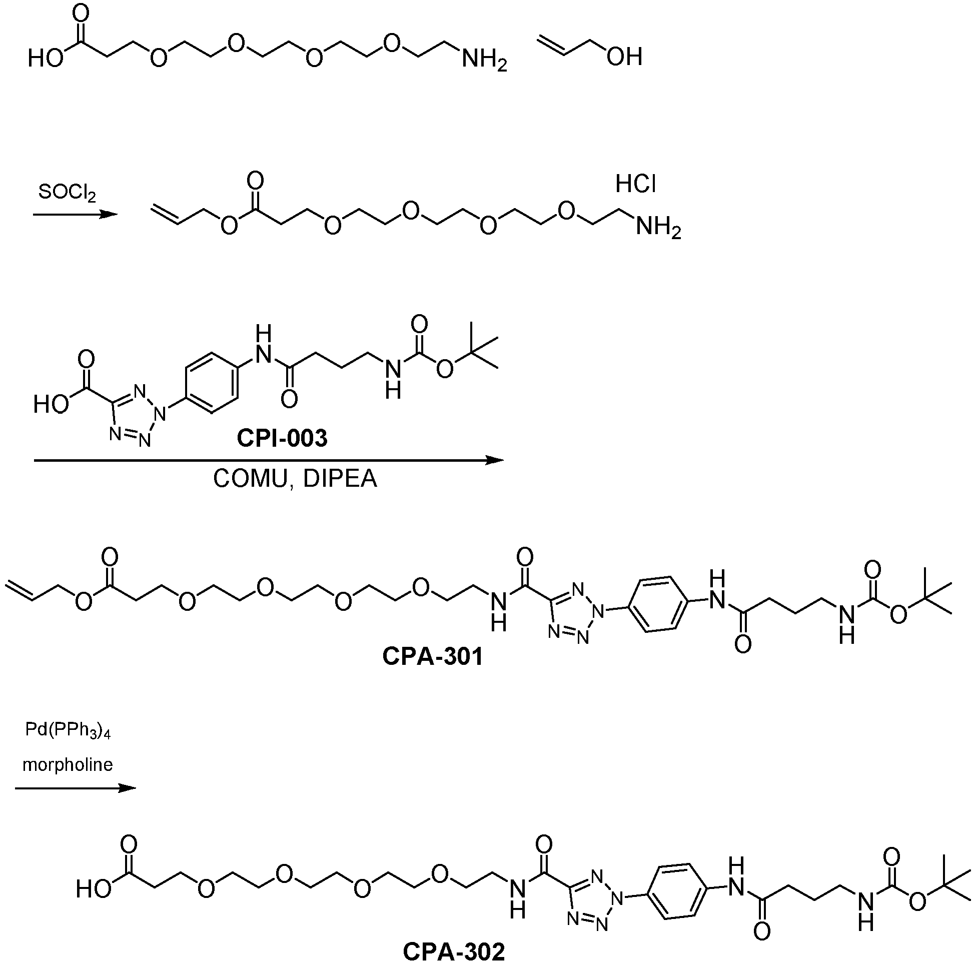

### (1) Production of CPA-301

Thionyl chloride (0.17 mL) was added to a mixture of 3-[2-[2-[2-(2-aminoethoxy)ethoxy]ethoxy]ethoxy]propionic acid (22 mg) and allyl alcohol (78 µL), followed by stirring at room temperature for 1 hour. After water was added thereto, the mixture was concentrated under reduced pressure. The residue and compound (CPI-003) produced in Production Example 1 (30 mg) were dissolved in DMF (2 mL), and DIPEA (40 µL) and COMU (36 mg) were added thereto. The mixture was stirred at room temperature for 3 hours, and the solvent was then distilled off. The residue was purified by column chromatography (ODS, water/MeCN) to give the desired product (37 mg) .
1H NMR (400 MHz, CDCl₃) δ 9.60 (s, 1H), 8.14 (dt, J = 9.0, 2.5 Hz, 2H), 7.88 (d, J = 9.0 Hz, 2H), 7.71 (t, J = 5.4 Hz, 1H), 5.95-5.86 (m, 1H), 5.31 (ddt, J = 17.2, 1.4, 1.4 Hz, 1H), 5.22 (ddt, J= 10.4, 1.4, 1.4 Hz, 1H), 4.86-4.82 (m, 1H), 4.59 (dt, J = 5.6, 1.4 Hz, 2H), 3.78-3.60 (m, 18H), 3.32-3.27 (m, 2H), 2.63 (t, J = 6.5 Hz, 2H), 2.44-2.41 (m, 2H), 1.92-1.86 (m, 2H), 1.50 (s, 9H); LC-MS: [M+H]⁺ = 678.27.

### (2) Production of CPA-302

A mixture of compound (CPA-301) produced above (36 mg), morpholine (9.3 µL), Pd(PPh₃)₄ (3.1 mg), and THF (1 mL) was stirred at room temperature for 6 hours. Morpholine (4.6 µL) and Pd(PPh₃)₄ (3.1 mg) were further added thereto, followed by stirring at room temperature for 1 hour. The solvent was then distilled off. The residue was purified by column chromatography (ODS, water/MeCN) to give the desired product (27 mg).
1H NMR (400 MHz, DMSO-d₆) δ 10.54 (s, 1H), 9.09 (t, J = 5.7 Hz, 1H), 8.05 (dt, J = 9.2, 2.4 Hz, 2H), 7.91 (dt, J = 9.2, 2.3 Hz, 2H), 6.91 (t, J = 5.2 Hz, 1H), 3.60-3.42 (m, 18H), 2.98 (dt, J = 6.4, 6.4 Hz, 2H), 2.66 (t, J = 6.4 Hz, 1H), 2.37 (t, J = 7.4 Hz, 2H), 2.29 (t, J = 6.4 Hz, 2H), 1.71 (tt, J = 6.4, 6.4 Hz, 2H), 1.37 (s, 9H) ;
LC-MS: [M+H]⁺ = 638.23.

### Production Example 15: Production of Compound (CPA-306)

Compound (CPA-306) was produced according to the method shown in the following scheme.

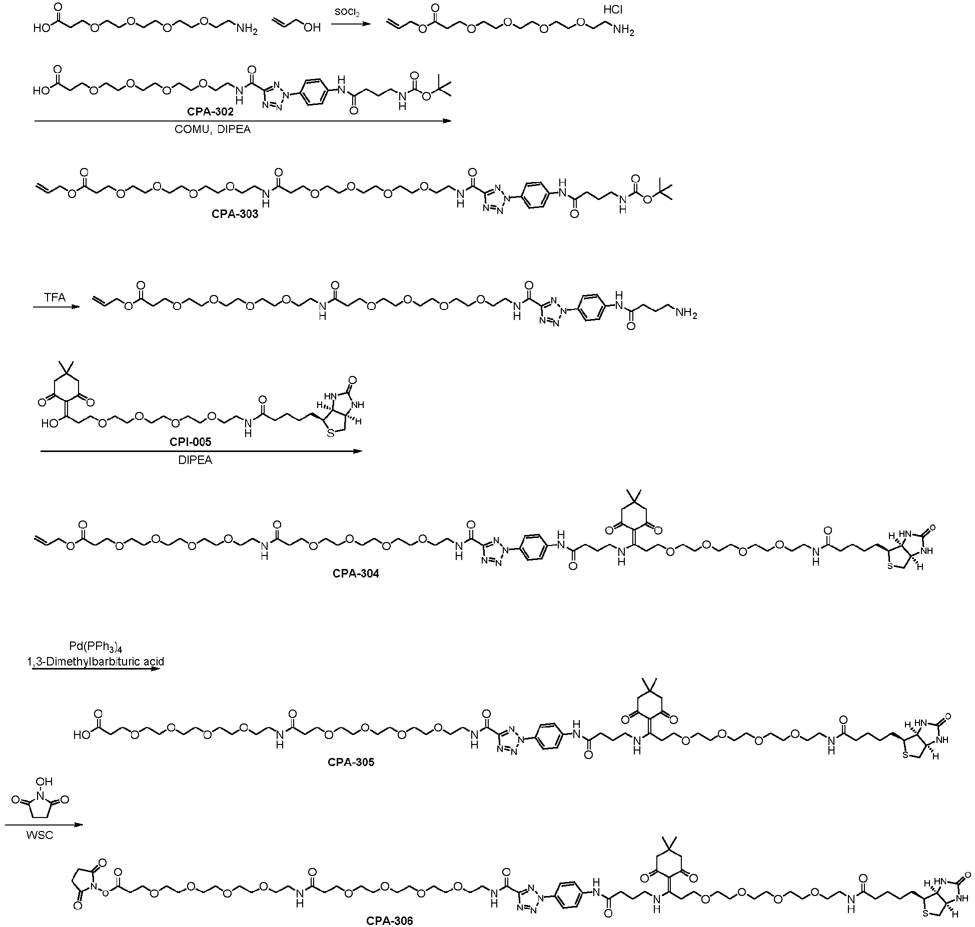

### (1) Production of CPA-303

A mixture of 3-[2-[2-[2-(2-aminoethoxy)ethoxy]ethoxy]ethoxy]propionic acid (11 mg), thionyl chloride (8.8 µL), and allyl alcohol (35 µL) was stirred at room temperature for 1 hour. After water was added thereto, the mixture was concentrated under reduced pressure. The residue and compound (CPA-302) produced in Production Example 14 (22 mg) were dissolved in DMF (1 mL), and COMU (16 mg) and DIPEA (18 µL) were added thereto. The mixture was stirred at room temperature for 5 hours, and the solvent was then distilled off. The residue was purified by column chromatography (ODS, water/MeCN) to give the desired compound (17 mg).
1H NMR (400 MHz, CDCl₃) δ 9.63 (s, 1H), 8.13 (dt, J = 9.0, 2.3 Hz, 2H), 7.89 (d, J = 9.0 Hz, 2H), 7.78 (t, J = 5.3 Hz, 1H), 6.72 (s, 1H), 5.96-5.86 (m, 1H), 5.31 (ddt, J= 17.2, 1.4, 1.4 Hz, 1H), 5.23 (ddt, J = 10.4, 1.4 , 1.4 Hz, 1H), 4.89 (s, 1H), 4.59 (dt, J = 5.7, 1.4 Hz, 2H), 3.78-3.60 (m, 32H), 3.54 (t, J = 5.3 Hz, 2H), 3.43 (dt, J = 5.3, 5.3 Hz, 2H), 3.28 (dt, J = 5.9 Hz, 2H), 2.62 (t, J = 6.5 Hz, 2H), 2.48-2.42 (m, 4H), 1.93-1.87 (m, 2H), 1.49 (s, 9H); LC-MS: [M+H]⁺= 925.27.

### (2) Production of CPA-304

Compound (CPA-303) produced above (17 mg) was dissolved in DCM (1 mL). TFA (0.14 mL) was added thereto dropwise, and the mixture was stirred at room temperature for 2 hours. The solvent was then distilled off. DIPEA (13 µL) was added to a solution (1 mL) of the residue and compound (CPI-005) produced in Production Example 2 (17 mg) in DMF, and the mixture was stirred at 60°C for 4 hours. The solvent was then distilled off. The residue was purified by column chromatography (ODS, water/MeCN) to give the desired product (22 mg).
1H NMR (400 MHz, CDCl₃) δ 13.68 (t, J = 5.0 Hz, 1H), 9.50 (s, 1H), 8.10 (d, J = 9.0 Hz, 2H), 7.89-7.87 (m, 3H), 6.79-6.76 (m, 1H), 6.66-6.63 (m, 1H), 5.96-5.86 (m, 2H), 5.31 (ddt, J= 17.2, 1.4, 1.4 Hz, 1H), 5.23 (ddt, J = 10.4, 1.4, 1.4 Hz, 1H), 5.11-5.06 (m, 1H), 4.59 (dt, J = 5.6, 1.4 Hz, 2H), 4.54-4.51 (m, 1H), 4.34-4.31 (m, 1H), 3.78-3.50 (m, 50H), 3.43 (dt, J = 5.4, 5.4 Hz, 2H), 3.38 (dt, J = 5.3, 5.3 Hz, 2H), 3.33 (dt, J = 5.5, 5.5 Hz, 2H), 3.17-3.12 (m, 1H), 2.92 (dd, J = 12.8, 4.9 Hz, 1H), 2.74 (d, J = 12.8 Hz, 1H), 2.63 (t, J = 6.5 Hz, 2H), 2.58 (t, J = 7.1 Hz, 2H), 2.47 (t, J = 6.1 Hz, 2H), 2.34 (s, 4H), 2.18 (t, J = 7.2 Hz, 2H), 2.12 (t, J = 6.9 Hz, 2H), 1.82-1.60 (m, 6H), 1.47-1.40 (m, 2H), 1.01 (s, 6H); LC-MS: [M+H]⁺= 1420.69.

### (3) Production of CPA-305

A mixture of compound (CPA-304) produced above (10 mg), 1,3-dimethylbarbituric acid (2.2 mg), Pd(PPh₃)₄ (0.81 mg), and AcOEt/DCM (1:1, 0.5 mL) was stirred at room temperature for 1 hour, and the solvent was then distilled off. The residue was purified by column chromatography (ODS, water/MeCN) to give the desired product (9.5 mg).
1H NMR (400 MHz, DMSO-d₆) δ 13.53 (t, J = 10.4 Hz, 1H), 10.57 (s, 1H), 9.10 (t, J = 5.2 Hz, 1H), 8.06 (d, J = 9.1 Hz, 2H), 7.95 (s, 1H), 7.91 (d, J = 9.1 Hz, 2H), 7.84 (t, J = 5.4 Hz, 1H), 6.42 (s, 1H), 6.36 (s, 1H), 4.29 (dd, J = 5.6, 5.2 Hz, 1H), 4.13-4.10 (m, 1H), 3.63-3.25 (m, under the water signal), 3.17 (tt, J = 5.8, 5.8 Hz, 4H), 3.10-3.06 (m, 1H), 2.81 (dd, J = 12.4, 5.1 Hz, 1H), 2.58-2.49 (m, overlapped with the DMSO signal), 2.35-2.28 (m, 8H), 2.05 (t, J = 7.4 Hz, 2H), 1.93 (tt, J = 7.0 Hz, 2H), 1.64-1.40 (m, 4H), 1.34-1.23 (m, 2H), 0.94 (s, 6H);
LC-MS: [M+H]⁺= 1380.38.

### (4) Production of CPA-306

A mixture of compound (CPA-305) produced above (8.5 mg), NHS (1.4 mg), WSC·HCl (2.4 mg), and DMF (0.5 mL) was stirred at room temperature for 4 hours. NHS (1.4 mg) and WSC·HCl (2.4 mg) were further added thereto, and the mixture was stirred for 2 hours. The solvent was then distilled off. The residue was purified by column chromatography (ODS, water/MeCN) to give the desired product (7.7 mg).
1H NMR (400 MHz, DMSO-d₆) δ 13.55-13.52 (m, 1H), 10.38 (s, 1H), 9.12-9.07 (m, 1H), 8.08-7.80 (m, 6H), 6.41 (s, 1H), 6.35 (s, 1H), 4.31-4.28 (m, 1H), 4.13-4.10 (m, 1H), 3.71 (t, J = 6.0 Hz, 1H), 3.63-3.25 (m, under the water signal), 3.17 (tt, J = 6.0, 6.0 Hz, 4H), 3.10-3.06 (m, 1H), 2.92 (t, J = 6.0 Hz, 2H), 2.83-2.79 (m, 4H), 2.58-2.44 (m, overlapped with the DMSO signal), 2.34-2.28 (m, 8H), 2.05 (t, J = 7.4 Hz, 2H), 1.97-1.90 (m, 2H), 1.64-1.40 (m, 4H), 1.32-1.23 (m, 2H), 0.94 (s, 6H);
LC-MS: [M+2H]²⁺= 1478.07

### Production Example 16: Production of Compound (CPA-310)

Compound (CPA-310) was produced according to the method shown in the following scheme.

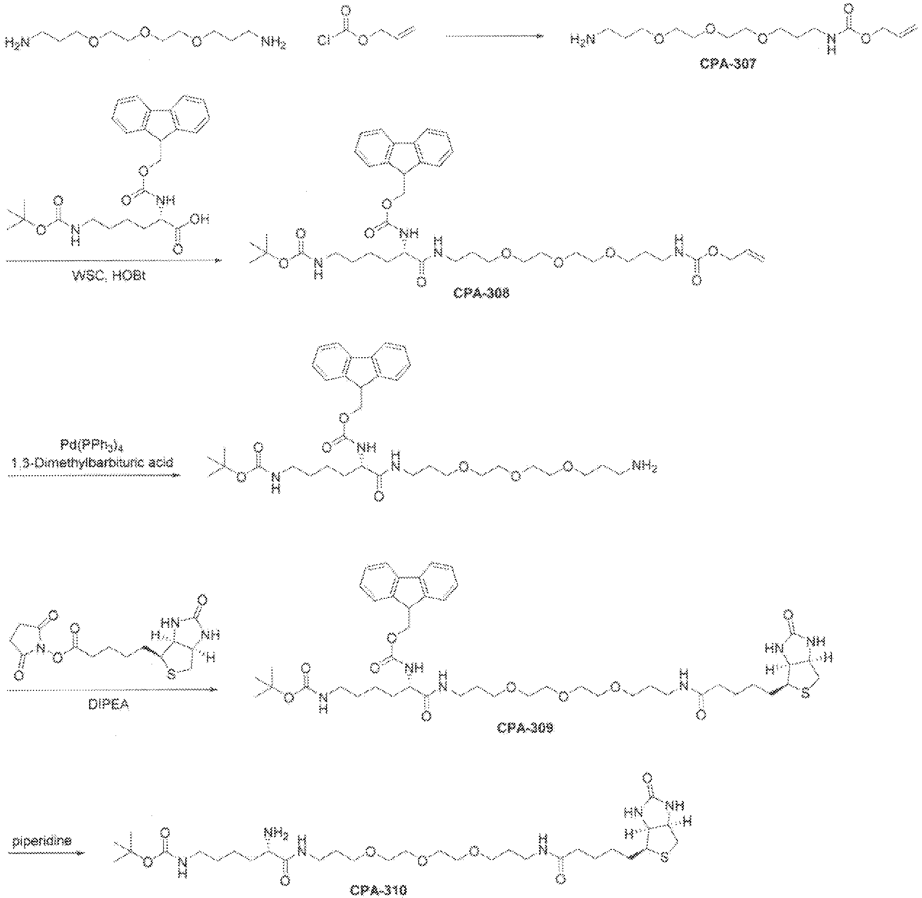

### (1) Production of CPA-307

Diethylene glycol bis(3-aminopropyl)ether (2.1 mL) was dissolved in DCM (200 mL), and allyl chloroformate (1.0 mL) was added thereto dropwise under ice-cooling, followed by stirring for 4 hours. The mixture was warmed to room temperature and stirred for 14 hours, and the solvent was then distilled off. The residue was purified by column chromatography (ODS, water/MeCN) to give the desired product (0.50 g).
1H NMR (400 MHz, DMSO-d₆) δ 8.26-7.37 (brs, 2H), 7.19 (t, J = 5.4, 1H), 5.95-5.85 (m, 1H), 5.26 (ddt, J = 13.8, 1.6, 1.6 Hz, 1H), 5.17 (ddt, J = 6.2, 1.6, 1.6 Hz, 1H), 4.45 (d, J = 5.3 Hz, 2H), 3.54-3.36 (m, 12H), 3.03 (dt, J = 6.6, 6.6 Hz, 2H), 2.83 (t, J = 7.0 Hz, 2H), 1.78 (tt, J = 6.6, 6.6 Hz, 2H), 1.62 (tt, J = 7.0, 7.0 Hz, 2H) ;
LC-MS: [M+H]⁺ = 305.16.

### (2) Production of CPA-308

Fmoc-Lys(Boc)-OH (0.77 g), WSC·HCl (0.38 g), and HOBt·H₂O (0.30 g) were added to a solution (20 mL) of compound (CPA-307) produced above (0.50 g) in DMF, at room temperature, and the mixture was stirred for 15 hours. The solvent was then distilled off. A saturated aqueous NaHCO₃ solution was added thereto, followed by extraction with AcOEt. The organic layer was washed with saturated saline and then dried over Na₂SO₄. The solvent was distilled off, and purification was performed by column chromatography (silica gel, AcOEt/MeOH) to give the desired product (0.80 g).
1H NMR (400 MHz, CDCl₃) δ 7.76 (d, J = 7.6 Hz, 2H), 7.60 (d, J = 7.4 Hz, 2H), 7.40 (t, J = 7.5 Hz, 2H), 7.31 (td, J = 7.4, 1.0 Hz, 2H), 6.83-6.73 (brs, 1H), 5.92-5.84 (m, 1H), 5.72-5.62 (brs, 1H), 5.44-5.34 (brs, 1H), 5.26 (d, J = 17.4 Hz, 1H), 5.17 (d, J = 10.6 hz, 2H), 4.75-4.63 (brs, 1H), 4.52 (d, J = 5.3 Hz, 2H), 4.42 (d, J = 7.0 Hz, 2H), 4.21 (t, J = 6.9 Hz, 1H), 4.14-4.10 (m, 1H), 3.59-3.43 (m, 13H), 3.35-3.25 (m, 3H), 3.11-3.08 (brm, 2H), 1.85-1.73 (m, 4H), 1.66-1.60 (m, 1H), 1.50-1.35 (m, 13H);
LC-MS: [M+H]⁺= 755.21.

### (3) Production of CPA-309

Pd(PPh₃)₄ (0.12 g) was added to a solution of compound (CPA-308) produced above (0.80 g) and 1,3-dimethylbarbituric acid (0.33 g) in DCM (10 mL), and the mixture was stirred at room temperature for 2 hours. The solvent was then distilled off. The residue was purified by column chromatography (silica gel, AcOEt/MeOH) to give an intermediate (0.41 g). Biotin-NHS (0.31 g), DIPEA (0.16 mL), and DMF (10 mL) were added to the intermediate (0.41 g), and the mixture was stirred at room temperature for 2 hours. The solvent was then distilled off. The residue was purified by column chromatography (ODS, water/MeCN) to give the desired product (0.15 g).
¹H NMR (400 MHz, DMSO-d₆) δ 7.89 (d, J = 7.5 Hz, 2H), 7.85 (t, J = 5.5 Hz, 1H), 7.74-7.72 (m, 3H), 7.44-7.40 (m, 3H), 7.33 (td, J = 7.4, 0.9 Hz, 2H), 6.76 (t, J = 5.3 Hz, 1H), 6.42 (s, 1H), 6.35 (s, 1H), 4.31-4.19 (m, 4H), 4.13-4.10 (m, 1H), 3.91-3.86 (m, 1H), 3.50-3.44 (m, 8H), 3.39-3.33 (m, 4H), 3.14-3.03 (m, 5H), 2.91-2.85 (m, 2H), 2.81 (dd, J = 12.4, 5.1 Hz, 1H), 2.57 (d, J = 12.4 Hz, 1H), 2.04 (t, J = 7.4 Hz, 2H), 1.64-1.43 (m, 10H), 1.36-1.21 (m, 15H); LC-MS: [M+H]⁺ = 897.11.

### (4) Production of CPA-310

A mixture of compound (CPA-309) produced above (0.15 g), piperidine (24 µL), and DMF (3 mL) was stirred at room temperature for 2 hours, and the solvent was then distilled off. The residue was purified by column chromatography (ODS, water/MeCN) to give the desired product (0.11 mg).
1H NMR (400 MHz, DMSO-d₆) δ 7.81 (t, J = 5.7 Hz, 1H), 7.75 (t, J = 5.5 Hz, 1H), 6.75 (t, J = 5.5 Hz, 1H), 6.42 (s, 1H), 6.35 (s, 1H), 4.32-4.29 (m, 1H), 4.14-4.11 (m, 1H), 3.53-3.45 (m, 8H), 3.41-3.33 (m, 4H), 3.13-3.03 (m, 6H), 2.90-2.80 (m, 3H), 2.57 (d, J = 12.4 Hz, 1H), 2.04 (t, J = 7.4 Hz, 2H), 1.66-1.57 (m, 5H), 1.54-1.43 (m, 4H), 1.37-1.20 (m, 16H); LC-MS: [M+H]⁺ = 675.21.

### Production Example 17: Production of Compound (CPA-311)

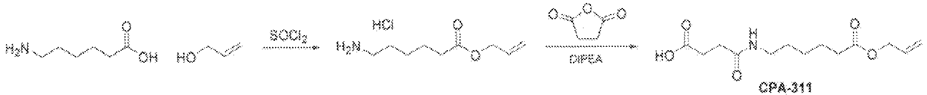

Thionyl chloride (0.33 mL) was added dropwise to 6-aminocaproic acid (0.20 g) and allyl alcohol (1.0 mL), and the mixture was stirred at room temperature for 1 hour. The solvent was then distilled off. Succinic anhydride (0.18 g), DIPEA (0.80 mL), and DMF (5 mL) were added to the residue, and the mixture was stirred at room temperature for 20 hours. Succinic anhydride (0.15 g) and DIPEA (0.4 mL) were further added thereto, and the mixture was stirred at room temperature for 1 hour. The solvent was then distilled off. The residue was purified by column chromatography (ODS, water/MeCN) to give the desired product (0.40 g).
1H NMR (400 MHz, CDCl₃) δ 6.02 (s, 1H), 5.97-5.87 (m, 1H), 5.32 (ddt, J = 14.1, 1.4, 1.4 Hz, 1H), 5.24 (ddt, J = 10.4, 1.4, 1.4 Hz, 1H), 4.58 (dt, J = 5.8, 1.4 Hz, 2H), 3.27 (dt, J = 6.6, 6.6 Hz, 2H), 2.71-2.68 (m, 2H), 2.52-2.49 (m, 2H), 2.36 (t, J = 7.3 Hz, 2H), 1.65 (tt, J = 7.5, 7.5 Hz, 2H), 1.53 (tt, J = 7.3, 7.3 Hz, 2H), 1.40-1.32 (m, 2H);
LC-MS: [M+H]⁺= 272.21.

### Production Example 18: Production of Compound (CPA-314)

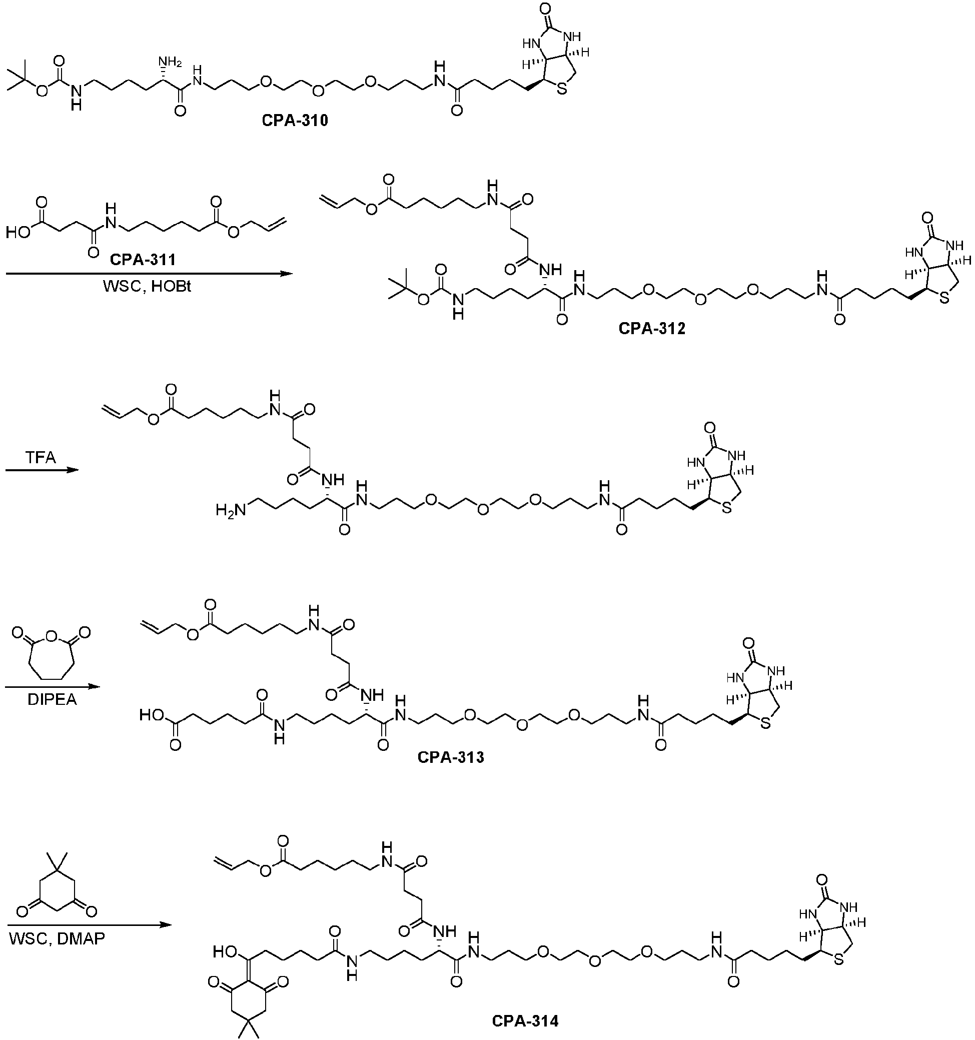

### (1) Production of CPA-312

Compound (CPA-310) produced in Production Example 16 (0.11 g) and compound (CPA-311) produced in Production Example 17 (43 mg) were dissolved in DMF (4 mL) . WSC·HCl (37 mg) and HOBt·H₂O (29 mg) were added thereto, and the mixture was stirred at room temperature for 14 hours. The solvent was then distilled off. The residue was purified by column chromatography (ODS, water/MeCN) to give the desired product (0.13 g).
1H NMR (400 MHz, DMSO-d₆) δ 7.95 (d, J = 8.0 Hz, 1H), 7.86-7.82 (m, 2H), 7.74 (t, J = 5.5 Hz, 1H), 6.74 (t, J = 5.4 Hz, 1H), 6.42 (s, 1H), 6.35 (s, 1H), 5.96-5.86 (m, 1H), 5.28 (ddt, J = 17.3, 1.5, 1.5 Hz, 1H), 5.20 (ddt, J = 10.5, 1.5, 1.5 Hz, 1H), 4.54 (dt, J = 5.4, 1.4 Hz, 2H), 4.32-4.29 (m, 1H), 4.14-4.06 (m, 2H), 3.52-3.45 (m, 8H), 3.40-3.33 (m, 4H), 3.12-2.98 (m, 7H), 2.89-2.80 (m, 3H), 2.57 (d, J = 12.4 Hz, 1H), 2.37-2.25 (m, 6H), 2.04 (t, J = 7.4 Hz, 2H), 1.65-1.19 (m, 31H); LC-MS: [M+H]⁺ = 928.30.

### (2) Production of CPA-313

TFA (1.1 mL) was added dropwise to a solution of compound (CPA-312) produced above (0.13 g) in DCM (3 mL), and the mixture was stirred at room temperature for 1 hour. The solvent was then distilled off. The residue was diluted with DMF (2 mL). Thereafter, DIPEA (0.12 mL) and adipic anhydride (36 mg) were added thereto, and the mixture was stirred at room temperature for 2 hours. Stirring was stopped, and the mixture was allowed to stand for 4 days. The solvent was then distilled off. The residue was purified by column chromatography (ODS, water/MeCN) to give the desired product (75 mg).
1H NMR (400 MHz, DMSO-d₆) δ 8.02-7.95 (m, 1H), 7.89-7.84 (m, 2H), 7.78-7.75 (m, 2H), 6.43 (s, 1H), 6.36 (s, 1H), 5.96-5.86 (m, 1H), 5.28 (ddt, J = 17.2, 1.5, 1.5 Hz, 1H), 5.20 (ddt, J = 10.5, 1.5 1.5 Hz, 1H), 4.53 (dt, J = 5.5, 1.5, 2H), 4.32-4.29 (m, 1H), 4.14-4.06 (m, 2H), 3.52-3.33 (m, 12H), 3.12-2.96 (m, 9H), 2.82 (dd, J = 12.5, 5.2 Hz, 1H), 2.57 (d, J = 12.5 Hz, 1H), 2.38-2.27 (m, 6H), 2.21-2.16 (m, 2H), 2.06-2.02 (m, 4H), 1.65-1.21 (m, 26H); LC-MS: [M+H]⁺ = 956.19.

### (3) Production of CPA-314

Compound (CPA-313) produced above (75 mg) was dissolved in DMF (2 mL). Dimedone (12 mg), WSC• HCl (16 mg), and DMAP (9.6 mg) were added thereto, and the mixture was stirred at room temperature for 14 hours. The solvent was then distilled off. The residue was purified by column chromatography (ODS, water/MeCN) to give the desired product (31 mg).
1H NMR (400 MHz, DMSO-d₆) δ 7.99 (d, J = 8.0 Hz, 1H), 7.89-7.82 (m, 2H), 7.74 (t, J = 10.6 Hz, 2H), 6.61 (d, J = 6.0 Hz, 1H), 6.42 (s, 1H), 6.35 (s, 1H), 5.96-5.86 (m, 1H), 5.28 (ddt, J = 17.2, 1.6, 1.6 Hz, 1H), 5.20 (ddt, J = 10.5, 1.5, 1.5 Hz, 1H), 4.53 (dt, J = 5.4, 1.4 Hz, 2H), 4.32-4.28 (m, 1H), 4.14-4.06 (m, 2H), 3.52-3.43 (m, 8H), 3.40-3.33 (m, 4H), 3.12-2.96 (m, 11H), 2.86-2.79 (m, 3H), 2.57 (d, J = 12.5 Hz, 1H), 2.36-2.30 (m, 8H), 2.06-1.96 (m, 4H), 1.65-1.21 (m, 26H), 0.97 (s, 6H);
LC-MS: [M+2H]²⁺ = 1079.50.

### Production Example 19: Production of Compound (CPA-318)

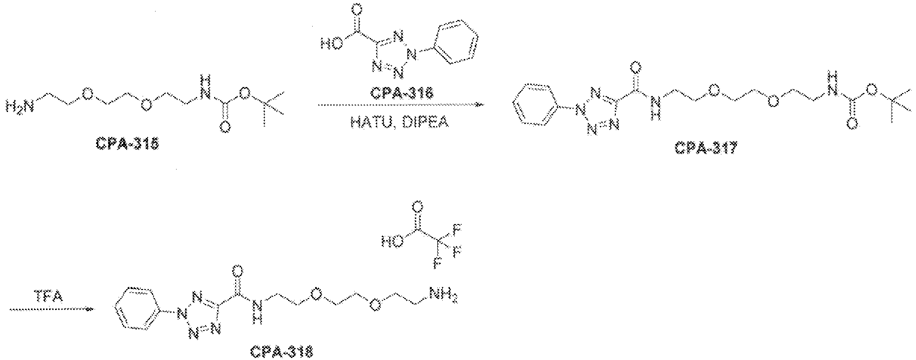

### (1) Production of CPA-317

Compound (CPA-315) shown in the above scheme (0.11 g) and compound (CPA-316) shown in the above scheme (0.087g) were dissolved in DMF (5 mL). HATU (0.209 g) and DIPEA (0.160 ml) were added thereto at room temperature, and the mixture was stirred for 20 hours. The solvent was then distilled off. The residue was purified by column chromatography (silica gel, hexane/AcOEt) to give the desired product (0.11 mg).
1H NMR (400 MHz, CDCl₃) δ 8.22-8.19 (m, 2H), 7.74-7.64 (brs, 1H), 7.61-7.52 (m, 3H), 5.15-5.02 (brs, 1H), 3.80-3.71 (m, 4H), 3.69-3.64 (m,4H), 3.58 (t, J = 5.2 Hz, 2H), 3.36-3.33 (m, 2H), 1.43 (s, 9H); LC-MS: [M+H]⁺ = 421.08.

### (2) Production of CPA-318

Compound (CPA-317) produced above (0.11 mg) was dissolved in DCM (2 mL). TFA (0.97 mL) was added thereto dropwise, and the mixture was stirred at room temperature for 1 hour. The solvent was then distilled off. The residue was purified by column chromatography (ODS, water/MeCN) to give the desired product (89 mg) .
1H NMR (400 MHz, DMSO-d₆) δ 9.15 (t, J = 5.4 Hz, 1H), 8.15-8.12 (m, 2H), 7.74-7.64 (m, 6H), 3.61-3.58 (m, 8H), 3.54-3.49 (m, 2H), 2.97 (t, J = 5.2 Hz, 2H); LC-MS: [M+H]⁺ = 321.06.

### Production Example 20: Production of Compound (CPA-321)

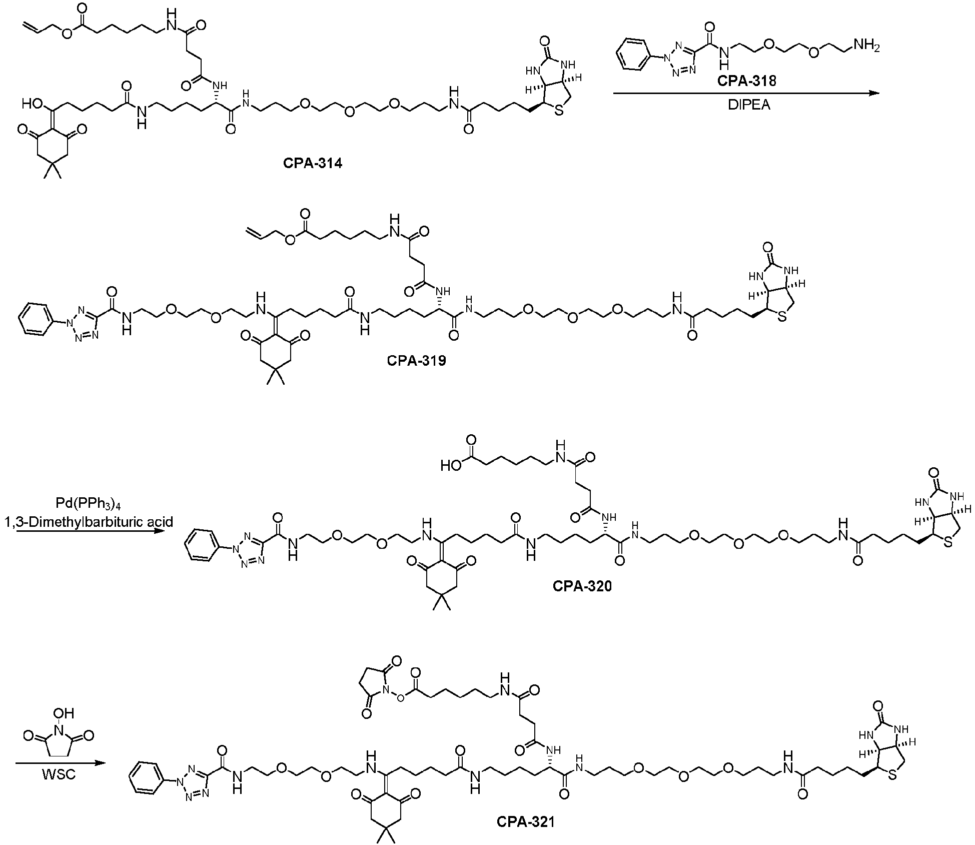

### (1) Production of CPA-319

Compound (CPA-318) produced in Production Example 19 (12 mg) and compound (CPA-314) produced in Production Example 18 (30 mg) were dissolved in DMF (1 mL). DIPEA (19 µL) was added thereto, and the mixture was stirred at 60°C for 2 hours. Purification was performed by column chromatography (ODS, water/MeCN) to give the desired product (30 mg).
1H NMR (400 MHz, DMSO-d₆) δ 13.35 (t, J = 4.5, 1H), 9.08 (t, J = 5.9 Hz, 1H), 8.13-8.10 (m, 2H), 7.96 (d, J = 8.0 Hz, 1H), 7.86-7.82 (m, 2H), 7.75-7.63 (m, 5H), 6.42 (s, 1H), 6.35 (s, 1H), 5.95-5.85 (m, 1H), 5.28 (ddt, J = 17.2, 1.5, 1.5 Hz, 1H), 5.20 (ddt, J = 10.4, 1.5, 1.5 Hz, 1H), 4.53 (dt, J = 5.5, 1.5 Hz, 2H), 4.32-4.28 (m, 1H), 4.13-4.05 (m, 2H), 3.63-3.60 (m, 8H), 3.50-3.43 (m, 10H), 3.39-3.33 (m, 4H), 3.11-2.90 (m, 11H), 2.81 (dd, J = 12.4, 5.1 Hz, 1H), 2.57 (d, J = 12.4 Hz., 1H), 2.36-2.27 (m, 8H), 2.23 (s, 4H), 2.11-2.02 (m, 4H), 1.65-1.21 (m, 26H), 0.91 (s, 6H);
LC-MS: [M+H]⁺ = 1380.98.

### (2) Production of CPA-320

A mixture of compound (CPA-319) produced above (29 mg), 1,3-dimethylbarbituric acid (6.6 mg), Pd(PPh₃)₄ (2.4 mg), and DCM (1 mL) was stirred at room temperature for 1 hour, and the solvent was then distilled off. The residue was purified by column chromatography (ODS, water/MeCN) to give the desired product (26 mg) .
1H NMR (400 MHz, DMSO-d₆) δ 13.35 (t, J = 4.3 Hz, 1H), 9.09 (t, J = 5.5, 1H), 8.13-8.10 (m, 3H), 7.99-7.89 (brs, 1H), 7.85 (t, J = 5.5 Hz, 1H), 7.78-7.75 (m, 2H), 7.72-7.63 (m, 3H), 6.43 (s, 1H), 6.36 (s, 1H), 4.32-4.28 (m, 1H), 4.14-4.05 (m, 2H), 3.63-3.60 (m, 8H), 3.50-3.45 (m, 10H), 3.39-3.33 (m, 4H), 3.11-2.90 (m, 11H), 2.82 (dd, J = 12.4, 5.1 Hz, 1H), 2.57 (d, J = 12.4 Hz, 1H), 2.36-2.27 (m, 6H), 2.23 (s, 4H), 2.16-2.02 (m, 6H), 1.65-1.21 (m, 26H), 0.91 (s, 6H); LC-MS: [M+H]⁺ = 1339.89.

### (3) Production of CPA-321

A mixture of compound (CPA-320) produced above (25 mg), NHS (4.3 mg), WSC•HCl (7.2 mg), and DMF (0.4 mL) was stirred at room temperature for 3 hours. NHS (4.3 mg) and WSC•HCl (7.2 mg) were further added thereto, and the mixture was stirred at room temperature for another 3 hours. Purification was performed by column chromatography (ODS, water/MeCN) to give the desired product (21 mg) .
1H NMR (400 MHz, DMSO-d₆) δ 13.35 (t, J = 4.4 Hz, 1H), 9.08 (t, J = 5.6 Hz, 1H), 8.13-8.10 (m, 2H), 7.96 (d, J = 7.9 Hz, 1H), 7.87-7.83 (m, 2H), 7.75-7.63 (m, 5H), 6.42 (s, 1H), 6.35 (s, 1H), 4.32-4.28 (m, 1H), 4.14-4.05 (m, 2H), 3.63-3.60 (m, 8H), 3.50-3.43 (m, 10H), 3.39-3.33 (m, 4H), 3.11-2.90 (m, 11H), 2.84-2.79 (m, 5H), 2.65 (t, J = 7.4 Hz, 2H), 2.57 (d, J = 12.4 Hz, 1H), 2.38-2.28 (m, 6H), 2.23 (s, 4H), 2.09-2.02 (m, 4H), 1.65-1.21 (m, 26H), 0.91 (s, 6H); LC-MS: [M+H]⁺ = 1437.12.

### Production Example 21: Synthesis of CPF-242

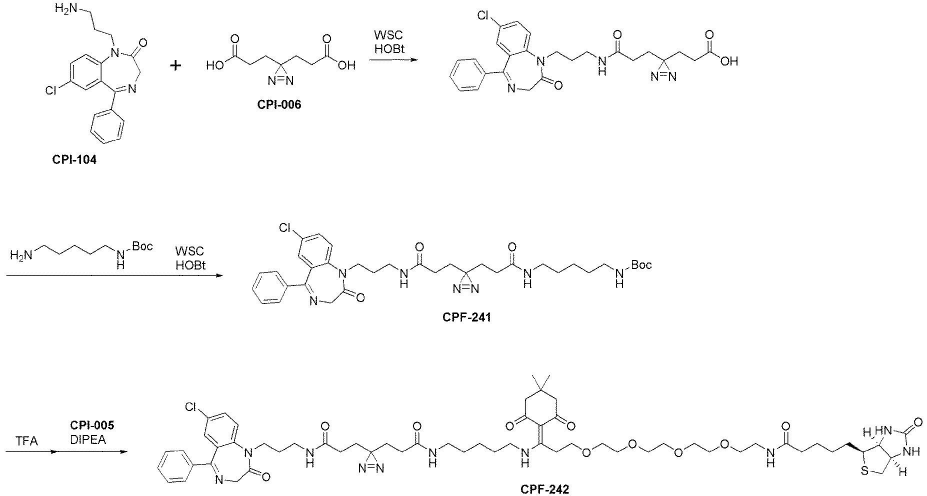

### (1) Synthesis of CPF-241

CPI-006 (6.6 mg), WSC•HCl (6.8 mg), and HOBt•H₂O (5.4 mg) were added to a mixture of CPI-104 (14 mg) and DMF (1 mL), and the mixture was stirred at room temperature for 3 hours. The solvent was distilled off, and the residue was purified by column chromatography (ODS, water/MeCN). N-Boc-1,5-diaminopentane (12 µL), WSC•HCl (11 mg), and HOBt•H₂O (9.0 mg) were added to a mixture of the purified product (8.9 mg) and DMF (1 mL), and the mixture was stirred at room temperature for 16 hours. The solvent was distilled off, and the residue was purified by column chromatography (ODS, water/MeCN) to give the desired product (7.4 mg).
¹H NMR (400 MHz, DMSO-d6) δ 7.77 (t, J = 5.4 Hz, 1H), 7.72-7.65 (m, 3H), 7.57-7.50 (m, 3H), 7.47-7.43 (m, 2H), 7.20 (d, J = 2.3 Hz, 1H), 6.75 (t, J = 5.5 Hz, 1H), 4.55 (d, J = 10.5 Hz, 1H), 4.25-4.18 (m, 1H), 3.77 (d, J = 10.5 Hz, 1H), 3.70-3.63 (m, 1H), 2.98 (dt, J = 6.5, 6.5 Hz, 2H), 2.90-2.80 (m, 4H), 1.86-1.77 (m, 4H), 1.58-1.31 (m, 19H), 1.24-1.18 (m, 2H);
LC-MS: [M+H]⁺ = 680.53.

### (2) Synthesis of CPF-242

TFA (149 µL) was added to a mixture of compound (CPF-241) produced above (6.6 mg) and DCM (1 mL), and the mixture was stirred at room temperature for 2 hours. The solvent was distilled off. DMF (1 mL), CPI-005 (6.0 mg), and DIPEA (17 µL) were added to the residue, and the mixture was stirred at 60°C for 3 hours. The solvent was distilled off, and the residue was purified by column chromatography (ODS, water/MeCN) to give the desired product (7.9 mg) .
¹H NMR (400 MHz, DMSO-d₆) δ 13.46 (t, J = 4.9 Hz, 1H), 7.84-7.79 (m, 2H), 7.72-7.65 (m, 3H), 7.57-7.50 (m, 3H), 7.47-7.43 (m, 2H), 7.20 (d, J = 2.4 Hz, 1H), 6.41 (s, 1H), 6.35 (s, 1H), 4.55 (d, J = 10.5 Hz, 1H), 4.31-4.28 (m, 1H), 4.25-4.18 (m, 1H), 4.14-4.10 (m, 1H), 3.76 (d, J = 10.5 Hz, 1H), 3.70-3.58 (m, 3H), 3.52-3.48 (m, 14H), 3.42-2.99 (m, overlapped with water signal), 2.85-2.79 (m, 3H), 2.59-2.49 (m, overlapped with DMSO signal), 2.28 (s, 4H), 2.06 (t, J = 7.4 Hz, 2H), 1.87-1.77 (m, 4H), 1.65-1.23 (m, 18H), 0.94 (s, 6H) ;
LC-MS: [M+H]⁺ = 1175.70.

### Production Example 22: Production of CPA-325

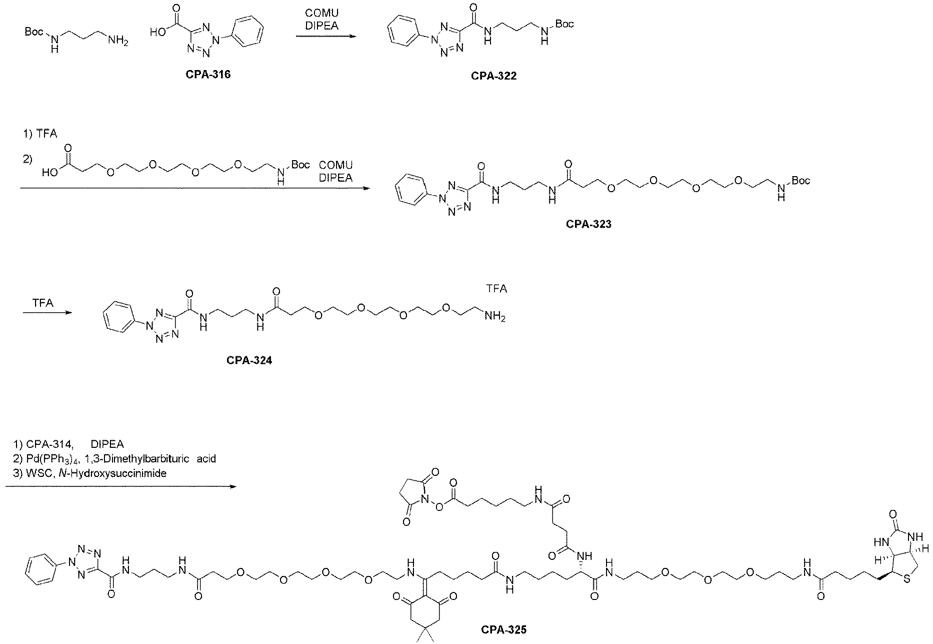

### (1) Production of CPA-322

Compound (CPA-316) shown in the above scheme (40 mg), N-Boc-1,3-diaminopropane (61 µL), and COMU (99 mg) were dissolved in DMF (1 mL). DIPEA (121 µL) was added thereto, and the mixture was stirred at room temperature for 26 hours. The solvent was distilled off, and the residue was purified by column chromatography (ODS, water/MeCN) to give the desired product (53 mg).
¹H NMR (500 MHz, DMSO-d₆) δ 9.15 (br, 1H), 8.13 (d, J = 7.9 Hz, 2H), 7.72-7.64 (m, 3H), 6.84 (br, 1H), 3.30 (br, overlapped with water signal), 2.99 (br, 2H), 1.67 (quin, J = 6.5 Hz, 2H), 1.38 (s, 9H); LC-MS: [M+Na]⁺ = 369.41.

### (2) Production of CPA-323

Compound (CPA-322) produced above (51 mg) was dissolved in DCM (1 mL). TFA (113 µL) was added thereto under ice-cooling, and the mixture was stirred at room temperature for 1 hour. The solvent was distilled off. 15-(Boc-amino)-4,7,10,13-tetraoxapentadecanoic acid (56 mg), COMU (76 mg), DIPEA (85 µL), and DMF (1 mL) were added to the residue, and the mixture was stirred at room temperature for 12 hours. The solvent was distilled off, and the residue was purified by column chromatography (ODS, water/MeCN) to give the desired product (76 mg).
¹H NMR (500 MHz, DMSO-d₆) δ 9.15 (t, J = 5.7 Hz, 1H), 8.13 (d, J = 7.7 Hz, 2H), 7.89 (t, J = 5.4 Hz, 1H), 7.72-7.69 (m, 2H), 7.66 (t, J = 7.2 Hz, 1H), 6.74 (t, J = 5.2 Hz, 1H), 3.60 (t, J = 6.4 Hz, 2H), 3.49-3.46 (m, 12H), 3.37-3.36 (m, overlapped with water signal), 3.12 (q, J = 6.4 Hz, 2H), 3.05 (q, J = 5.9 Hz, 2H), 2.32 (t, J = 6.4 Hz, 2H), 1.68 (quin, J = 7.0 Hz, 2H), 1.36 (s, 9H); LC-MS: [M+H]⁺ = 594.60.

### (3) Production of CPA-324

Compound (CPA-323) produced above (67 mg) was dissolved in DCM (1 mL). TFA (130 µL) was added thereto, and the mixture was stirred at room temperature for 2 hours. The solvent was distilled off to give the desired product (68 mg).
¹H NMR (400 MHz, DMSO-d₆) δ 9.16 (t, J = 5.8 Hz, 1H), 8.15-8.11 (m, 2H), 7.92 (t, J = 5.6 Hz, 1H), 7.75-7.64 (m, 6H), 3.62-3.48 (m, 16H), 3.34 (dt, J = 6.7, 6.7 Hz, 2H), 3.12 (dt, J = 6.7, 6.7 Hz, 2H), 3.01-2.94 (m, 2H), 2.33 (t, J = 6.4 Hz, 2H), 1.69 (tt, J = 6.7, 6.7 Hz, 2H);
LC-MS: [M+H]⁺ = 494.38.

### (4) Production of CPA-325

The desired product was produced in the same manner as in Production Example 20, except that compound (CPA-324) produced above was used in place of CPA-318.
¹H NMR (400 MHz, DMSO-d₆) δ 13.35 (s, 1H), 9.15 (t, J = 5.9 Hz, 1H), 8.14-8.11 (m, 2H), 7.97 (d, J = 8.4 Hz, 1H), 7.91-7.83 (m, 3H), 7.76-7.63 (m, 5H), 6.42 (s, 1H), 6.35 (s, 1H), 4.30 (dd, J = 7.6, 5.2 Hz, 1H), 4.14-4.05 (m, 2H), 3.61-3.34 (m, 32 H), 3.17-2.91 (m, 13 H), 2.84-2.79 (m, 5H), 2.65 (t, J = 7.4 Hz, 2H), 2.57 (d, J = 12.4 Hz, 2H), 2.37-2.26 (m, 10H), 2.09-2.02 (m, 4H), 1.71-1.19 (m, 28H), 0.93 (s, 6H);
LC-MS: [M+2H]²⁺ = 1611.63.

### Production Example 23: Production of CPA-326

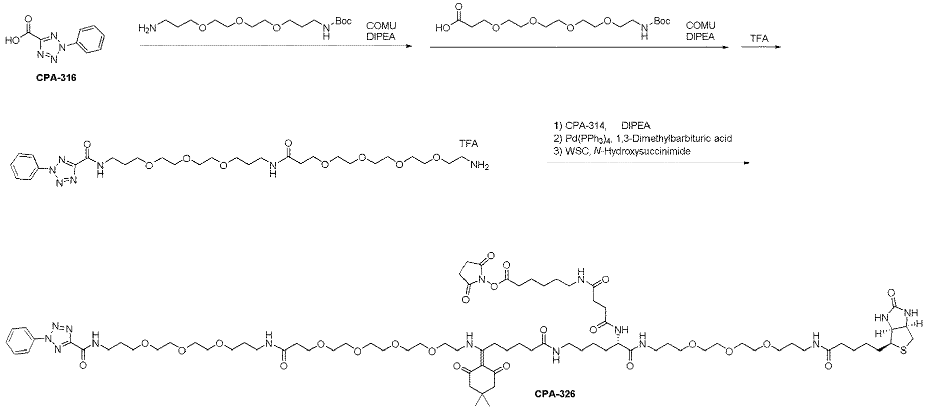

The desired product was produced in the same manner as in Production Example 21, except that N-Boc-3,3'-((oxybis(ethane-2,1-diyl))bis(oxy))bis(propan-1-amine) was used in place of N-Boc-1,3-diaminopropane.
¹H NMR (400 MHz, DMSO-d₆) δ 13.35 (s, 1H), 9.14 (t, J = 5.8 Hz, 1H), 8.14-8.11 (m, 2H), 7.96 (d, J = 8.0 Hz, 1H), 7.85 (dd, J = 9.9, 5.4 Hz, 2H), 7.79 (t, J = 5.6 Hz, 1H), 7.87-7.63 (m, 5H), 6.42 (s, 1H), 6.35 (s, 1H), 4.30 (dd, J = 7.6, 5.2 Hz, 1H), 4.14-4.05 (m, 2H), 3.61-3.34 (m, 40H), 3.11-2.92 (m, 15H), 2.84-2.79 (m, 5H), 2.65 (t, J = 7.4 Hz, 2H), 2.57 (d, J = 12.4, 1H), 2.37-2.26 (m, 12H), 2.09-2.02 (m, 4H), 1.83-1.77 (m, 2H), 1.65-1.19 (m, 28H), 0.93 (s, 6H); LC-MS: [M+2H]²⁺ = 1757.83.

### Production Example 24: Production of CPA-332

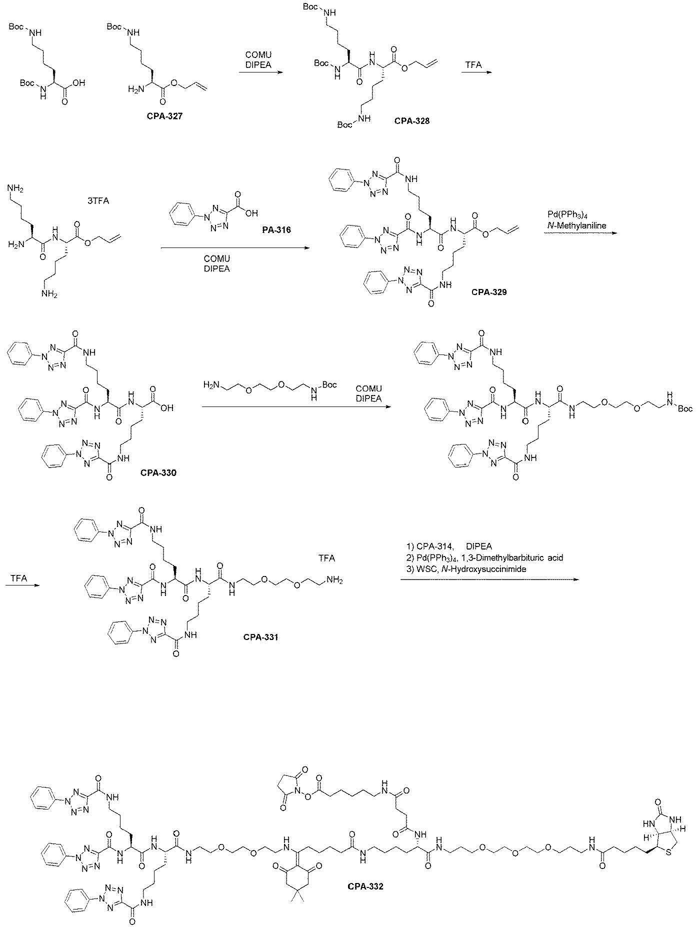

### (1) Production of CPA-328

Compound (CPA-327) shown in the above scheme (60 mg) and Nα,Nε-di-Boc-L-lysine (87 mg) were dissolved in DMF (2 mL). COMU (108 mg) and DIPEA (109 µL) were added thereto, and the mixture was stirred at room temperature for 1 hour. The solvent was distilled off, and the residue was purified by column chromatography (ODS, water/MeCN) to give the desired product (129 mg).
¹H NMR (500 MHz, DMSO-d₆) δ 8.13 (d, J = 7.4 Hz, 1H), 6.77-6.39 (m, 3H), 5.88 (ddt, J = 17.3, 10.5, 5.3 Hz, 1H), 5.30 (dd, J = 17.3, 1.6 Hz, 1H), 5.20 (dd, J = 10.5, 1.5 Hz, 1H), 4.59-4.52 (m, 2H), 4.22 (br, 1H), 3.91 (br, 1H), 2.92-2.82 (m, 4H), 1.72-1.66 (m, 1H), 1.64-1.52 (m, 2H), 1.49-1.42 (m, 1H), 1.37-1.24 (m, 35H);
LC-MS: [M+H]⁺ = 615.68.

### (2) Production of CPA-329

Compound (CPA-328) synthesized above (126 mg) was dissolved in DCM (2 mL). TFA (474 µL) was added thereto, and the mixture was stirred at room temperature for 1 hour. The solvent was distilled off. Compound (CPA-316) shown in the above scheme (128 mg), DMF (2 mL), COMU (288 mg), and DIPEA (235 µL) were added to the residue, the mixture was stirred at room temperature for 1 hour. The solvent was distilled off, and the residue was purified by column chromatography (ODS, water/MeCN) to give the desired product (132 mg).
¹H NMR (500 MHz, DMSO-d₆) δ 9.18 (t, J = 6.0 Hz, 1H), 9.15 (t, J = 6.0 Hz, 1H), 8.92 (d, J = 8.0 Hz, 1H), 8.52 (d, J = 7.3 Hz, 1H), 8.12-8.09 (m, 6H), 7.70-7.62 (m, 9H), 5.87 (ddt, J = 17.3, 10.5, 5.3 Hz, 1H), 5.29 (dq, J = 17.2, 1.6 Hz, 1H), 5.17 (dq, J = 10.5, 1.5 Hz, 1H), 4.60 (q, J = 7.3 Hz, 1H), 4.56 (dq, J = 5.4, 1.6 Hz, 2H), 4.32-4.27 (m, 1H), 3.32-3.30 (m, overlapped with water signal), 1.86-1.77 (m, 3H), 1.73-1.66 (m, 1H), 1.64-1.55 (m, 4H), 1.49-1.36 (m, 4H);
LC-MS: [M+H]⁺ = 831.77.

### (3) Production of CPA-330

Compound (CPA-329) produced above (42 mg) was dissolved in DCM (3 mL). Pd(PPh₃)₄ (5.8 mg) and N-methylaniline (27 µL) were added thereto, and the mixture was stirred at room temperature for 1 hour. The solvent was distilled off, and the residue was purified by column chromatography (ODS, water/MeCN) to give the desired product (31 mg) .
¹H NMR (500 MHz, DMSO-d₆) δ 12.60 (br, 1H), 9.18 (t, J = 5.8 Hz, 1H), 9.15 (t, J = 5.8 Hz, 1H), 8.92 (d, J = 8.0 Hz, 1H), 8.36 (d, J = 7.7 Hz, 1H), 8.12-8.07 (m, 5H), 7.80-7.77 (m, 1H), 7.71-7.62 (m, 9H), 4.60 (q, J = 7.5 Hz, 1H), 4.24-4.19 (m, 1H), 3.34-3.29 (m, overlapped with water signal), 1.87-1.76 (m, 3H), 1.70-1.54 (m, 5H), 1.49-1.35 (m, 4H);
LC-MS: [M+H]⁺ = 791.46.

### (4) Production of CPA-331

The desired product was produced in a manner similar to that in Production Example 22.
¹H NMR (400 MHz, DMSO-d₆) δ 9.18-9.14 (m, 2H), 8.99 (d, J = 8.0 Hz, 1H), 8.17-8.07 (m, 7H), 7.97 (t, J = 5.4 Hz, 1H), 7.71-7.62 (m, 12H), 4.56 (dt, J = 7.2, 7.2 Hz, 2H), 4.26 (dt, J = 7.2, 7.2 Hz, 2H), 3.59-3.53 (m, 6H), 3.42-3.16 (m, 8H), 3.00-2.96 (m, 2H), 1.86-1.80 (m, 2H), 1.70-1.28 (m, 10H);
LC-MS: [M+H]⁺ = 921.30.

### (5) Production of CPA-332

The desired product was produced in the same manner as in Production Example 21, except that CPA-331 was used in place of CPA-318.
¹H NMR (400 MHz, DMSO-d₆) δ 13.34 (s, 1H), 9.17-9.12 (m, 2H), 8.98 (d, J = 8.4 Hz. 1H), 8.17 (d, J = 8.1 Hz, 1H), 8.12-8.06 (m, 6H), 8.06-7.96 (m, 2H), 7.87-7.83 (m, 2H), 7.76-7.74 (m, 2H), 7.70-7.53 (m, 9H), 6.42 (s, 1H), 6.36 (s, 1H), 4.57 (dt, J = 7.5, 7.5 Hz, 1H), 4.32-4.24 (m, 2H), 4.14-4.05 (m, 2H), 3.60-2.90 (m, 37H), 2.84-2.79 (m, 5H), 2.66-2.50 (m, 3H), 2.36-2.25 (m, 10H), 2.09-2.02 (m, 4H), 1.86-1.80 (m, 2H), 1.68-1.20 (m, 36H), 0.91 (s. 6H); LC-MS: [M+2H]²⁺ = 2039.42.

### Production Example 25: Production of CPA-403

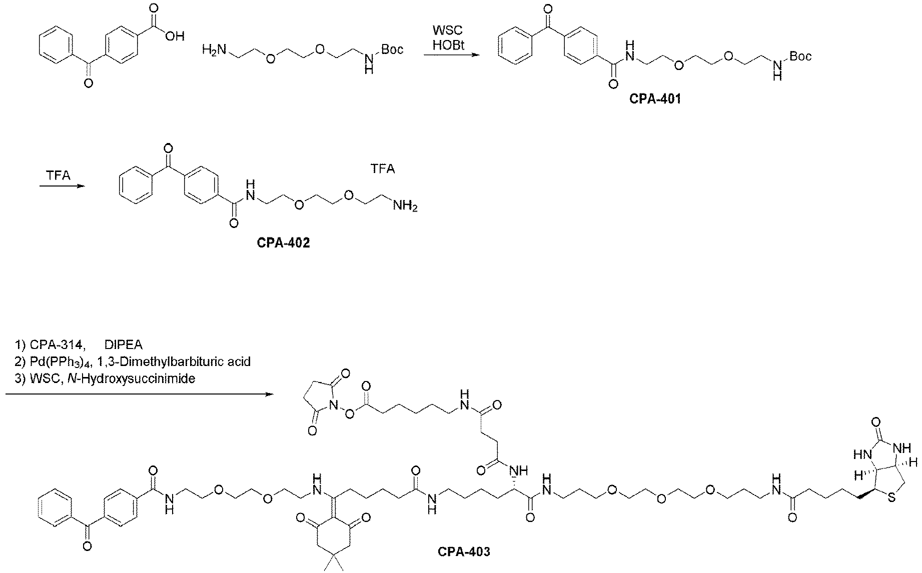

### (1) Production of CPA-401

WSC•HCl (42 mg) and HOBt•H₂O (34 mg) were added to a mixture of 4-benzoylbenzoic acid (50 mg), N-Boc-2,2'-(ethylenedioxy) diethylamine (55 mg), and DMF (3 mL), and the mixture was stirred at room temperature for 15 hours. The solvent was distilled off, and the residue was purified by column chromatography (silica gel, hexane/AcOEt) to give the desired product (61 mg).
¹H NMR (400 MHz, DMSO-d₆) δ 8.07-7.88 (brm, 2H), 7.86-7.79 (m, 4H), 7.62 (tt, J = 7.4, 1.2 Hz, 1H), 7.50 (t, J = 7.6 Hz, 2H), 6.99-6.88 (brs, 1H), 5.03-4.91 (brs, 1H), 3.71-3.56 (m, 10H), 3.36-3.23 (brm, 2H), 1.42 (s, 9H);
LC-MS: [M+H]⁺ = 457.45.

### (2) Production of CPA-402

TFA (515 µL) was added to a mixture of compound (CBI-712) produced above (61 mg) and DCM (2 mL), and the mixture was stirred at room temperature for 14 hours. The solvent was distilled off to give the desired product (63 mg).
¹H NMR (400 MHz, DMSO-d₆) δ 8.73 (t, J = 5.6 Hz, 1H), 8.01-7.98 (m, 2H), 7.82-7.69 (m, 8H), 7.61-7.57 (m, 2H), 3.60-3.56 (m, 8H), 3.49-3.38 (m, 2H), 3.00-2.93 (m, 2H);
LC-MS: [M+H]⁺ = 357.36.

### (3) Production of CPA-403

The desired product was produced in the same manner as in Production Example 22, except that CPA-402 was used in place of CPA-316.
¹H NMR (400 MHz, DMSO-d₆) δ 13.38-13.32 (brm, 1H), 8.72 (t, J = 5.6 Hz, 1H), 8.00-7.96 (m, 3H), 7.87-7.83 (m, 2H), 7.80-7.68 (m, 7H), 7.60-7.56 (m, 2H), 6.42 (s, 1H), 6.35 (s, 1H), 4.30 (dd, J = 7.6, 5.1 Hz, 1H), 4.14-4.05 (m, 3H), 3.62-3.36 (m, 22H), 3.11-2.91 (m, 11H), 2.84-2.79 (m, 5H), 2.65 (t, J = 7.2 Hz, 2H), 2.57 (d, J = 12.6 Hz, 1H), 2.37-2.24 (m, 10H), 1.68-1.19 (m, 26H), 0.91 (s, 6H); LC-MS: [M+2H]²⁺ = 1474.68.

### Production Example 26: Production of CPA-503

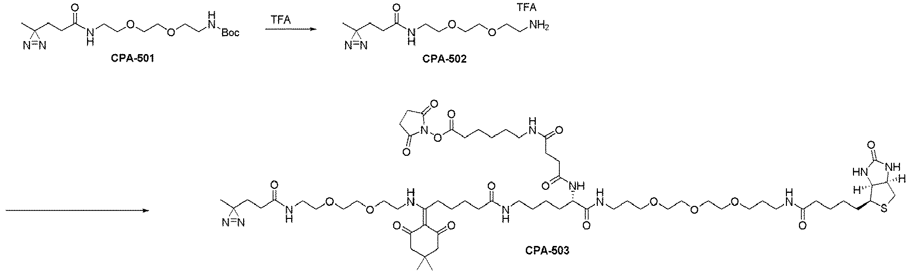

### (1) Production of CPA-502

Compound (CPA-501) shown in the above scheme (40 mg) was dissolved in DCM (2 mL). TFA (258 µL) was added thereto, and the mixture was stirred at room temperature for 1 hour. The solvent was distilled off to give the desired product (41 mg).
¹H NMR (400 MHz, DMSO-d₆) δ 7.93 (t, J = 5.4 Hz, 1H), 7.87-7.66 (brs, 3H), 3.60-3.52 (m, 6H), 3.41 (t, J = 6.0 Hz, 2H), 3.20 (dt, J = 5.9, 5.9 Hz, 2H), 2.97 (t, J = 5.2 Hz, 2H), 1.97 (m, 2H), 1.56 (m, 2H), 0.98 (s, 3H);
LC-MS: [M+H]⁺ = 259.28.

### (2) Production of CPA-503

The desired product was produced in the same manner as in Production Example 22, except that CPA-502 was used in place of CPA-316.
¹H NMR (400 MHz, DMSO-d₆) δ 13.35 (s, 1H), 7.98-7.84 (m, 4H), 7.77-7.74 (m, 2H), 6.42 (s, 1H), 6.36 (s, 1H), 4.30 (dd, J = 7.6, 5.2 Hz, 1H), 4.14-4.06 (m, 2H), 3.61-3.16 (m, 22H), 3.12-2.92 (m, 11H), 2.84-2.80 (m, 5H), 2.65 (t, J = 7.3 Hz, 2H), 2.59-2.56 (m, 1H), 2.37-2.27 (m, 10H), 2.10-2.02 (m, 4H), 1.97 (t, J = 7.7 Hz), 1.65-1.23 (m, 28H), 0.97 (s, 3H), 0.94 (s, 6H);
LC-MS: [M+2H]²⁺ = 1376.31.

### Production Example 27: Production of CPA-334

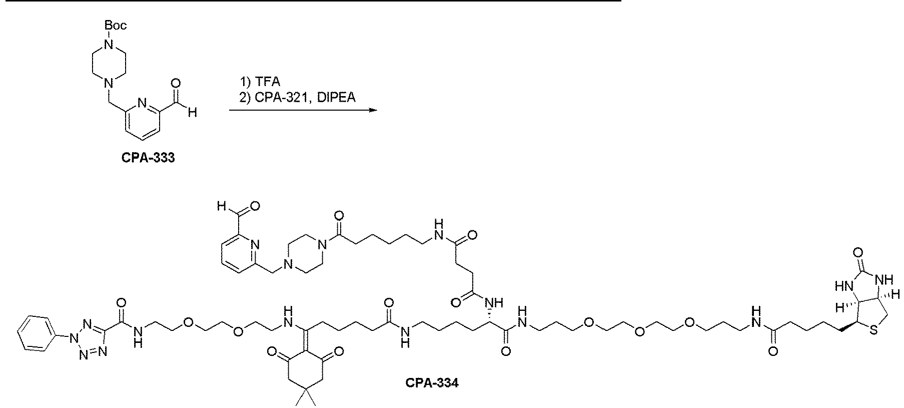

TFA (80 µL) was added to a mixture of compound (CPA-333) shown in the above scheme and DCM (1 mL), and the mixture was stirred at room temperature for 1 hour. The solvent was distilled off. Compound (CPA-321) shown in the above scheme (15 mg), DMF (1 mL), and DIPEA (5.5 µL) were added to the residue, and the mixture was stirred at room temperature for 8 hours. DIPEA (1.8 µL) was further added thereto, and the mixture was stirred at room temperature for 14 hours. The solvent was distilled off, and the residue was purified by column chromatography (ODS, water/MeCN) to give the desired product (12 mg).
¹H NMR (400 MHz, DMSO-d₆) δ 13.34 (t, J = 4.5 Hz, 1H), 9.96 (d, J = 0.7 Hz, 1H), 9.08 (t, J = 5.7 Hz, 1H), 8.13-8.10 (m, 2H), 8.04 (t, J = 7.5 Hz, 1H), 7.96 (d, J = 8.0 Hz, 1H), 7.87-7.82 (m, 3H), 7.78-7.63 (m, 6H), 6.42 (s, 1H), 6.35 (s, 1H), 4.30 (dd, J = 7.5, 5.3 Hz, 1H), 4.14-4.05 (m, 2H), 3.63-3.60 (m, 8H), 3.50-3.29 (m, overlapped with water signal), 3.11-2.89 (m, 11H), 2.83-2.79 (m, 1H), 2.59-2.23 (m, overlapped with DMSO signal), 2.09-2.02 (m, 4H), 1.65-1.24 (m, 26H), 0.91 (s, 6H);
LC-MS: [M+H]⁺ = 1528.03.

### Production Example 28: Production of CPI-007

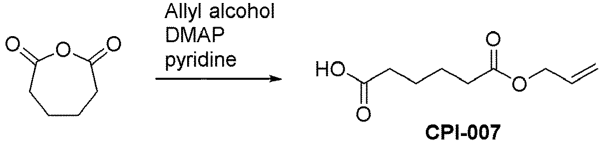

Allyl alcohol (96 µL), DMAP (9.5 mg), and pyridine (170 µL) were added to a mixture of adipic anhydride (100 mg) and DCM (5 mL) under ice-cooling, and the mixture was stirred for 1 hour under ice-cooling. The mixture was warmed to room temperature and stirred for 15 hours. The reaction mixture was washed with a 2M aqueous HCl solution and saturated saline and then dried over Na₂SO₄. The solvent was distilled off to give the desired product (101 mg).
¹H NMR (400 MHz, CDCl₃) δ 5.97-5.87 (m, 1H), 5.34-5.29 (m, 1H), 5.26-5.22 (m, 1H), 4.58 (ddt, J = 5.7, 1.3, 1.3 Hz, 2H), 2.42-2.34 (m, 4H), 1.75-1.64 (m, 4H);
LC-MS: [M+H]⁺ = 187.20.

### Production Example 29: Production of CPA-338

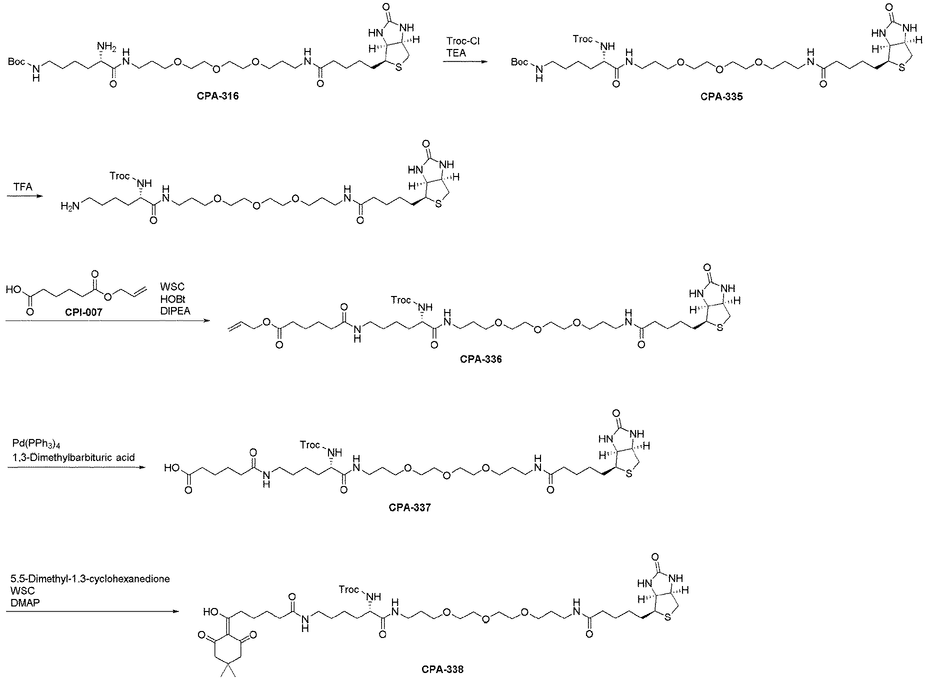

### (1) Production of CPA-335

TEA (43 µL) and 2,2,2-trichloroethyl chloroformate (33 µL) were added to a mixture of compound (CPA-310) shown in the above scheme (139 mg) and DCM (5 mL), and the mixture was stirred for 1 hour under ice-cooling. The solvent was distilled off, and the residue was purified by column chromatography (ODS, water/MeCN) to give the desired product (172 mg).
¹H NMR (400 MHz, DMSO-d₆) δ 7.89 (t, J = 5.6 Hz, 1H), 7.78-7.73 (m, 2H), 6.75 (t, J = 5.4 Hz, 1H), 6.42 (s, 1H), 6.35 (s, 1H), 4.82 (d, J = 12.4 Hz, 1H), 4.75 (d, J = 12.4 Hz, 1H), 4.30 (dd, J = 7.6, 5.2 Hz, 1H), 4.14-4.12 (m, 1H), 3.93-3.87 (m, 1H), 3.52-3.54 (m, 8H), 3.40-3.34 (m, 4H), 3.12-3.04 (m, 5H), 2.88-2.80 (m, 3H), 2.57 (d, J = 12.4 Hz, 1H), 2.04 (t, J = 7.4 Hz, 2H), 1.65-1.41 (m, 10H), 1.37-1.21 (m, 15H);
LC-MS: [M+H]⁺ = 849.56.

### (2) Production of CPA-336

TFA (468 µL) was added to a mixture of compound (CPA-335) produced above (172 mg) and DCM (3 mL), followed by stirring at room temperature for 1 hour. The solvent was distilled off. DMF (3 mL), CBI-741 (41 mg), WSC•HCl (47 mg), HOBt•H₂O (37 mg), and DIPEA (141 µL) were added to the residue, and the mixture was stirred at room temperature for 13 hours. The solvent was distilled off, and the residue was purified by column chromatography (ODS, water/MeCN) to give the desired product (134 mg).
¹H NMR (400 MHz, DMSO-d₆) δ 7.89 (t, J = 5.6 Hz, 1H), 7.80-7.73 (m, 3H), 6.42 (s, 1H), 6.35 (s, 1H), 5.96-5.86 (m, 1H), 5.28 (ddt, J = 17.2, 1.5, 1.5 Hz, 1H), 5.20 (ddt, J = 10.5, 1.5, 1.5 Hz, 1H), 4.82 (d, J = 12.4 Hz, 1H), 4.75 (d, J = 12.4 Hz, 1H), 4.53 (dt, J = 5.4, 1.5 Hz, 2H), 4.30 (dd, J = 7.6, 5.2 Hz, 1H), 4.14-4.11 (m, 1H), 3.93-3.87 (m, 1H), 3.52-3.44 (m, 8H), 3.39-3.30 (m, 4H), 3.15-3.04 (m, 5H), 3.01-2.96 (m, 2H), 2.82 (dd, J = 12.4, 5.1 Hz, 1H), 2.57 (d, J = 12.4 Hz, 1H), 2.35-2.31 (m, 2H), 2.06-2.02 (m, 4H), 1.64-1.21 (m, 20H);
LC-MS: [M+H]⁺ = 917.63.

### (3) Production of CPA-337

Pd(PPh₃)₄ (17 mg) and 1,3-dimethylbarbituric acid (47 mg) were added to a mixture of compound (CPA-336) produced above (134 mg) and DCM (3 mL), followed by stirring at room temperature for 1 hour. The solvent was distilled off, and the residue was purified by column chromatography (ODS, water/MeCN) to give the desired product (96 mg).
¹H NMR (400 MHz, DMSO-d₆) δ 7.91 (t, J = 5.5 Hz, 1H), 7.80-7.74 (m, 3H), 6.42 (s, 1H), 6.36 (s, 1H), 4.82 (d, J = 12.4 Hz, 1H), 4.75 (d, J = 12.4 Hz), 4.30 (dd, J = 7.6, 5.2 Hz, 1H), 4.14-4.11 (m, 1H), 3.93-3.87 (m, 1H), 3.52-3.35 (m, 12H), 3.12-3.04 (m, 5H), 3.01-2.96 (m, 2H), 2.82 (dd, J = 12.4, 5.1 Hz, 1H), 2.57 (d, J = 12.4 Hz, 1H), 2.18 (t, J = 6.8 Hz, 2H), 2.06-2.01 (m, 4H), 1.64-1.21 (m, 20H);
LC-MS: [M+H]⁺ = 877.54.

### (4) Production of CPA-338

Dimedone (31 mg), WSC•HCl (42 mg), and DMAP (27 mg) were added to a mixture of compound (CPA-337) produced above (96 mg) and DMF (2 mL), followed by stirring at 60°C for 2 hours. The solvent was distilled off, and the residue was purified by column chromatography (ODS, water/MeCN, containing 0.1% AcOH) to give the desired product (69 mg).
¹H NMR (400 MHz, DMSO-d₆) δ 12.12-11.83 (brs, 1H), 7.90 (t, J = 5.5 Hz, 1H), 7.78-7.74 (m, 3H), 6.42 (s, 1H), 6.35 (s, 1H), 4.82 (d, J = 12.4 Hz, 1H), 4.75 (d, J = 12.4 Hz, 1H), 4.30 (dd, J = 7.6, 5.2 Hz, 1H), 4.14-4.11 (m, 1H), 3.93-3.87 (m, 1H), 3.52-3.30 (m, 12H), 3.12-3.04 (m, 5H), 3.01-2.96 (m, 2H), 2.93-2.90 (m, 2H), 2.82 (dd, J = 12.4, 5.1 Hz, 1H), 2.59-2.43 (m, 5H), 2.04 (t, J = 7.3 Hz, 2H), 1.64-1.21 (m, 20H), 0.99 (s, 6H);
LC-MS: [M+H]⁺ = 999.69.

### Production Example 30: Production of CPI-009

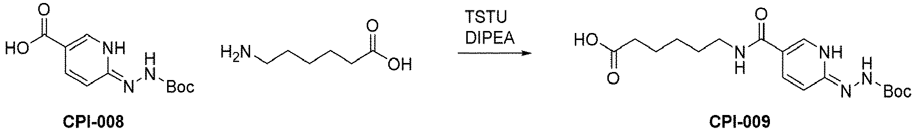

TSTU (105 mg) was added to a mixture of compound (CPI-008) shown in the above scheme (80 mg), DMF (3 mL), and DIPEA (166 µL), followed by stirring at room temperature for 5 minutes. 6-Aminohexanoic acid (41 mg) was added thereto, and the mixture was stirred at room temperature for 1 hour. The mixture was warmed to 50°C and stirred for 5 hours. The solvent was distilled off, and the residue was purified by column chromatography (ODS, water/MeCN) to give the desired product (102 mg).
¹H NMR (400 MHz, CD₃OD) δ 8.53 (d, J = 2.1 Hz, 1H), 8.37 (t, J = 5.4 Hz, 1H), 7.99 (dd, J = 8.8, 2.3 Hz, 1H), 6.70 (d, J = 8.8 Hz, 1H), 3.39-3.32 (m, 2H), 2.31 (t, J = 7.3 Hz, 2H), 1.70-1.58 (m, 4H), 1.53-1.33 (m, 11H);
LC-MS: [M+H]⁺ = 367.30.

### Production Example 31: Production of CPA-342

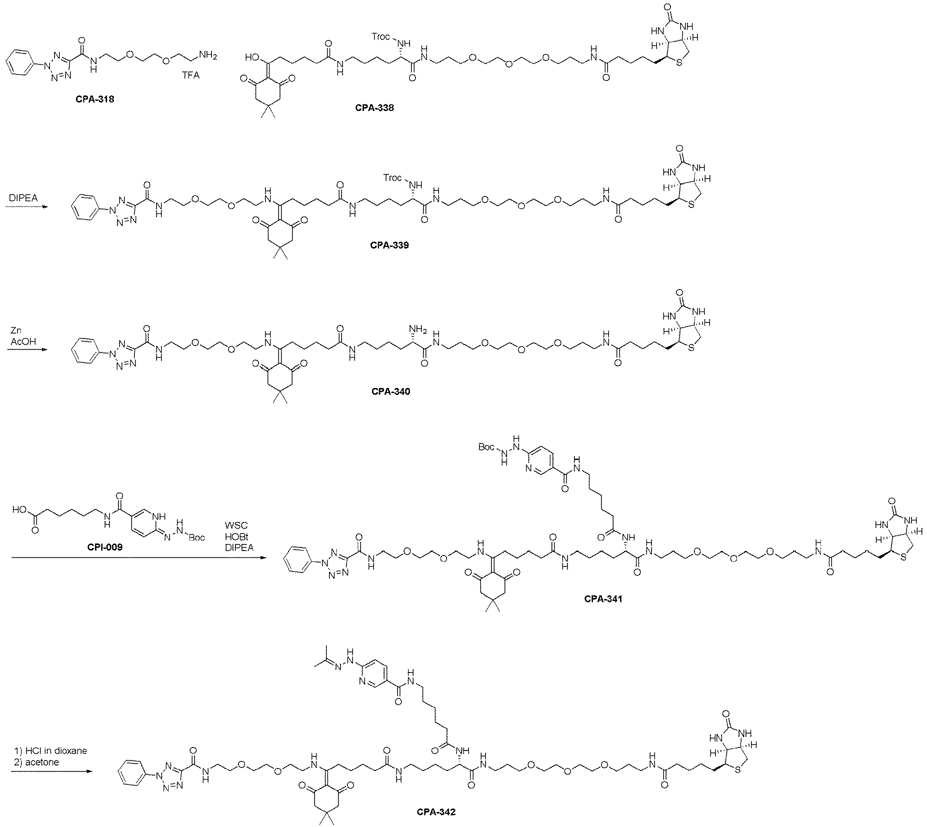

### (1) Production of CPA-339

DIPEA (25 µL) was added to a mixture of compound (CPA-338) shown in the above scheme (29 mg), compound (CPA-318) produced in Production Example 19 (13 mg), and DMF (1 mL), followed by stirring at 60°C for 1 hour. The solvent was distilled off, and the residue was purified by column chromatography (ODS, water/MeCN) to give the desired product (31 mg).
¹H NMR (400 MHz, DMSO-d₆) δ 13.36-13.34 (m, 1H), 9.06 (t, J = 5.8 Hz, 1H), 8.14-8.11 (m, 2H), 7.89 (t, J = 5.5 Hz, 1H), 7.78-7.63 (m, 6H), 6.42 (s, 1H), 6.35 (s, 1H), 4.82 (d, J = 12.3 Hz, 1H), 4.75 (d, J = 12.3 Hz, 1H), 4.32-4.29 (m, 1H), 4.14-4.11 (m, 1H), 3.93-3.87 (m, 1H), 3.63-3.60 (m, 10H), 3.52-3.30 (m, 14H), 3.11-3.04 (m, 5H), 3.00-2.96 (m, 2H), 2.93-2.90 (m, 2H), 2.82 (dd, J = 12.4, 5.1 Hz, 1H), 2.57 (d, J = 12.4 Hz, 1H), 2.23 (s, 4H), 2.09-2.02 (m, 4H), 1.63-1.21 (m, 20H), 0.91 (s, 6H);
LC-MS: [M+H]⁺ = 1301.83.

### (2) Production of CPA-340

Zinc (16 mg) and acetic acid (68 µL) were added to a mixture of compound (CPA-339) produced above (31 mg) and 1,4-dioxane (1 mL), followed by stirring at 60°C for 2 hours. The reaction mixture was purified by column chromatography (ODS, water/MeCN, containing 0.1% AcOH) to give the desired product (21 mg) .
¹H NMR (400 MHz, DMSO-d₆) δ 13.34 (t, J = 4.2 Hz, 1H), 9.09 (t, J = 5.7 Hz, 1H), 8.14-8.11 (m, 2H), 7.84 (t, J = 5.8 Hz, 1H), 7.77-7.63 (m, 5H), 6.42 (s, 1H), 6.36 (s, 1H), 4.32-4.29 (m, 1H), 4.14-4.11 (m, 1H), 3.63-3.04 (m, 32H), 3.01-2.96 (m, 2H), 2.94-2.90 (m, 2H), 2.82 (dd, J = 12.5, 5.1 Hz, 1H), 2.57 (d, J = 12.5 Hz, 1H), 2.24 (s, 4H), 2.09-2.02 (m, 4H), 1.65-1.21 (m, 20H), 0.91 (s, 6H); LC-MS: [M+H]⁺ = 1127.92.

### (3) Production of CPA-341

WSC•HCl (3.4 mg), HOBt•H₂O (2.7 mg), and DIPEA (6.2 µL) were added to a mixture of compound (CPA-340) produced above (10 mg), compound (CPI-009) produced in Production Example 28 (1) (4.7 mg), and DMF (1 mL), followed by stirring at room temperature for 12 hours. The solvent was distilled off, and the residue was purified by column chromatography (ODS, water/MeCN) to give the desired product (5.1 mg).
¹H NMR (400 MHz, DMSO-d₆) δ 13.34 (t, J = 4.7 Hz, 1H), 9.09 (t, J = 5.7 Hz, 1H), 8.91 (s, 1H), 8.63 (s, 1H), 8.52 (d, J = 1.6 Hz, 1H), 8.24 (t, J = 5.5 Hz, 1H), 8.13-8.10 (m, 2H), 7.93 (dd, J = 8.9, 1.7 Hz, 1H), 7.86-7.84 (m, 2H), 7.77-7.63 (m, 5H), 6.43 (s, 1H), 6.36 (s, 1H), 4.32-4.29 (m, 1H), 4.17-4.11 (m, 2H), 3.62-2.89 (m, 35H), 2.81 (dd, J = 12.5, 5.1 Hz, 1H), 2.57 (d, J = 12.5 Hz, 1H), 2.23 (s, 4H), 2.13-2.02 (m, 6H), 1.67-1.24 (m, 35H), 0.90 (s, 6H); LC-MS: [M+H]⁺ = 1476.01.

### (4) Production of CPA-342

4M HCl in 1,4-dioxane solution were added to a mixture of compound (CPA-341) produced above (4.7 mg) and MeOH (50 µL), followed by stirring at room temperature for 1 hour. MeOH (100 µL), acetone (100 µL), and DIPEA (70 µL) were added thereto, followed by stirring at room temperature for 1 hour. The reaction mixture was purified by column chromatography (ODS, water/MeCN) to give the desired product (2.9 mg).
¹H NMR (400 MHz, DMSO-d₆) δ 13.34 (t, J = 4.6 Hz, 1H), 9.62 (s, 1H), 9.08 (t, J = 5.7 Hz, 1H), 8.57 (d, J = 1.8 Hz, 1H), 8.26 (t, J = 5.6 Hz, 1H), 8.14-8.11 (m, 2H), 7.99 (dd, J = 8.8, 2.3 Hz, 1H), 7.86-7.84 (m, 2H), 7.7607.63 (m, 5H), 7.04 (d, J = 8.8 Hz, 1H), 6.43 (s, 1H), 6.36 (s, 1H), 4.31-4.28 (m, 1H), 4.17-4.10 (m, 2H), 3.62-3.59 (m, 10H), 3.50-2.89 (m, 25H), 2.81 (dd, J = 12.6, 5.1 Hz, 1H), 2.57 (d, J = 12.6 Hz, 1H), 2.23 (s, 4H), 2.14-2.02 (m, 6H), 1.96 (s, 3H), 1.92 (s, 3H), 1.63-1.15 (m, 26H), 0.90 (s, 6H); LC-MS: [M+H]⁺ = 1415.76.

### Production Example 32: Production of CPA-344

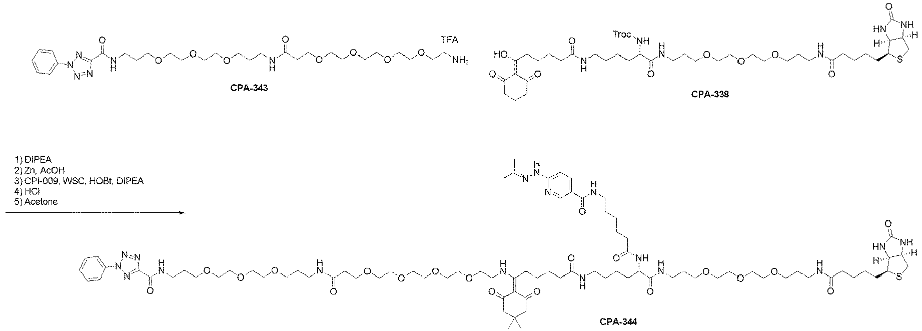

The desired product was produced in the same manner as in Production Example 31, except that CPA-343 was used in place of CPA-321.
¹H NMR (400 MHz, DMSO-d₆) δ 13.35 (s, 1H), 9.62 (s, 1H), 9.14 (t, J = 5.6 Hz, 1H), 8.57 (d, J = 1.9 Hz, 1H), 8.26 (t, J = 5.5 Hz, 1H), 8.14-8.12 (m, 2H), 7.99 (dd, J = 8.8, 2.4 Hz, 1H), 7.86-7.84 (m, 2H), 7.81-7.63 (m, 6H), 7.04 (d, J = 8.8 Hz, 1H), 6.43 (s, 1H), 6.36 (s, 1H), 4.32-4.28 (m, 1H), 4.17-4.10 (m, 2H), 3.60-2.91 (m, overlapped with water signal), 2.81 (dd, J = 12.4, 5.1 Hz, 1H), 2.61-2.45 (m, overlapped with DMSO signal), 2.29-2.26 (m, 6H), 2.14-2.02 (m, 8H), 1.96 (s, 3H), 1.93 (s, 3H), 1.80 (tt, J = 6.6, 6.6 Hz, 2H), 1.68-1.24 (m, 28H), 0.93 (s, 6H);
LC-MS: [M+H]⁺ = 1735.36.

### Production Example 33: Production of CPI-016

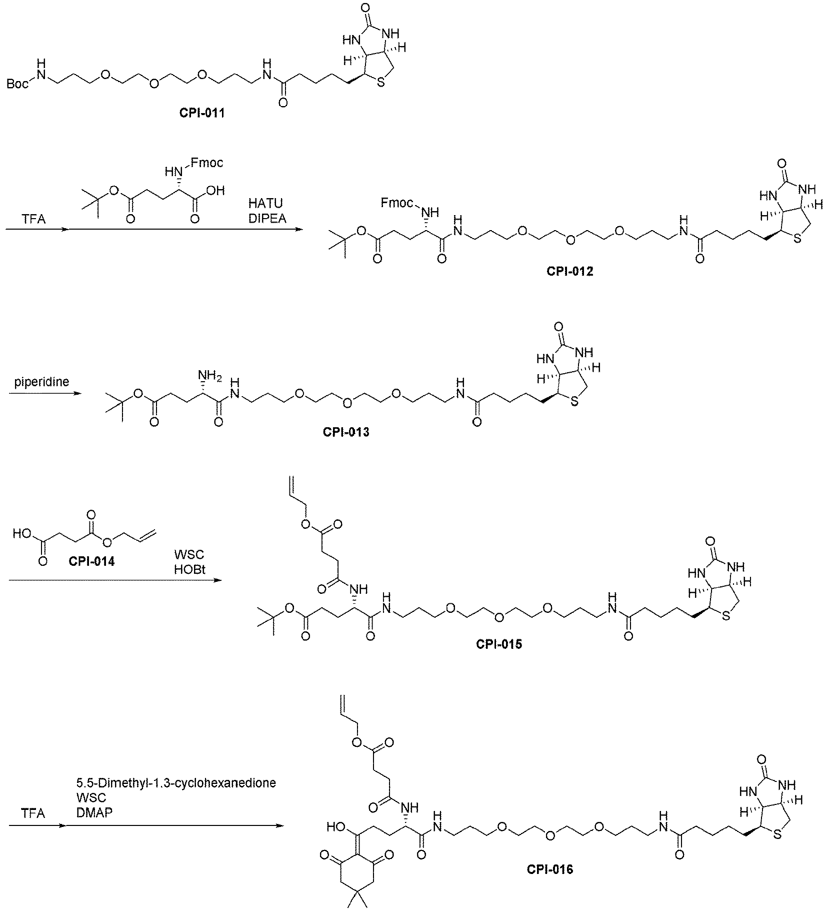

### (1) Production of CPI-012

TFA (683 µL) was added to a mixture of compound (CPI-011) shown in the above scheme (97 mg) and DCM (2 mL), followed by stirring at room temperature for 1 hour. The solvent was then distilled off. DMF (2 mL), N-[(9H-fluoren-9-ylmethoxy)carbonyl]-L-glutamic acid 5-tert-butyl ester hydrate (75 mg), HATU (81 mg), and DIPEA (155 µL) were added to the residue, and the mixture was stirred at room temperature for 1 hour. N-[(9H-fluoren-9-ylmethoxy)carbonyl]-L-glutamic acid 5-tert-butyl ester hydrate (75 mg) and HATU (68 mg) were further added thereto, and the mixture was stirred at room temperature for 1 hour. The solvent was distilled off, and the residue was purified by column chromatography (ODS, water/MeCN) to give the desired product (136 mg) .
¹H NMR (400 MHz, CDCl₃) δ 7.76 (d, J = 7.4 Hz, 2H), 7.66-7.60 (m, 2H), 7.40 (dd, J = 7.4, 7.4 Hz, 2H), 7.32 (ddd, J = 7.4, 7.4, 1.0 Hz, 2H), 7.28-7.25 (m, 1H), 6.68 (s, 1H), 6.55 (s, 1H), 6.13-6.05 (brm, 1H), 4.89-4.78 (brm, 1H), 4.50-4.47 (m, 1H), 4.38-4.17 (m, 5H), 3.65-3.51 (m, 12H), 3.43-3.28 (m, 4H), 3.16-3.11 (m, 1H), 2.89 (dd, J = 12.8, 4.9 Hz, 1H), 2.68 (d, J = 12.8 Hz, 1H), 2.42-2.28 (m, 2H), 2.19-2.05 (m, 3H), 1.98-1.89 (m, 1H), 1.81-1.57 (m, 8H), 1.49-1.39 (m, 11H);
LC-MS: [M+H]⁺ = 854.39.

### (2) Production of CPI-013

Piperidine (24 µL) was added to a mixture of compound (CPI-012) produced above (136 mg) and DMF (2 mL), followed by stirring at room temperature for 5 hours. The solvent was distilled off, and the residue was purified by column chromatography (ODS, water/MeCN) to give the desired product (87 mg).
¹H NMR (400 MHz, DMSO-d₆) δ 7.83 (t, J = 5.7 Hz, 1H), 7.75 (t, J = 5.6 Hz, 1H), 6.42 (s, 1H), 6.35 (s, 1H), 4.32-4.29 (m, 1H), 4.14-4.11 (m, 1H), 3.53-3.45 (m, 8H), 3.41-3.33 (m, 6H), 3.13-3.04 (m, 6H), 2.82 (dd, J = 12.5, 5.1 Hz, 1H), 2.57 (d, J = 12.5 Hz, 1H), 2.24-2.20 (m, 2H), 2.04 (t, J = 7.4 Hz, 2H), 1.80-1.71 (m, 1H), 1.66-1.43 (m, 9H), 1.39 (s, 9H), 1.35-1.23 (m, 2H);
LC-MS: [M+H]⁺ = 632.38.

### (3) Production of CPI-015

WSC•HCl (32 mg) and HOBt•H₂O (25 mg) were added to a mixture of compound (CPI-013) produced above (87 mg), compound (CPI-014) shown in the above scheme (22 mg), and DMF (2 mL), followed by stirring at room temperature for 5 hours. The solvent was distilled off, and the residue was purified by column chromatography (ODS, water/MeCN) to give the desired product (92 mg) .
¹H NMR (400 MHz, DMSO-d₆) δ 7.80-7.71 (brm, 1H), 7.22 (t, J = 5.6 Hz, 1H), 6.85 (t, J = 5.3 Hz, 1H), 6.61 (s, 1H), 5.95-5.85 (m, 1H), 5.31 (dd, J = 15.8, 1.4 Hz, 1H), 5.22 (dd, J = 9.2, 1.4 Hz, 1H), 5.01-4.79 (brs, 1H), 4.57 (d, J = 5.7 Hz, 2H), 4.53-4.50 (m, 1H), 4.39-4.34 (m, 2H), 3.70-3.49 (m, 12H), 3.43-3.34 (m, 3H), 3.28-3.14 (m, 2H), 2.92 (dd, J = 12.8, 4.8 Hz, 1H), 2.72 (d, J = 12.8 Hz, 1H), 2.69-2.65 (m, 2H), 2.61-2.57 (m, 2H), 2.39-2.26 (m, 2H), 2.15-2.03 (m, 3H), 1.98-1.36 (m, 20H);
LC-MS: [M+H]⁺ = 772.45.

### (4) Production of CPI-016

TFA (200 µL) was added to a mixture of compound (CPI-015) produced above (92 mg) and DCM (1 mL), followed by stirring at room temperature for 20 hours. The solvent was distilled off, and DMF (2 mL), dimedone (33 mg), WSC•HCl (46 mg), and DMAP (29 mg) were added to the residue, followed by stirring at 60°C for 4 hours. The solvent was distilled off, and the residue was purified by column chromatography (ODS, water/MeCN, containing 0.1% AcOH) to give the desired product (45 mg).
¹H NMR (400 MHz, DMSO-d₆) δ 8.06 (d, J = 8.0 Hz, 1H), 7.84 (t, J = 5.4 Hz, 1H), 7.74 (t, J = 5.6 Hz, 1H), 6.42 (s, 1H), 6.35 (s, 1H), 5.94-5.84 (m, 1H), 5.29 (ddt, J = 17.2, 1.5, 1.5 Hz, 1H), 5.19 (ddt, J = 10.5, 1.5, 1.5 Hz, 2H), 4.52 (dt, J = 5.3, 1.5 Hz, 2H), 4.32-4.29 (m, 1H), 4.22-4.16 (m, 1H), 4.14-4.11 (m, 1H), 3.52-2.45 (m, 8H), 3.40-3.30 (m, 4H), 3.14-3.02 (m, 5H), 3.01-2.91 (brs, 2H), 2.82 (dd, J = 12.4, 5.1 Hz, 1H), 2.59-2.41 (m, 9H), 2.04 (t, J = 7.4 Hz, 2H), 1.95-1.87 (m, 1H), 1.80-1.71 (m, 1H), 1.65-1.57 (m, 5H), 1.54-1.41 (m, 3H), 1.35-1.24 (m, 2H), 0.99 (s, 6H);
LC-MS: [M+H]⁺ = 838.42.

### Production Example 34: Production of CPA-346

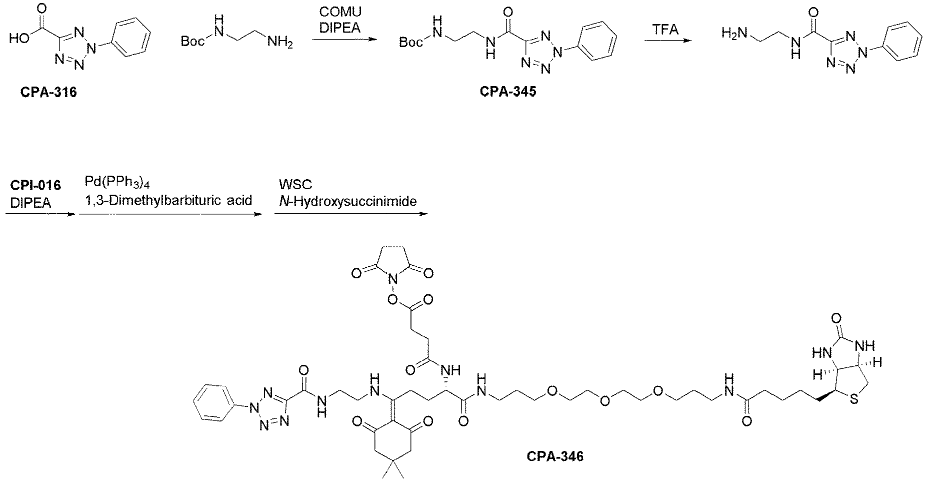

### (1) Production of CPA-345

N-Boc-1,2-diaminoethane (7.3 µL), COMU (22 mg), and DIPEA (24 µL) were added to a mixture of compound (CPA-316) shown in the above scheme (8.8 mg) and DMF (1 mL), followed by stirring at room temperature for 5 hours. The solvent was distilled off, and the residue was purified by column chromatography (ODS, water/MeCN) to give the desired product (8.3 mg).

¹H NMR (400 MHz, CDCl₃) δ 8.22-8.19 (m, 2H), 7.81-7.70 (brs, 1H), 7.61-7.52 (m, 3H), 4.99-4.87 (brs, 1H), 3.68 (dt, J = 5.7, 5.7 Hz, 2H), 3.45 (dt, J = 5.7, 5.7 Hz, 2H), 1.44 (s, 9H);
LC-MS: [M+H]⁺ = 333.16.

### (2) Production of CPA-346

The desired product was produced in a manner similar to that in Production Example 20, using CPA-345 and CPI-016.
¹H NMR (400 MHz, DMSO-d₆) δ 13.31 (t, J = 5.9 Hz, 1H), 9.43 (t, J = 5.6 Hz, 1H), 8.15-8.12 (m, 2H), 7.85 (t, J = 5.6 Hz, 1H), 7.75-7.65 (m, 4H), 6.41 (s, 1H), 6.35 (s, 1H), 4.48 (dd, J = 10.8, 3.5 Hz, 1H), 4.32-4.28 (m, 1H), 4.14-4.10 (m, 1H), 3.70-3.67 (m, 2H), 3.58-3.29 (m, 14H), 3.12-2.89 (m, 7H), 2.84-2.66 (m, 5H), 2.59-2.49 (m, 5H), 2.26-2.19 (m, 5H), 2.04 (t, J = 7.4 Hz, 2H), 1.65-1.41 (m, 8H), 1.35-1.24 (m, 2H), 0.91 (s, 6H);
LC-MS: [M+H]⁺ = 1109.51.

### Production Example 35: Production of CPA-347

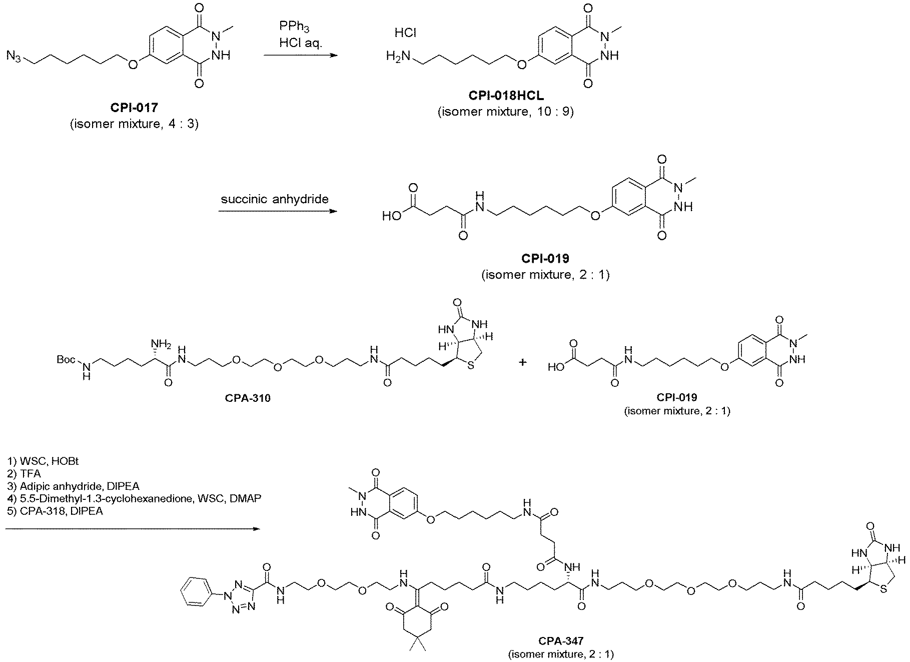

### (1) Production of CPI-018HCL

Triphenylphosphine (78 mg) and H₂O (0.40 mL) were added to a mixture of compound (CPI-017, isomer ratio of 4:3) shown in the above scheme (63 mg) and THF (2 mL), followed by stirring at room temperature overnight. A 1 M aqueous HCl solution (0.10 mL) was added thereto, followed by stirring at room temperature for 1 hour. A 1M aqueous HCl solution (0.30 mL) was further added thereto, followed by stirring at room temperature for 1 hour. The mixture was warmed to 60°C and stirred for 6 hours, followed by stirring at room temperature for 4 days. The solvent was distilled off, and the residue was purified by column chromatography (ODS, water/MeCN) to give the desired product (39 mg, isomer ratio of 10:9).
¹H NMR (400 MHz, CD₃OD) δ 8.20 (dd, J = 8.4, 0.8 Hz, 1H), 7.99 (d, J = 8.8 Hz, 0.9H), 7.69 (d, J = 2.6 Hz, 0.9H), 7.45-7.37 (m, 2 + 0.9H), 4.21-4.15 (m, 2 + 0.9 × 2H), 3.67 (s, 0.9 × 3H), 3.65 (s, 3H), 2.95 (t, J = 7.6 Hz, 2 + 0.9 × 2H), 1.94-1.85 (m, 2 + 0.9 × 2H), 1.76-1.66 (m, 2 + 0.9 × 2H), 1.65-1.45 (m, 4 + 0.9 × 4H); LC-MS: [M+H]⁺ = 292.23.

### (2) Production of CPI-019

Succinic anhydride (13 mg) was added to a mixture of compound (CPI-018HCL) produced above (39 mg), DMF (2 mL), and DIPEA (93 µL), followed by stirring at room temperature overnight. The solvent was distilled off, and the residue was purified by column chromatography (ODS, water/MeCN) to give the desired product (32 mg, isomer ratio of 2:1).
¹H NMR (400 MHz, CD₃OD) δ 8.19 (dd, J = 7.6, 2.0 Hz, 1H), 7.98 (d, J = 8.8 Hz, 0.5H), 7.69 (d, J = 2.6 Hz, 0.5H), 7.45-7.37 (m, 2 + 0.5H), 4.19-4.12 (m, 2 + 0.5 × 2H), 3.66 (s, 0.5 × 3H), 3.64 (s, 3H), 3.19 (t, J = 6.9 Hz, 2 + 0.5 × 2H), 2.60-2.55 (m, 2 + 0.5 × 2H), 2.45 (t, J = 6.8 Hz, 2 + 0.5 × 2H), 1.91-1.82 (m, 2 + 0.5 × 2H), 1.60-1.50 (m, 4 + 0.5 × 4H), 1.48-1.39 (m, 2 + 0.5 × 2H); LC-MS: [M+H]⁺ = 392.33.

### (3) Production of CPA-347

The desired product was produced in a manner similar to those in Production Example 18 and Production Example 20 (isomer ratio of 2:1).
¹H NMR (400 MHz, DMSO-d₆) δ 13.34 (t, J = 4.9 Hz, 1H), 9.10 (t, J = 5.5 Hz, 1H), 8.13-9.10 (m, 2.3H), 8.00 (d, J = 7.9 Hz, 1H), 7.89-7.63 (m, 8.3H), 7.52 (s, 0.7H), 7.36 (s, 0.7H), 6.43 (s, 1H), 6.36 (s, 1H), 4.31-4.28 (m, 1H), 4.13-4.06 (m, 4H), 3.62-2.89 (m, 36H), 2.81 (dd, J = 12.5, 5.2 Hz, 1H), 2.60-2.49 (m, 3H), 2.37-2.28 (m, 4H), 2.23 (s, 4H), 2.09-2.02 (m, 4H), 1.78-1.71 (m, 2H), 1.65-1.23 (m, 26H), 0.90 (s, 6H);
LC-MS: [M+H]⁺ = 1500.96.

### Production Example 36: Production of CPA-353

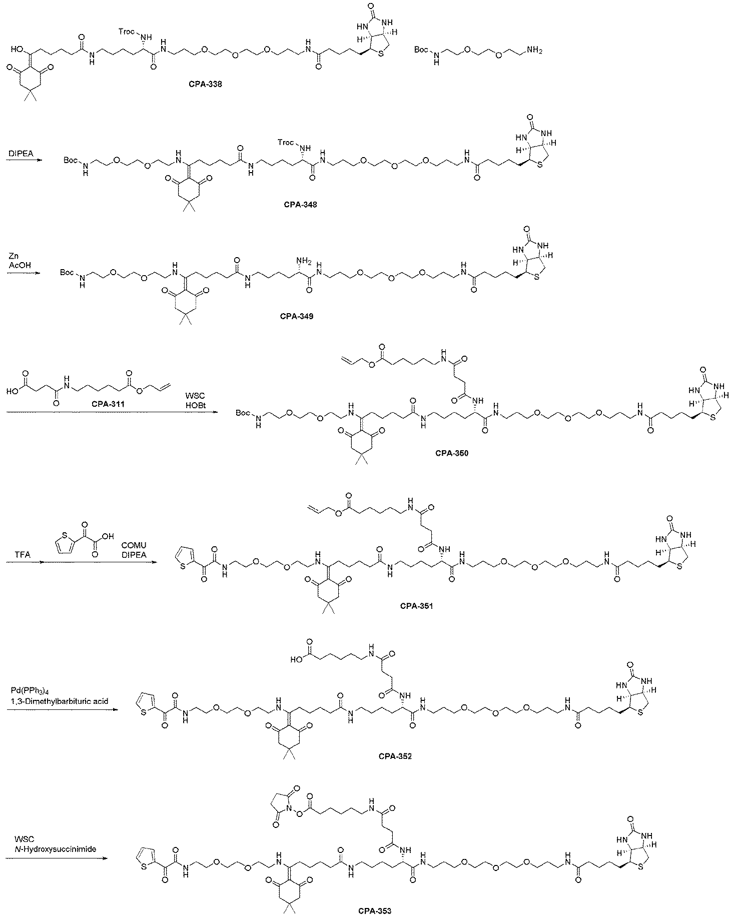

### (1) Production of CPA-348

A mixture of compound (CPA-338) shown in the above scheme (11 mg), N-Boc-2,2'-(ethylenedioxy)diethylamine (2.7 mg), and DMF (1 mL) was stirred at 60°C for 2 hours. The solvent was distilled off, and the residue was purified by column chromatography (ODS, water/MeCN) to give the desired product (10 mg).
¹H NMR (400 MHz, DMSO-d₆) δ 13.35 (s, 1H), 7.89 (t, J = 5.6 Hz, 1H), 7.78-7.74 (m, 3H), 6.77 (t, J = 5.3 Hz, 1H), 6.42 (s, 1H), 6.35 (s, 1H), 4.82 (d, J = 12.4 Hz, 1H), 4.75 (d, J = 12.4 Hz, 1H), 4.32-4.29 (m, 1H), 4.14-4.11 (m, 1H), 3.93-3.87 (m, 1H), 3.61-2.92 (m, 33H), 2.82 (dd, J = 12.4, 5.1 Hz, 1H), 2.59-2.49 (m, 1H), 2.27 (s, 4H), 2.10-2.02 (m, 4H), 1.73-1.21 (m, 29H), 0.94 (s, 6H);
LC-MS: [M+H]⁺ = 1229.65.

### (2) Production of CPA-349

Zinc (11 mg) and acetic acid (47 µL) were added to a mixture of compound (CPA-348) produced above (10 mg) and 1,4-dioxane (1 mL), followed by stirring at 60°C for 30 minutes. Zinc (11 mg) was further added thereto, followed by stirring at 60°C for 30 minutes. The solvent was distilled off, and the residue was purified by column chromatography (ODS, water/MeCN, containing 0.1% AcOH) to give the desired product (8.6 mg).
¹H NMR (400 MHz, DMSO-d₆) δ 13.35 (s, 1H), 7.98-7.92 (brs, 1H), 7.75 (t, J = 5.5 Hz, 2H), 6.77 (t, J = 5.4 Hz, 1H), 6.42 (s, 1H), 6.35 (s, 1H), 6.32-6.29 (m, 1H), 4.14-4.11 (m, 1H), 3.61-3.28 (m, 27H), 3.14-2.92 (m, 9H), 2.82 (dd, J = 12.4, 5.1 Hz, 1H), 2.59-2.49 (m, 1H), 2.27 (s, 4H), 2.10-2.02 (m, 4H), 1.64-1.23 (m, 29H), 0.94 (s, 6H) ;
LC-MS: [M+H]⁺ = 1055.66.

### (3) Production of CPA-350

WSC•HCl (3.1 mg) and HOBt•H₂O (2.5 mg) were added to a mixture of compound (CPA-349) produced above (8.6 mg), CPA-311 (2.2 mg), and DMF (1 mL), followed by stirring at room temperature for 1 hour. The solvent was distilled off, and the residue was purified by column chromatography (ODS, water/MeCN) to give the desired product (2.3 mg).
¹H NMR (400 MHz, DMSO-d₆) δ 13.34 (s, 1H), 7.96 (d, J = 7.9 Hz, 1H), 7.87-7.82 (m, 2H), 7.75-7.73 (m, 2H), 6.80-6.74 (brs, 1H), 6.42 (s, 1H), 6.35 (s, 1H), 5.96-5.86 (m, 1H), 5.32-5.26 (m, 1H), 5.22-5.19 (m, 1H), 4.54-4.53 (m, 2H), 4.32-4.28 (m, 1H), 4.14-4.04 (m, 2H), 3.61-3.16 (m, overlapped with water signal), 3.12-2.89 (m, 11H), 2.82 (dd, J = 12.4, 5.1 Hz, 1H), 2.61-2.49 (m, overlapped with DMSO signal), 2.33-2.22 (m, 10H), 2.10-2.02 (m, 4H), 1.85 (s, 2H), 1.65-1.21 (m, 33H), 0.94 (s, 6H);
LC-MS: [M+H]⁺ = 1309.01.

### (4) Production of CPA-351

TFA (130 µL) was added to a mixture of compound (CPA-350) produced above (2.2 mg) and DCM (0.50 mL), followed by stirring at room temperature for 1 hour. The solvent was distilled off, and DMF (0.50 mL), 2-thiopheneglyoxylic acid (1.3 mg), COMU (3.6 mg), and DIPEA (15 µL) were added to the residue, followed by stirring at room temperature for 1 hour. The solvent was distilled off, and the residue was purified by column chromatography (ODS, water/MeCN) to give the desired product (1.4 mg).
¹H NMR (400 MHz, DMSO-d₆) δ 13.36-13.33 (m, 1H), 8.88 (t, J = 5.5 Hz, 1H), 8.19-8.16 (m, 2H), 7.97 (d, J = 8.3 Hz, 1H), 7.87-7.83 (m, 2H), 7.77-7.73 (m, 2H), 7.29 (dd, J = 4.8, 4.0 Hz, 1H), 6.42 (s, 1H), 6.35 (s, 1H), 5.96-5.86 (m, 1H), 5.31-5.26 (m, 1H), 5.22-5.19 (m, 1H), 4.53 (dd, J = 5.4, 1.2 Hz, 2H), 4.32-4.28 (m, 1H), 4.14-4.05 (m, 2H), 3.61-2.91 (m, overlapped with water signal), 2.82 (dd, J = 12.4, 5.1 Hz, 1H), 2.61-2.49 (m, overlapped with DMSO signal), 2.34-2.25 (m, 10H), 2.09-2.02 (m, 4H), 1.63-1.19 (m, 26H), 0.92 (s, 6H);
LC-MS: [M+H]⁺ = 1346.89.

### (5) Production of CPA-352

Pd(PPh₃)₄ (0.1 mg) and 1,3-dimethylbarbituric acid (0.3 mg) were added to a mixture of compound (CPA-351) produced above (1.4 mg) and DCM (0.50 mL), followed by stirring at room temperature for 1 hour. The solvent was distilled off, and the residue was purified by column chromatography (ODS, water/MeCN) to give the desired product (0.7 mg).
¹H NMR (400 MHz, DMSO-d₆) δ 13.35-13.33 (m, 1H), 8.94-8.91 (m, 1H), 8.19-8.16 (m, 2H), 8.07-7.99 (m, 1H), 7.94-7.91 (m, 2H), 7.29 (dd, J = 4.8, 3.9 Hz, 1H), 6.47 (s, 1H), 6.37 (s, 1H), 4.31-4.28 (m, 1H), 4.14-4.04 (m, 2H), 3.62-2.94 (m, overlapped with water signal), 2.82 (dd, J = 12.4, 5.1 Hz, 1H), 2.61-2.49 (m, overlapped with DMSO signal), 2.34-2.25 (m, 10H), 2.11-2.03 (m, 4H), 1.94-1.88 (brm, 2H), 1.62-1.11 (m, 24H), 0.92 (s, 6H);
LC-MS: [M+H]⁺ = 1306.84.

### (6) Production of CPA-353

N-Hydroxysuccinimide (0.6 mg) and WSC•HCl (1.0 mg) were added to a mixture of compound (CPA-352) produced above (0.7 mg) and DMF (0.50 mL), followed by stirring at room temperature for 16 hours. N-Hydroxysuccinimide (0.6 mg) and WSC•HCl (1.0 mg) were further added thereto, followed by stirring at room temperature for 1 hour. The solvent was distilled off, and the residue was purified by column chromatography (ODS, water/MeCN) to give the desired product (0.7 mg).
¹H NMR (400 MHz, DMSO-d₆) δ 13.37-13.33 (m, 1H), 8.94-8.87 (m, 1H), 8.19-8.16 (m, 2H), 7.98-7.96 (m, 1H), 7.87-7.83 (m, 2H), 7.76-7.73 (m, 2H), 7.29 (dd, J = 4.9, 3.9 Hz, 1H), 6.42 (s, 1H), 6.36 (s, 1H), 4.31-4.28 (m, 1H), 4.14-4.04 (m, 2H), 3.62-2.79 (m, overlapped with water signal), 2.61-2.25 (m, overlapped with DMSO signal), 2.09-2.02 (m, 4H), 1.63-1.15 (m, 26H), 0.92 (s, 6H);
LC-MS: [M+2H]²⁺ = 1404.71.

### Production Example 37: Synthesis of CPI-018

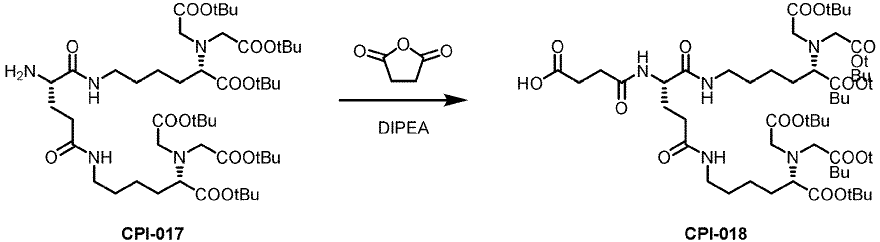

### Synthesis of CPI-018

Succinic anhydride (2.4 mg) was added to a mixture of compound (CPI-017) shown in the above scheme (20 mg), DMF (0.5 mL), and DIPEA (14 µL), followed by stirring at room temperature overnight. AcOEt was added to the reaction solution, and the solution was washed with water and saturated saline and dried over Na₂SO₄. The solvent was distilled off, and the residue was purified by column chromatography (ODS, water/MeCN) to give the desired product (14 mg) .
¹H NMR (500 MHz, CD₃OD) δ 4.30-4.23 (m, 1H), 3.53-3.38 (m, 8H), 3.31-3.25 (m, 2H), 3.20-3.13 (m, 4H), 2.54-2.40 (m, 4H), 2.30-2.24 (m, 2H), 2.20-2.10 (m, 1H), 1.89-1.80 (m, 1H), 1.69-1.27 (m, 66H); LC-MS: [M+H]⁺ = 1073.20.

### Production Example 38: Production of CPA-354

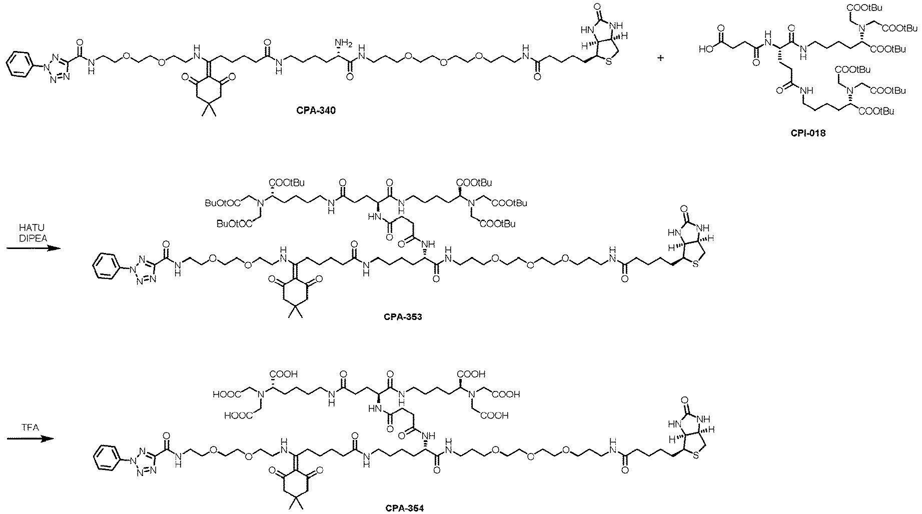

### Synthesis of CPA-353

HATU (2.3 mg) and DIPEA (2.6 µL) were added to a mixture of compound (CPI-018) produced above (5.3 mg), compound (CPA-340) produced in Production Example 30 (2) (5.6 mg), and DMF (0.5 mL), followed by stirring at room temperature for 1 hour. The solvent was distilled off, and the residue was purified by column chromatography (ODS, water/MeCN) to give the desired product (5.8 mg) .
¹H NMR (400 MHz, CD₃OD) δ 8.20-8.15 (m, 2H), 7.69-7.59 (m, 3H), 4.52-4.47 (m, 1H), 4.33-4.19 (m, 3H), 3.78-3.38 (m, 34H), 3.29-3.10 (m, 11H), 3.00-2.89 (m, 3H), 2.70 (d, J = 12.7 Hz, 1H), 2.64-2.48 (m, 4H), 2.33-2.05 (m, 11H), 1.93-1.27 (m, 87H), 0.97 (s, 6H).

### Synthesis of CPA-354

TFA (0.5 mL) was added to a mixture of compound (CPA-353) produced above (5.8 mg) and DCM (1 mL), followed by stirring at room temperature for 9 hours. The solvent was distilled off to give the desired product (4.5 mg).
¹H NMR (400 MHz, CD₃OD) δ 8.19-8.15 (m, 2H), 7.69-7.59 (m, 3H), 4.52-4.46 (m, 1H), 4.34-4.20 (m, 3H), 3.80-3.46 (m, 34H), 3.29-3.10 (m, 11H), 3.00-2.89 (m, 3H), 2.74-2.68 (m, 1H), 2.67-2.48 (m, 4H), 2.33-2.09 (m, 11H), 1.93-1.26 (m, 33H), 0.97 (s, 6H);
LC-MS: [M+H]²⁺ = 923.48.

### Production Example 39: Production of Labeled Antibody (CPA-321-Labeled Antibody)

Anti-CD71 antibody OKT9 (#16-0719-85, Thermo Fisher Scientific), or a mouse IgG1 isotype control antibody (#553447, BD Biosciences) as a control antibody was placed in a 20-µL tube and diluted with NHS labeling buffer (25 mM HEPES-NaOH pH 8.2, 150 mM NaCl) to a final concentration of about 0.1 mg/ml. About 1.5 µL of a solution of compound (CPA-321) produced in Production Example 20 in DMSO (10 mg/ml in DMSO) was added thereto, and the mixture was reacted at room temperature for 2 hours. Subsequently, about 5 µL of a lysine solution (100 mM) was added thereto, and the mixture was reacted at room temperature for 20 minutes. The resulting mixture was desalted by applying it to a desalting column (Zeba Spin, Thermo Scientific, 89882), and a solution containing a CPA-321-labeled antibody (CPA-321-labeled antibody solution) was collected.

### Test Examples

The action of the compound of the present invention is described below by showing test results using representative compounds among the tetra-functional compounds and tetra-functional chemical probes of the present invention. However, the present invention is not limited to these Test Examples.

### Test Example 1: Isolation and Detection of Target Molecule from Living Cell Using Compound (CPP-127)

### 1. Cell Culture and Method for Treating Cultured Cell with This Compound

CHO-K1 cells stably expressing human dopamine receptor D2 (DRD2) were cultured on a 10-cm plate until they became confluent.

The cells were washed with a medium, and the medium was replaced with a medium containing DMSO or an unmodified ligand, followed by a reaction for 30 minutes. Subsequently, the medium was discarded and replaced with a medium containing compound (CPP-127) synthesized in Production Example 12, and the cells were allowed to stand at 37°C for 30 minutes. The medium was discarded, and the cells were washed with PBS. PBS (1 ml) was added to the cells on the plate, and then the cells were irradiated with 302 nm UV light for 1 minute using a UV crosslinker. The cells were collected with a cell scraper after UV irradiation, and the collected cell pellets were cryopreserved at -80°C.

### 2. Preparation And Purification of Membrane Fraction Protein

The cryopreserved cells were suspended in 50 mM Tris Homogenate Buffer (pH 7.4, 2.5 mM EDTA, 5 mM MgCl₂, 320 mM sucrose) and homogenized using a sonicator. The suspension after homogenization was centrifuged with a centrifuge (1,000 g, 15 minutes, 4°C). The supernatant obtained after centrifugation was transferred to another tube and centrifuged (30,000×g, 30 minutes, 4°C). After centrifugation, the supernatant was discarded, and 100 mM sodium carbonate buffer (Na₂CO₃) was added to the precipitate, followed by homogenization with a sonicator and incubation on ice for 10 minutes. The suspension was centrifuged by ultracentrifugation (45,2000×g, 30 minutes, 4°C), the supernatant was discarded, and the resulting precipitate was rinsed with Milli-Q water and used as membrane fraction proteins.

### 3. Membrane Protein Elution

Membrane lysis buffer (50 mM Tris-HCl (pH 7.5), 150 mM NaCl, 0.1% SDS, 0.5% SDC, 1% Triton-X-100, 8M Urea) was added to the membrane fraction proteins prepared by the above method, and the membrane fraction proteins were dissolved with a sonicator. Streptavidin beads (#88816, Thermo Fisher Scientific) were added to the solubilized membrane protein solution, and the biotin-labeled proteins were adsorbed onto the beads by stirring and mixing for 60 minutes. After the beads were washed with membrane lysis buffer, 40 µL of a 2% N₂H₄ solution containing 0.05% SDS was added, and the beads were allowed to stand at room temperature for 30 minutes. The beads were removed by magnetic separation, and the solution was collected. The collected solution was neutralized by adding a 20% TFA solution, and then concentrated with a centrifugal evaporator. Dithiothreitol and iodoacetamide were added to the concentrated solution for reduction and alkylation, and a trypsin solution was added, followed by digestion at 37°C overnight.

### 4. LC-MS Analysis and Identification of Target Protein

The obtained tryptic digest was desalted and purified, and protein analysis was performed by LC-MS. LC-MS was performed using an LC-MS system that uses a combination of a nano-HPLC apparatus (Ultimate 3000, Thermo Scientific) and a mass spectrometer (Q Exactive, Thermo Scientific). In the nano-HPLC apparatus, gradient analysis was performed by reverse-phase chromatography using a column (stationary phase) composed of a trap column (C18 Acclaim PepMap, Thermo Scientific) and a chip column (75 µm i.d., 12 cm long, 3 µm C18 particles, Nikkyo Technos, Co., Ltd.), and a 0.1% formic acid-acetonitrile system as a mobile phase. The flow rate was 300 nl/min, and peptides were eluted with a linear gradient in which the acetonitrile concentration was increased from 4% to 35% over a period of 48 minutes. Mass spectrometry was performed by nanoelectrospray ionization in positive ion mode. A full scan MS spectrum was acquired in the m/z range of 380 to 1600, and a product ion spectrum was measured in data dependent acquisition mode. The data obtained by LC-MS were analyzed by Proteome Discoverer 2.2 analysis software (PD 2.2, Thermo Fisher Scientific). For protein identification, the search engine Sequest provided in PD 2.2 was used to conduct a search across the database downloaded from UniProt. Calculations using the false discovery rate were performed. After filtering was performed so that the false-positive protein identification rate was less than 1%, those with a PSM value of 5 or more were regarded as identified proteins in order to select more reliable candidate proteins. Further, the data were narrowed down using the number of unique peptides identified. In the quantitative analysis, the proteins were quantified by LFQ, which is a quantification method provided in PD 2.2, and quantitative comparison analysis with the group treated with the unmodified ligand was performed to distinguish the specifically collected proteins. The analysis was performed in two independent tests, and the results can be expressed as a ratio relative to those in the group treated with the unmodified ligand.

### 5. Results

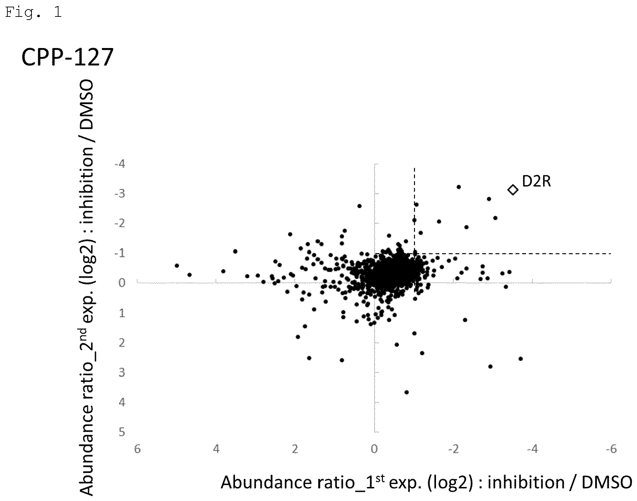

Fig. 1 shows the results. As shown in Fig. 1, when the tetra-functional chemical probe (compound (CPP-127)) of the present invention was used, detection of dopamine receptor D2 was significantly suppressed in the group treated with the unmodified ligand, confirming that dopamine receptor D2 (DRD2) was specifically collected.

### Test Example 2: Isolation and Detection of Target Molecule from Living Tissue Using Compounds (CPF-224, CPF-202, and CPF-242)

A DBA/2CrSlc mouse was anesthetized by isoflurane inhalation and then decapitated, and the brain was quickly removed. The removed brain was immersed in artificial cerebrospinal fluid (ACSF) at 0°C for 60 seconds while being continuously bubbled with a gas mixture (95% O₂, 5% CO₂). The olfactory bulb and cerebellum were removed with a scalpel, and the brain was fixed to the stage of a vibrating blade microtome (Leica, VT1200) using instant glue. Sliced sections (thin sections having a thickness of 0.3 mm) of the brain containing the striatum and hippocampus were prepared in ACSF at 0°C continuously bubbled with the gas mixture. The sliced sections were placed in a dish containing artificial cerebrospinal fluid. An unmodified ligand or DMSO was added, and the dish was allowed to stand at room temperature for 5 minutes. Subsequently, compound (CPF-224) produced in Production Example 10 or compound (CPF-202) produced in Production Example 8 was added, and the dish was allowed to stand at room temperature for 30 minutes. The solution was removed, and the sliced sections were irradiated with UV light (in the cases of CPF-224 and CPF-202, 302 nm UV light for 1 minute; in the case of CPF242, 365 nm UV light for 15 minutes). The sliced sections were washed and collected with PBS, and the supernatant was removed by centrifugation (3000×g, 4°C, 5 minutes). The obtained pellets were analyzed by the same method as in Test Example 1. The analysis was performed in two independent tests, and the results can be expressed as a ratio relative to those in the group treated with the unmodified ligand.

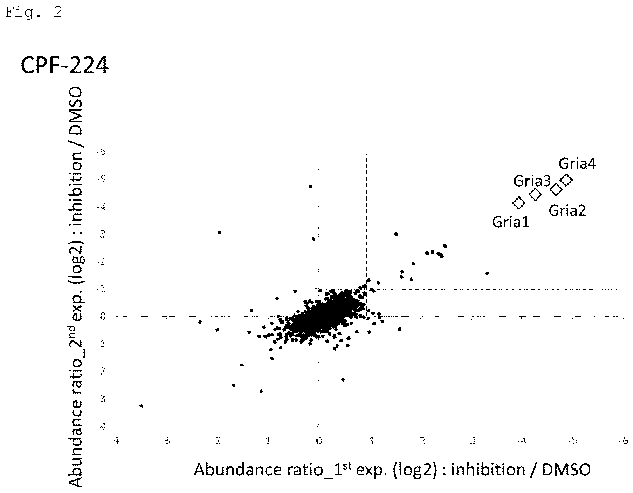

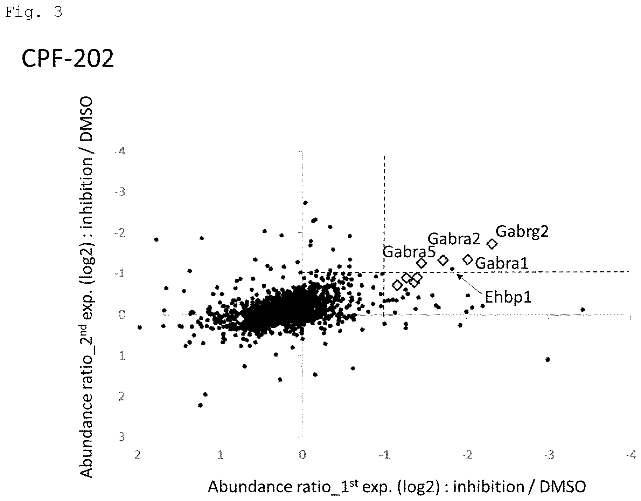

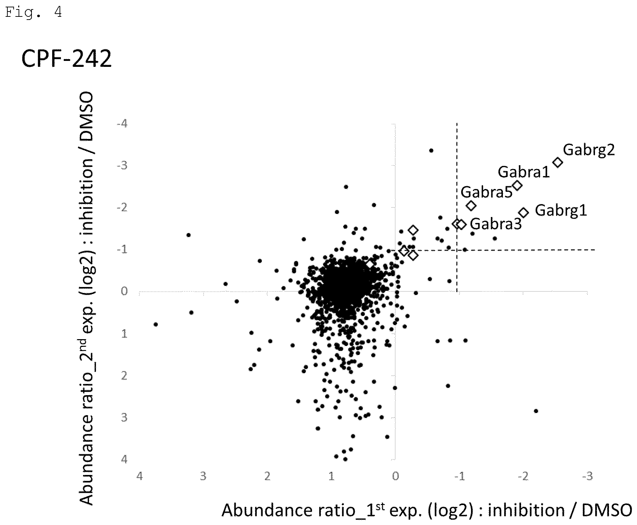

Fig. 2 shows the results obtained using compound (CPF-224). Fig. 3 shows the results obtained using compound (CPF-202). Fig. 4 shows the results obtained using compound (CPF-242). As shown in these figures, when the tetra-functional chemical probes of the present invention, i.e., CPF-224, CPF-202, and CPF242, were used, detection of AMPA-type glutamate receptors (Gria1, Gria2, Gria3, Gria4) and GABA receptor subunits (Gabra1, Gabra2, Gabra5, Gabrg2) contained in brain tissue were significantly suppressed in the group treated with the unmodified ligand, confirming that the membrane proteins were specifically collected.

### Test Example 3: Capture of Cell Surface Antigen Using Labeled Antibody

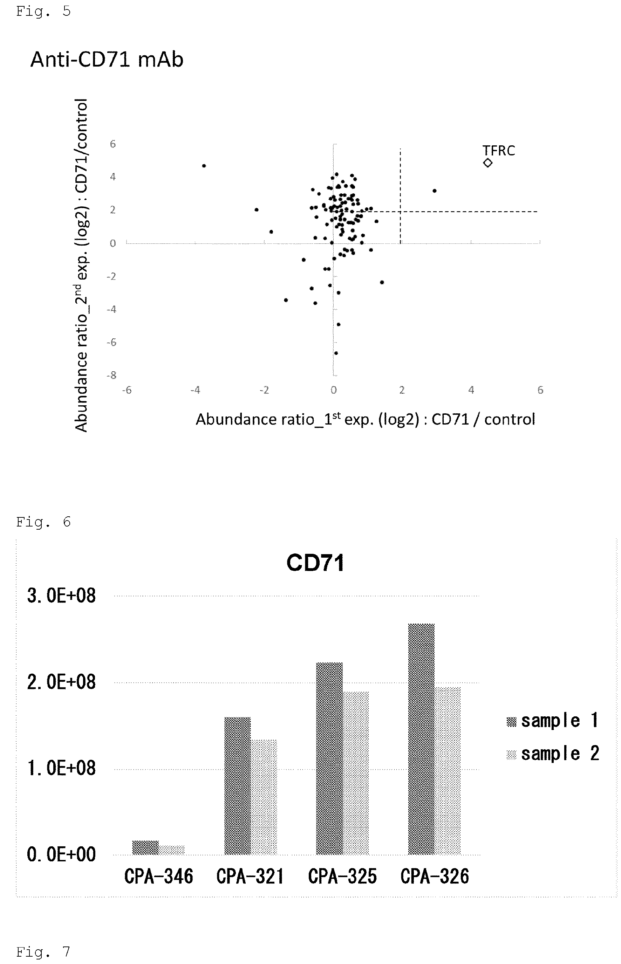

Antigen molecules were obtained from THP-1 cells using an anti-CD71 antibody labeled with CPA-321 produced in Production Example 21 or anti-mouse IgG1 control antibody labeled with CPA-321 produced in Production Example 21. Specifically, THP-1 cells were collected and washed with SB buffer (PBS buffer containing 2% FCS). The cells after washing were suspended in SB buffer containing a 0.1% Fc block reagent (Invitrogen, 16-9161-73) and reacted at 4°C for 15 minutes. The supernatant was removed by centrifugation (600×g, 3 minutes, 4°C), and an antibody solution (obtained by adding SB buffer to a solution (20 µg) of an antibody labeled with CPA-321 produced in Production Example 21 to make a total volume of 1 mL) was added, followed by a reaction at 4°C for 20 minutes. The antibody solution was removed by centrifugation, and the cells were washed with SB buffer and then suspended in 0.5 ml of SB buffer. The cell suspension was irradiated with 302 nm UV light for 1 minute, and the SB buffer was removed by centrifugation. The obtained cells were analyzed by the method shown in Test Example 1. The analysis was performed in two independent tests, and the results were expressed as the ratio of the amount of protein obtained with the anti-CD71 antibody to that in the case of the mouse IgG1 isotype control antibody (anti-mouse IgG antibody) (Fig. 5) .

As shown in Fig. 5, the two analysis tests confirmed that TFRC (CD71) was significantly obtained.

### Test Examples 4 to 13: Capture of Cell Surface Antigen Using Labeled Antibody (1)

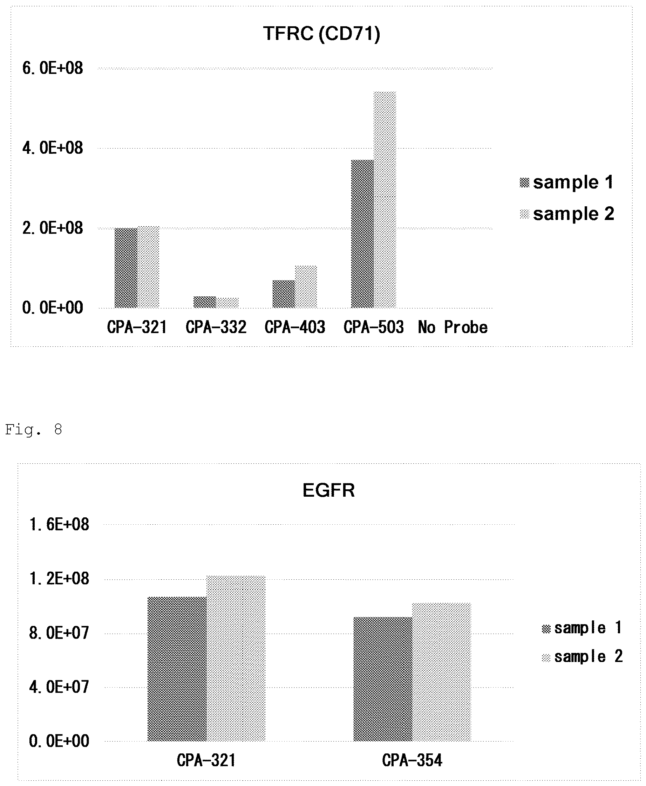

Investigation of antigen molecules from the cells shown in Table 1 was performed using the labeled antibodies shown in Table 1. Specifically, the cells shown in Table 1 were washed with SB buffer (PBS buffer containing 2% FCS), suspended in SB buffer containing a 0.1% Fc block reagent (Invitrogen, 16-9161-73), and reacted at 4°C for 15 minutes. Each supernatant was individually removed by centrifugation (600×g, 3 minutes, 4°C), and each of solutions containing labeled antibodies produced according to the production method for labeled antibodies described below was individually added, followed by a reaction at 4°C for 20 minutes. Each antibody solution was individually removed by centrifugation, and the cells were washed with SB buffer and then suspended in 0.5 ml of SB buffer. Each cell suspension was individually irradiated with ultraviolet light of the wavelength shown in the table for the time period shown in the table, and the SB buffer was removed by centrifugation. The collected cells were analyzed by the method shown in Test Example 1. The analysis was performed in two independent test, and the detection amounts of the membrane proteins to be analyzed shown in the table were compared (Figs. 6 to 8).

### Production of Labeled Antibody (Test Examples 4 to 12)

The antibodies shown in Table 1 were individually placed in separate tubes, and diluted with NHS labeling buffer (25 mM HEPES-NaOH pH 8.2, 150 mM NaCl) to a final concentration of about 0.05 mg/ml. About 0.9 µL of each of solutions of the probes shown in Table 1 in DMSO (10 mg/ml in DMSO) was individually added thereto, followed by a reaction at room temperature for 2 hours. Subsequently, about 5 µL of a lysine solution (100 mM) was added thereto, followed by a reaction at room temperature for 20 minutes. The resulting solutions were stored at 4°C until use.

### Production of Labeled Ligand (Test Example 13)

12.5 µg of His-Tag EGF was placed in a tube and diluted with NHS labeling buffer to a final concentration of about 0.25 mg/ml. 2 µL of 5 mM CPA-354 was added to 48 µL of this solution to prepare a labeled ligand solution. This solution was stored at 4°C until use.

**Table 2**

| Test Example | Probe | Antibody | Final concentration | Cell | Ultraviolet light (wavelength, irradiation time) | Membrane protein to be analysed |
|---|---|---|---|---|---|---|
| Test Example 4 | CPA-321 | Anti-CD71 antibody OKT9 (5 µg) | 0.05 mg/ml | THP-1 | 302 nm, 1 min | CD71 |
| Test Example 5 | CPA-325 | Anti-CD71 antibody OKT9 (5 µg) | 0.05 mg/ml | THP-1 | 302 nm, 1 min | CD71 |
| Test Example 6 | CPA-326 | Anti-CD71 antibody OKT9 (5 µg) | 0.05 mg/ml | THP-1 | 302 nm, 1 min | CD71 |
| Test Example 7 | CPA-346 | Anti-CD71 antibody OKT9 (5 µg) | 0.05 mg/ml | THP-1 | 302 nm, 1 min | CD71 |
| Test Example 8 | CPA-321 | Anti-CD71 antibody OKT9 (5 µg) | 0.05 mg/ml | A431 | 302 nm, 1 min | CD71 |
| Test Example 9 | CPA-332 | Anti-CD71 antibody OKT9 (5 µg) | 0.05 mg/ml | A431 | 302 nm, 1 min | CD71 |
| Test Example 10 | CPA-403 | Anti-CD71 antibody OKT9 (5 µg) | 0.05 mg/ml | A431 | 365 nm, 30 min | CD71 |
| Test Example 11 | CPA-503 | Anti-CD71 antibody OKT9 (5 µg) | 0.05 mg/ml | A431 | 365 nm, 15 min | CD71 |
| Test Example 12 | CPA-321 | His-Tag EGF (12.5 µg) | 0.25 mg/ml | MDA-MB-231 | 302 nm, 1 min | EGFR |
| Test Example 13 | CPA-354 | His-Tag EGF (12.5 µg) | 0.25 mg/ml | MDA-MB-231 | 302 nm, 1 min | EGFR |

### Test Example 14: Capture of Cell Surface Antigen Using Labeled Antibody (2)

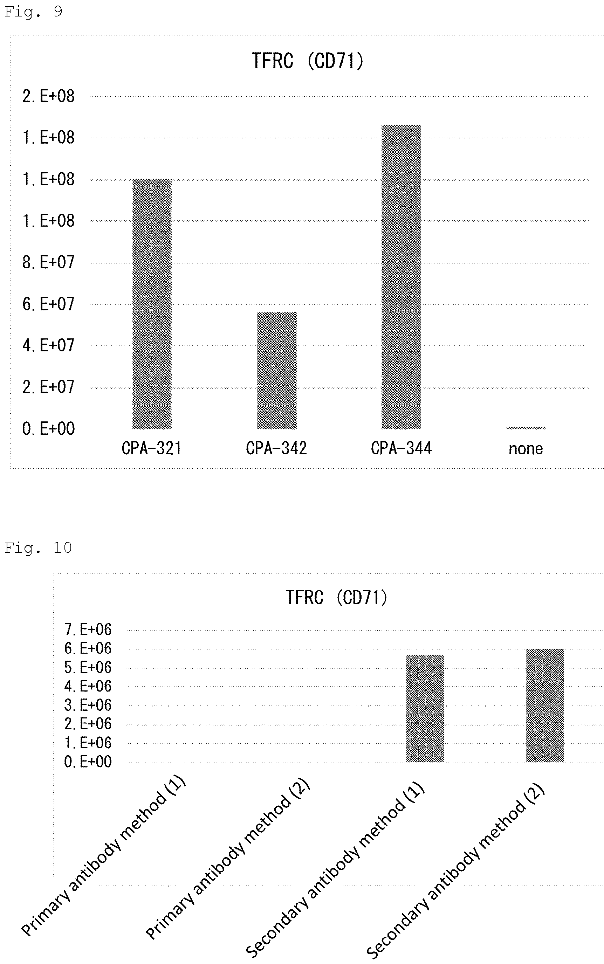

Investigation of antigen molecules from A431 cells was performed using labeled OKT9 produced by the methods shown in the table below. First, the cells were washed with SB buffer (PBS buffer containing 2% FCS). Thereafter, SB buffer containing a 0.1% Fc block reagent (Invitrogen, 16-9161-73) was added, and the cells were reacted at 4°C for 15 minutes. The supernatant was removed, and 250 µL of a ligand solution diluted 10-fold with SB buffer was added, followed by a reaction at 4°C for 20 minutes. The ligand solution was removed, and the cells were washed with PBS and then irradiated with 302 nm UV light for 1.5 minutes. Proteins were extracted from the cells with lysis buffer containing 1% NP-40 and analyzed by the method shown in Test Example 1. The amount of CD71 detected when each ligand that was formed into a probe was used was compared with that when an OKT9 antibody that was not formed into a probe was used as a negative control (No Probe) (Fig. 9).

### Production of Labeled Antibody

(1) 10 µg of anti-CD71 antibody OKT9 (Thermo Fisher Scientific, Cat # 16-0719-85) was placed in a tube and diluted with NHS labeling buffer (25 mM HEPES-NaOH pH 8.2, 150 mM NaCl) to a final concentration of about 0.1 mg/ml. 0.9 µL of a CPA-321 solution (10 mg/ml in DMSO) was added thereto, followed by a reaction at room temperature for 2 hours. Subsequently, about 5 µL of a lysine solution (100 mM) was added thereto, followed by a reaction at room temperature for 20 minutes. The resulting solution was stored at 4°C until use.
(2) 10 µg of anti-CD71 antibody OKT9 was placed in a tube, and 5 µL of 30 mM sodium periodate (in phosphate buffer, pH 6.5) was added. Thereafter, phosphate buffer (pH 6.5) was added to make a total volume of 100 µL. After a reaction at room temperature for 15 minutes, solvent exchange to PBS was performed using a Zeba Spin column (Thermo Scientific). 1 µL of a 500 mM aqueous 5-MA solution was added to 100 µL of the obtained antibody solution. Subsequently, 1 µL of a solution of 10 mg/ml CPA-342 or CPA-344 in DMSO was added, followed by a reaction at room temperature under light-shielded conditions for 1.5 hours. After the reaction, solvent exchange to PBS was performed using a Zeba Spin column, and the excess reagent was removed.

**Table 3**

| Test Example | Probe | Production method for labeled antibody |
|---|---|---|
| Test Example 14 | CPA-321 | (1) |
| Test Example 15 | CPA-342 | (2) |
| Test Example 16 | CPA-344 | (2) |

### Test Example 17: Capture of Cell Surface Antigen Using Labeled Antibody (Secondary Antibody Method)

Investigation of antigen molecules from THP-1 cells was performed using a labeled secondary antibody produced by the production method for a CBP-198-labeled secondary antibody. Specifically, THP-1 cells were washed with SB buffer (PBS buffer containing 2% FCS) and suspended in SB buffer containing a 0.1% Fc block reagent (Invitrogen, 16-9161-73), and then the cells were reacted at 4°C for 15 minutes. The supernatant was removed by centrifugation (600×g, 3 minutes, 4°C). An anti-CD71 antibody DF1513 solution diluted to 5 µg/ mL with SB buffer was added to the cells, and the cells were reacted at 4°C for 20 minutes. The antibody solution was removed by centrifugation, and the cells were washed with SB buffer. Subsequently, the cells were suspended in a solution (obtained by adding SB buffer to a labeled secondary antibody produced by the production method for a CPA-503-labeled secondary antibody to a total volume of 1 mL) and reacted at 4°C for 20 minutes. The antibody solution was removed by centrifugation, and the cells were washed with SB buffer and then suspended in 0.5 ml of SB buffer. Irradiation with 365 nm UV light was performed for 15 minutes, and the SB buffer was removed by centrifugation. The collected cells were analyzed by the method shown in Test Example 1. The analysis was performed in two independent tests, and the amounts of CD71 detected were compared.

As a comparative example, the amount of CD71 detected was calculated in the same manner as in Test Example 11 except that anti-CD71 antibody DF1513 was used in place of anti-CD71 antibody OKT9 (Fig. 10).

### Production of CPA-503-labeled Secondary Antibody

Goat anti-mouse IgG (5 µg) was placed in a tube and diluted with NHS labeling buffer (25 mM HEPES-NaOH pH 8.2, 150 mM NaCl) to a final concentration of about 0.05 mg/m shown in the table. About 0.9 µL of a solution of CPA-503 in DMSO (10 mg/ml in DMSO) was added, followed by a reaction at room temperature for 2 hours. Subsequently, about 5 µL of a lysine solution (100 mM) was added thereto, followed by a reaction at room temperature for 20 minutes. The resulting solution was stored at 4°C until use.

By using the secondary antibody method, a membrane protein that cannot be detected by the primary antibody method can be detected.

### Sequence Listing Free Text

SEQ ID NOs: 1 and 2 are both amino acid sequences of peptides, each of which is an embodiment of a compound that forms the cleavable moiety (E) of the tetra-functional compound or tetra-functional chemical probe of the present invention.

## Claims

1. A tetra-functional compound comprising
a ligand-binding moiety (A),
a reactive moiety (D),
a cleavable moiety (E), and
a biotin tag (B), the moieties (A), (D), (E), and (B) being linked optionally via a spacer,
wherein
the ligand-binding moiety (A) is at least one activated functional group selected from the group consisting of an amine-reactive group, a hydroxyl-reactive group, a thiol-reactive group, an aldehyde-reactive or ketone-reactive group, an alkyl-halide-reactive or aryl-halide-reactive group, an alkyl- sulfonate-reactive or aryl-sulfonate-reactive group, an amide-reactive group, a sulfonamide-reactive group, an aryl-reactive group, a diol-reactive group, and a carboxyl-reactive group, or at least one reactive functional group selected from the group consisting of - COOH, -NH₂, -OH, -SH, -CH=CH-, -(C=O)-CH=CH-, alkyl halide, vinyl halide, aryl halide, alkyl sulfonate, vinyl sulfonate, aryl sulfonate, alkynyl, azido, epoxy, and a click tag,
the reactive moiety (D) is a group having a structure of at least one compound selected from the group consisting of 2-aryl-5-carbonyltetrazole, phenyl azido, diazirine, α-ketoamide, 4-hydroxybenzene, phthalhydrazide, and benzophenone,
the cleavable moiety (E) is a group having a structure of at least one compound selected from the group consisting of 1-(4,4-dimethyl-2,6-dioxocyclohex-1-ylidene)ethyl, levulinoyl ester, vicinal diol, diazobenzene, bisaryl hydrazone, dialkoxydiphenylsilane, disulfide, a peptide containing the sequence of ENLYFQG (SEQ ID NO: 1), and a peptide containing the sequence of ENLYFQS (SEQ ID NO: 2),
the biotin tag (B) is a group represented by the following formula (1) 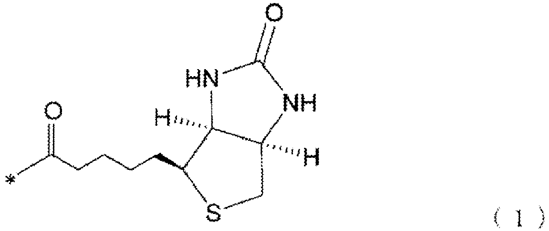 wherein symbol * represents a bond with an adjacent group;
the spacer is a linear or branched alkylene group having one or more carbon atoms, or a linear or branched alkylene group having three or more carbon atoms wherein non-adjacent -CH₂- groups are independently replaced with at least one crosslinking group selected from the group consisting of -O-, -OCH₂-, -CH₂O-, -CO-, -NRₐ- wherein Rₐ represents a hydrogen atom or a C₁₋₆ alkyl group, the same applies below, -CO-NRₐ-, -NRₐ-CO-, a group represented by the following formula (1-1), and a group represented by the following formula (1-2) 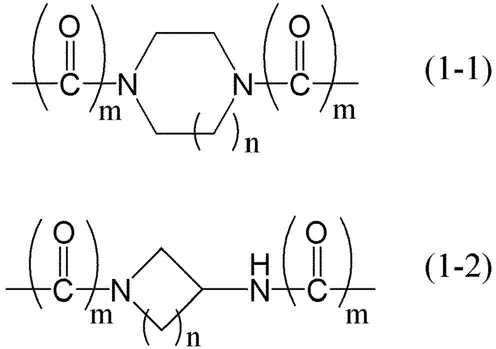 wherein m represents 0 or 1, and n represents 1 or 2.

2. The tetra-functional compound according to claim 1, wherein the reactive moiety (D) is a group represented by the following formula (2) or (2'), 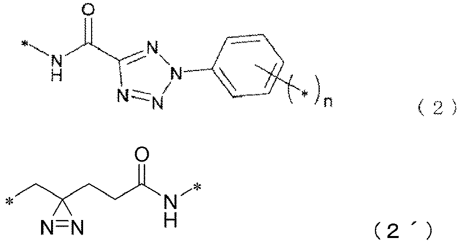
wherein symbol * represents a bond with an adjacent group, and n represents 0 or 1,
and/or,
the cleavable moiety (E) is a group represented by the following formula (3) 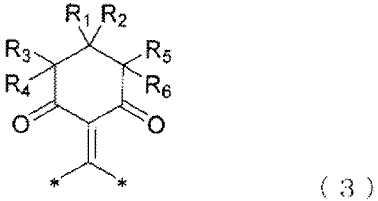
wherein R₁, R₂, R₃, R₄, R₅, and R₆ each independently represent a hydrogen atom or a C₁₋₆ alkyl group, and symbol * represents a bond with an adjacent group.

3. The tetra-functional compound according to claim 1 or 2, which is represented by the following formula (I) or formula (II): 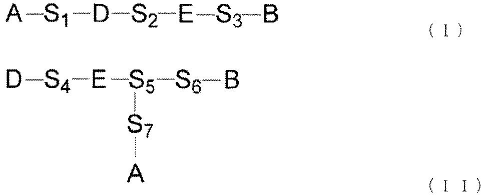 wherein A, B, C, D, and E represent the respective corresponding moieties of claim 1, and S₁, S₂, S₃, S₄, S₅, S₆, and S₇ each independently represent a spacer.

4. The tetra-functional compound according to any one of claims 1 to 3, wherein the ligand-binding moiety (A) is an N-hydroxysuccinimide ester group.

5. The tetra-functional compound according to claim 4, which is represented by the following formula (i), (ii), or (iii): 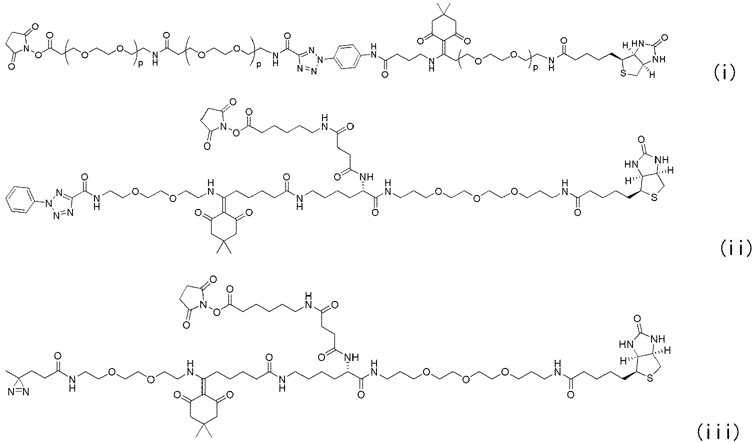 wherein p represents 2.

6. A tetra-functional chemical probe comprising
the tetra-functional compound of any one of claims 1 to 5, and
a ligand binding to the ligand-binding moiety (A) of the tetra-functional compound.

7. The tetra-functional chemical probe according to claim 6, wherein the ligand is one member selected from the group consisting of a protein, a peptide, a lipid, a carbohydrate, and a small molecule compound that all have an affinity for a membrane protein derived from a living cell or living tissue.

8. The tetra-functional chemical probe according to claim 6 or 7, which is represented by formula (iv): 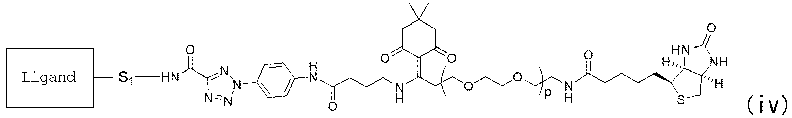 wherein S₁ represents a spacer, and p represents 2.

9. A method for detecting a target protein in a cell or tissue by using the tetra-functional chemical probe of any one of claims 6 to 8, the method comprising
(1) reacting a cell or tissue with the tetra-functional chemical probe to bind the ligand in the tetra-functional chemical probe with a target protein,
(2) forming a covalent bond between the reactive moiety (D) in the tetra-functional chemical probe and the target protein,
(3) purifying a fraction containing the target protein bound to the tetra-functional chemical probe,
(4) binding the biotin tag in the tetra-functional chemical probe to avidin to form a conjugate of the tetra-functional chemical probe and the avidin,
(5) cleaving the formed conjugate of the tetra-functional chemical probe and the avidin at the cleavable moiety in the tetra-functional chemical probe, and
(6) detecting the fraction containing the target protein.
